# FASCICULE DE BREVET EUROPEEN

(11) **EP 2 513 088 B1**
(45) Date de publication et mention de la délivrance du brevet: **01.07.2015**
(21) Numéro de dépôt: 10801663.5
(22) Date de dépôt: 13.12.2010
(51) Int. Cl.: C07D 401/14, C07D 403/14, C07D 407/14, C07D 409/14, C07D 495/04, A61K 31/496, A61K 31/497, A61K 31/4995, A61K 31/506, A61K 31/551, A61P 25/00, C07D 401/04, C07D 407/04, C07D 409/04

(54) **NOUVEAUX DÉRIVÉS (HÉTÉROCYCLE-TÉTRAHYDRO-PYRIDINE)-(PIPERAZINYL)-1-ALCANONE ET (HÉTÉROCYCLE-DIHYDRO-PYRROLIDINE)-(PIPÉRAZINYL)-1-ALCANONE ET LEUR UTILISATION COMME INHIBITEURS DE P75**
NEUE (HETEROCYCLISCHE/TETRAHYDROPYRIDIN)-(PIPERAZINYL)-1-ALCANON UND (HETEROCYCLISCHE/DIHYDROPYRROLIDIN)-(PIPERAZINYL)-1-ALCANON-DERIVATE UND DEREN VERWENDUNG ALS P75-INHIBITOREN
NOVEL (HETEROCYCLE/TETRAHYDROPYRIDINE)-(PIPERAZINYL)-1-ALCANONE AND (HETEROCYCLE/DIHYDROPYRROLIDINE)-(PIPERAZINYL)-1-ALCANONE DERIVATIVES, AND USE THEREOF AS P75 INHIBITORS

(30) Priorité: 14.12.2009 FR 0906025
(43) Date de publication de la demande: 24.10.2012
(73) Titulaire: SANOFI, 75008 Paris (FR)
(72) Inventeur: BARONI, Marco, F-75013 Paris (FR); BONO, Françoise, F-75013 Paris (FR); DELBARY-GOSSART, Sandrine, F-75013 Paris (FR); VERCESI, Valentina, F-75013 Paris (FR)
(74) Mandataire: Quellari, Mylène Audrey Séverine
(86) Numéro de dépôt international: PCT/FR2010/052686
(87) Numéro de publication internationale: WO 2011/080445

(56) Documents cités:
- WO-A1-03/104226
- DATABASE CHEMCATS [Online] CHEMICAL ABSTRACTS SERVICE, COLUMBUS, OHIO, US; 5 novembre 2009 (2009-11-05), "RN: 1087792-03-7", XP002570783, extrait de STN Database accession no. 0000883057 & "Enamine Screening Library", 5 novembre 2009 (2009-11-05), Enamine, 23 Alexander Matrosova Street, Kiev, 01103, Ukraine
- SARAGOVI H U ET AL: "Small molecule and protein-based neurotrophic ligands: Agonists and antagonists as therapeutic agents", EXPERT OPINION ON THERAPEUTIC PATENTS 1999 GB, vol. 9, no. 6, 1999, pages 737-751, XP002570814, ISSN: 1354-3776

## Description

La présente invention a pour objet des dérivés (hétérocycle-tétrahydro-pyridine)-(pipérazinyl)-1-alcanone et (hétérocycle-dihydro-pyrrolidine)-(pipérazinyl)-1-alcanone, leur préparation et leur application en thérapeutique.

Les composés selon la présente invention présentent une affinité pour le récepteur p75^{NTR} des neurotrophines.

Les neurotrophines appartiennent à une famille de protéines ayant notamment pour effet biologique la survie et la différenciation cellulaires.

Le récepteur p75^{NTR}, récepteur de toutes les neurotrophines, est une glycoprotéine transmembranaire de la famille du récepteur du facteur de nécrose tumorale (TNF, de l'anglais Tumor Necrosis Factor) (W. J. Friedman et L. A. Greene, Exp. Cell. Res., 1999,253, 131-142). Le récepteur p75^{NTR} est exprimé dans plusieurs types cellulaires, et plusieurs fonctions biologiques lui sont attribuées : d'une part, la modulation de l'affinité des neurotrophines pour les récepteurs tyrosine kinase (trk) ; d'autre part, en l'absence de trk, une induction d'un signal de mort cellulaire par apoptose. Par ailleurs, les précurseurs des neurotrophines, les proneurotrophines sont capables de se fixer sur p75^{NTR} avec une haute affinité, et sont considérés comme de puissants inducteurs de l'apoptose dépendant de p75^{NTR} dans les neurones et certaines lignées cellulaires.

Au niveau du système nerveux central, de nombreux travaux montrent que l'apoptose intervient dans plusieurs pathologies comme la sclérose latérale amyotrophique, la sclérose en plaques, les maladies d'Alzheimer, de Parkinson et d'Huntington et les maladies à prion. P75^{NTR} est également connu pour être surexprimé dans différents types de maladies neurodégénératives comme la maladie d'Alzheimer et la sclérose latérale amyotrophique (ALS) (Longo F. M. et al., Curr. Alzheimer Res. 2007;4: 503-506; Lowry K.S. et al., Amyotroph. Lateral. Scier. Other. Motor. Neuron. Disord. 2001; 2:127-34).

Des résultats suggèrent que p75^{NTR} peut jouer un rôle prépondérant dans les mécanismes conduisant à la mort neuronale par apoptose post-ischémie (P. P. Roux et al., J. Neurosci., 1999, 19, 6887- 6896).

Des résultats (V. Della-Bianca et al., J. Biol. Chem., 2001, 276 : 38929-33), (S. Rabizadeh et al., Proc. Natl. Acad. Sci. USA, 1994,91, 10703-10706) supportent l'hypothèse selon laquelle p75^{NTR} jouerait un rôle important dans la mort neuronale induite par la protéine prion infectieuse (encéphalopathie spongiforme transmissible) ou par la protéine beta Amyloide (maladie d'Alzheimer).

Le récepteur p75^{NTR} est également associé au récepteur Nogo et impliqué dans la signalisation des effets inhibiteurs de ces protéines de la myéline vis-à-vis de la croissance axonale. De ce fait, le récepteur p75^{NTR} joue un rôle majeur dans la régulation de la plasticité neuronale et dans les interactions neurones-glie et représente ainsi une cible thérapeutique de choix pour promouvoir la régénération nerveuse.

Au-delà du système nerveux et des maladies neurodégénératives, il a été suggéré que p75^{NTR} pouvait jouer un rôle dans des maladies cardiovasculaires telles que l'athérosclérose et l'ischémie du myocarde (M. L. Bochaton-Pialat et al., Am. J. Pathol., 1995,146, 1-6 ; H. Perlman, Circulation, 1997,95, 981-987). Des travaux récents montrent une augmentation de l'expression de p75^{NTR} et des neurotrophines, et une apoptose massive dans des lésions d'athérosclérose.

Plusieurs études suggèrent également que p75^{NTR} est un médiateur de l'inflammation (Rihl M. et al., Ann. Rheum. Dis. 2005; 64(11):1542-9 ; Raychaudhuri S.P. et al., Prog. Brain. Res. 2004;146: 433-7, Tokuoka S. et al., Br. J. Pharmacol. 2001, 134: 1580-1586).

P75^{NTR} est également décrit pour jouer un rôle important dans la douleur inflammatoire. En effet, la lésion du nerf augmenterait de manière sélective l'expression et le transport axonal de p75^{NTR}, impliqué dans l'induction de la douleur neuropathique. De plus, l'utilisation d'anticorps spécifique de p75^{NTR} ou d'antisens oligodeoxynucleotide capable de bloquer l'activité du récepteur *in vivo* serait capable de réverser la douleur neuropathique (hyperalgésie par la chaleur et le froid et allodynie mécanique) induite chez le rat après lésion du nerf spinal L5 (Obata K. et al., J. Neurosci. 2006 ; 26: 11974-11986). Un anticorps neutralisant anti-p75^{NTR} réduit de manière considérable la douleur inflammatoire induite par l'injection d'adjuvant dans la voute plantaire chez la souris, ainsi que dans un modèle de crush du nerf sciatique chez la souris (Watanabe T. et al., J. Neurosci. Res. 2008 ; 86: 3566-357 ; Fukui Y. et al.. J Orthop Res. 2010; 28(3): 279-83).

L'expression de p75^{NTR} est aussi décrite dans la pancréatite chronique, avec une implication dans le processus apoptotique du pancréas exocrine et endocrine. (Zhu Z. et al., Dig. Dis. Sci. 2003; 48 (4): 717-25).

D'autres rapports ont également décrit l'importance de p75^{NTR} dans le développement de la fibrose hépatique (Kendall T. J. et al., Hepatology. 2009; 49 (3): 901-10).

P75^{NTR} joue également un rôle critique dans la biologie tumorale.

De nombreux composés sont connus pour interagir avec le système trkA/NGF/p75^{NTR} ou pour posséder une activité de type NGF (facteur de croissance neuronale, de l'anglais nerve growth factor). Ainsi la demande de brevet WO 00/59893 décrit des dérivés de pyrimidines substituées qui présentent une activité de type NGF et/ou qui augmentent l'activité du NGF sur les cellules PC12. De la demande de brevet WO03/104226 sont connus des dérivés de pipérazinylacylpipéridine présentant une affinité pour le récepteur p75 des neurotrophines. Saragovi H.U et al (Exp. Opin. on Ther. patents (1999) 9(6) :737-751) décrit par ailleurs différents agents ayant une activité neurotrophique.

La présente invention a pour objet les composés répondant à la formule (I) : 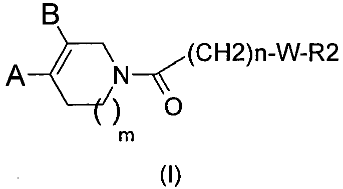 dans laquelle :
- n represente 1 ou 2 ;
- m représente 0 ou 1 ;
- A représente un groupe hétérocyclique condensé de formule (Y) 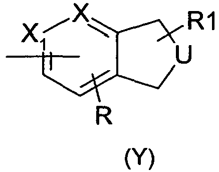 et B représente un atome d'hydrogène ;
   ou
   A représente un atome d'hydrogène ;
   et B représente un groupe hétérocyclique condensé de formule (Y) 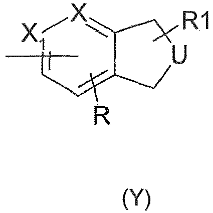
L'hétérocycle condensé de formule Y pouvant être rattaché au reste de la molécule par l'un quelconque des atomes de carbone disponibles et dans lequel :
- U complète :
   - soit un noyau à 6 atomes, aromatique ou saturé, contenant 1 ou 2 atomes d'azote, le noyau pouvant être substitué par un ou deux atomes d'halogène, un ou deux groupes (C1-C4) alkyle ou (C1-C4) alcoxy, un ou deux radicaux perfluoroalkyle ;
   - soit un noyau à 5 atomes, aromatique ou saturé, contenant un atome d'azote, d'oxygène ou de soufre, le noyau pouvant être substitué par un ou deux groupes (C1-C4) alkyle ;
- X et X1 représentent CH ou N ;
- R et R1 situés sur l'une quelconque des positions disponibles, représentent indépendamment un atome d'hydrogène, un atome d'halogène, un groupe (C1-C4)alkyle, (C1-C4)alcoxy, un radical perfluoroalkyle, trifluorométhoxy, un cyano, un groupe COOH, COOalkyle, CONR3R4 ou NHCOR3 ;
   - -W- est un hétérocycle azoté choisi parmi :
      -W- = 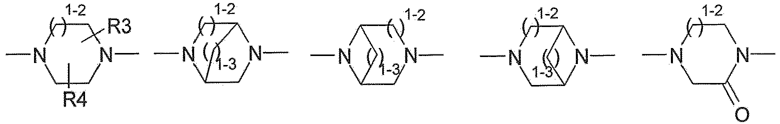
- 1-2 représente 1 ou 2 ;
- 1-3 représente 1, 2 ou 3 ;
- R2 représente un groupe de formule : 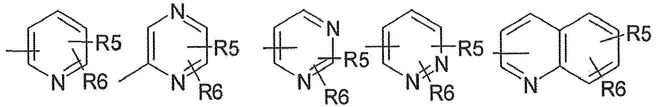
- dans lequel R5 et R6, situés sur l'une quelconque des positions disponibles, représentent indépendamment un atome d'hydrogène, un atome d'halogène, un groupe (C1-C4)alkyle, (C1-C4)alcoxy, un radical trifluorométhyle, trifluorométhoxy, un cyano, un groupe COOH, COOalkyle, COOcycloalkyle, SOalkyle, SO₂alkyle, CONR3R4, NR3R4 ou NHCOR3 ;
ou un des R5 et R6 peut aussi représenter un hétérocycle choisi parmi : 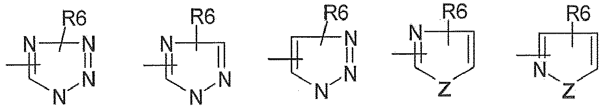 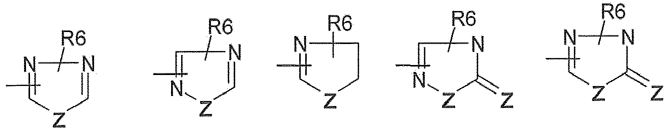
- Z représente un atome d'oxygène ou de soufre ;
- R3 et R4 représentent un hydrogène ou un groupe (C1-C6) alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par:
- un atome d'halogène : un fluor, un chlore, un brome ou un iode ;
- un groupe alkyle : un groupe aliphatique saturé linéaire ramifié, ou cyclique. A titre d'exemples, on peut citer un groupe (C1-C4) alkyle pouvant représenter un méthyle, éthyle, propyle, isopropyle, butyle, isobutyle, tertbutyle, le cyclopropyl ou le cyclobutyl ;
- un groupe fluoroalkyle : un groupe alkyle dont un ou plusieurs atomes d'hydrogène ont été substitués par un atome de fluor ;
- un groupe perfluoroalkyle : un groupe alkyle dont tous les atomes d'hydrogène ont été substitués par un atome de fluor, par exemple trifluoroalkyle ;
- un groupe alcoxy : un radical -O-alkyle où le groupe alkyle est tel que précédemment défini ;
- un groupe perfluoroalcoxy : un groupe alcoxy dont tous les atomes d'hydrogène ont été substitués par un atome de fluor, par exemple trifluoroalcoxy ;
- un groupe cycloalkyle : un groupe alkyle cyclique. A titre d'exemples, on peut citer les groupes cyclopropyle, méthylcyclopropyle, cyclobutyle, cyclopentyle, cyclohexyle, etc.

Parmi les composés de formule (I) objets de l'invention, un autre groupe de composés est constitué par les composés de formule (I) dans laquelle :
- n représente 1 ou 2 ; et/ou
- m représente 0 ou 1 ; et/ou
- A représente un groupe hétérocyclique condensé de formule (Y) 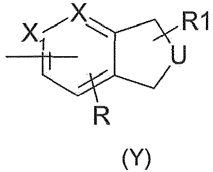 et B représente un atome d'hydrogène ;
   ou
   A représente un atome d'hydrogène ;
   et B représente un groupe hétérocyclique condensé de formule (Y) 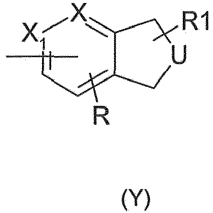 l'hétérocycle condensé de formule Y pouvant être rattaché au reste de la molécule par l'un quelconque des atomes de carbone disponibles du noyau benzénique ;
- U complète :
   - soit un noyau à 6 atomes, aromatique ou saturé, contenant 1 ou 2 atomes d'azote, le noyau pouvant être substitué par un ou deux atomes d'halogène, un ou deux groupes (C1-C4)alkyle ou (C1-C4)alcoxy, un ou deux radicaux perfluoroalkyle ;
   - soit un noyau à 5 atomes, aromatique ou saturé, contenant un atome d'azote, d'oxygène ou de soufre, le noyau pouvant être substitué par un ou deux groupes (C1-C4) alkyle ;et/ou
- X et X1 représentent CH ou N ; et/ou
- R et R1 situés sur l'une quelconque des positions disponibles, représentent indépendamment un atome d'hydrogène, un atome d'halogène, un groupe (C1-C4)alkyle, COOalkyle; et/ou
- -W- est un hétérocycle azoté choisi parmi : 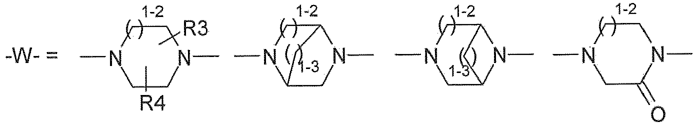 Ou bien 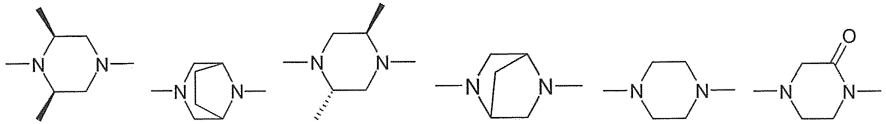 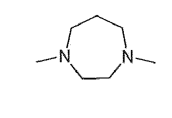 et/ou;
- R2 représente un groupe de formule : 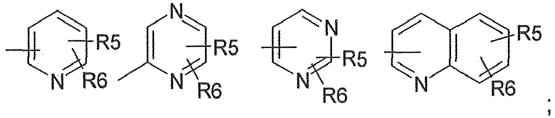 et/ou
- R5 et R6 situés sur l'une quelconque des positions disponibles, représentent indépendamment un atome d'halogène, un radical trifluorométhyle, un groupe COOH, COOalkyle ou COOcycloalkyle; ou
un des R5 et R6 peut aussi représenter un hétérocycle choisi parmi : 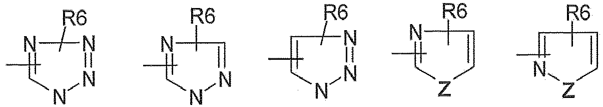 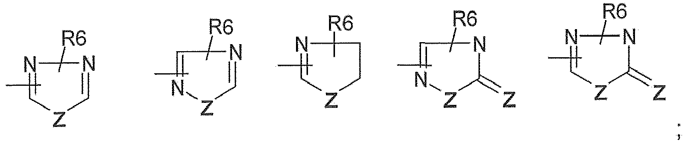 et/ou
- Z représente un atome d'oxygène ou de soufre ; et/ou
- R3 et R4 représentent un hydrogène ou un groupe méthyle.

Parmi les composés de formule (I) objets de l'invention, on peut notamment citer les composés suivants :
- Composé n°1: 1-(4-Benzo[b]thiophèn-7-yl-3,6-dihydro-2H-pyridin-1-yl)-2-(3,5-diméthyl-2,3,5,6-tétrahydro-[1,2']bipyrazinyl-4-yl)-éthanone ;
- Composé n° 2: 1-(4-Benzo[b]thiophèn-7-yl-3,6-dihydro-2H-pyridin-1-yl)-2-(8-pyrimidin-2-yl-3,8-diaza-bicyclo[3.2.1]oct-3-yl)-éthanone ;
- Composé n°3: 1-(4-Benzo[b]thiophèn-5-yl-3,6-dihydro-2H-pyridin-1-yl)-2-(8-pyrimidin-2-yl-3,8-diaza-bicyclo[3.2.1]oct-3-yl)-éthanone ;
- Composé n°4: 1-(4-Benzo[b]thiophèn-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°5: 1-(4-Benzo[b]thiophèn-5-yl-3,6-dihydro-2H-pyridin-1-yl)-2-((2S,5R)-2,5-diméthyl-2,3,5,6-tétrahydro-[1,2']bipyrazinyl-4-yl)-éthanone ;
- Composé n°6: 1-(4-Benzo[b]thiophèn-7-yl-3,6-dihydro-2H-pyridin-1-yl)-2-((2S,5R)-2,5-diméthyl-2,3,5,6-tétrahydro-[1,2']bipyrazinyl-4-yl)-éthanone ;
- Composé n°7: 1-(4-Benzo[b]thiophèn-5-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°8: 1-(4-Benzo[b]thiophèn-7-yl-3,6-dihydro-2H-pyridin-1-yl)-2-[8-(5-trifluorométhyl-pyridin-2-yl)-3,8-diaza-bicyclo[3.2.1]oct-3-yl]-éthanone ;
- Composé n°9 : 1-(4-Benzo[b]thiophèn-7-yl-3,6-dihydro-2H-pyridin-1-yl)-2-[(2R,5S)-2,5-diméthyl-4-(5-trifluorométhyl-pyridin-2-yl)-pipérazin-1-yl]-éthanone ;
- Composé n°10 : 2-[(2S,6R)-2,6-Diméthyl-4-(5-trifluorométhyl-pyridin-2-yl)-pipérazin -1-yl]-1-[4-(2-méthyl-benzo[b]thiophèn-7-yl)-3,6-dihydro-2H-pyridin-1-yl]-éthanone ;
- Composé n°11 : 2-[(2S,6R)-2,6-Diméthyl-4-(5-trifluorométhyl-pyridin-2-yl)-pipérazin -1-yl]-1-[4-(2-propyl-benzo[b]thiophèn-7-yl)-3,6-dihydro-2H-pyridin-1-yl]-éthanone ;
- Composé n°12: 1-(4-Benzo[b]thiophèn-7-yl-3,6-dihydro-2H-pyridin-1-yl)-2-[5-(5-trifluorométhyl-pyridin-2-yl)-2,5-diaza-bicyclo[2.2.1]hept-2-yl]-éthanone ;
- Composé n°13 : 1-(4-Benzo[b]thiophèn-6-yl-3,6-dihydro-2H-pyridin-1-yl)-2-[(2R,5S)-2,5-diméthyl-4-(5-trifluorométhyl-pyridin-2-yl)-pipérazin-1-yl]-éthanone ;
- Composé n°14 : 1-(4-Benzofuran-7-yl-3,6-dihydro-2H-pyridin-1-yl)-2-[(2R,5S)-2,5-diméthyl-4-(5-trifluorométhyl-pyridin-2-yl)-pipérazin-1-yl]-éthanone ;
- Composé n'15 : 1-[4-(2-Méthyl-benzo[b]thiophèn-5-yl)-3,6-dihydro-2H-pyridin-1-yl]-2-(8-pyrimidin-2-yl-3,8-diaza-bicyclo[3.2.1]oct-3-yl)-éthanone ;
- Composé n°16 : 1-[4-(2-Méthyl-benzo[b]thiophèn-5-yl)-3,6-dihydro-2H-pyridin-1-yl]-2-[4-(5-trifluorométhyl-pyridin-2-yl)-pipérazin-1-yl]-éthanone ;
- Composé n°17 : 4-{2-[4-(2-Méthyl-benzo[b]thiophèn-5-yl)-3,6-dihydro-2H-pyridin-1-yl]-2-oxo-éthyl}-1-(5-trifluorométhyl-pyridin-2-yl)-pipérazin-2-one ;
- Composé n°18 : 2-[(2S,6R)-2,6-Diméthyl-4-(5-trifluorométhyl-pyridin-2-yl)-pipérazin -1-yl]-1-[4-(2-méthyl-benzo[b]thiophèn-5-yl)-3,6-dihydro-2H-pyridin-1-yl]-éthanone ;
- Composé n°19 : 1-(4-Benzo[b]thiophèn-7-yl-3,6-dihydro-2H-pyridin-1-yl)-2-((2S,6R)-2,6-diméthyl-4-quinolein-2-yl-pipérazin-1-yl)-éthanone ;
- Composé n°20 : 1-(4-Quinolin-8-yl-3,6-dihydro-2H-pyridin-1-yl)-2-[4-(5-trifluorométhyl-pyridin-2-yl)-pipérazin-1-yl]-éthanone ;
- Composé n°21 : 1-[4-(2,3-Dihydro-benzo[1,4]dioxin-6-yl)-3,6-dihydro-2H-pyridin-1-yl]-2-[4-(5-trifluorométhyl-pyridin-2-yl)-pipérazin-1-yl]-éthanone ;
- Composé n°22 : 2-[8-(5-Fluoro-pyrimidin-2-yl)-3,8-diaza-bicyclo[3.2.1]oct-3-yl]-1-[4-(2-méthyl-benzo[b]thiophèn-5-yl)-3,6-dihydro-2H-pyridin-1-yl]-éthanone ;
- Composé n°23 : 1-(4-Benzofuran-7-yl-3,6-dihydro-2H-pyridin-1-yl)-2-[8-(5-fluoro-pyrimidin-2-yl)-3,8-diaza-bicyclo[3.2.1]oct-3-yl]-éthanone ;
- Composé n°24 : 1-[4-(2,3-Dihydro-benzo[1,4]dioxin-6-yl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°25 : 2-[8-(5-Fluoro-pyrimidin-2-yl)-3,8-diaza-bicyclo[3.2.1]oct-3-yl]-1-(4-thieno[3,2-c]pyridin-4-yl-3,6-dihydro-2H-pyridin-1-yl)-éthanone ;
- Composé n°26 : 1-(4-Benzofuran-3-yl-3,6-dihydro-2H-pyridin-1-yl)-2-[8-(5-fluoro-pyrimidin-2-yl)-3,8-diaza-bicyclo[3.2.1]oct-3-yl]-éthanone ;
- Composé n°27 : 2-[8-(5-Fluoro-pyrimidin-2-yl)-3,8-diaza-bicyclo[3.2.1]oct-3-yl]-1-[4-(1H-indol-3-yl)-3,6-dihydro-2H-pyridin-1-yl]-éthanone ;
- Composé n°28 : 1-[4-(6-Fluoro-1H-indol-3-yl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°29 : 1-(4-Benzo[b]thiophèn-3-yl-3,6-dihydro-2H-pyridin-1-yl)-2-[8-(5-fluoro-pyrimidin-2-yl)-3,8-diaza-bicyclo[3.2.1]oct-3-yl]-éthanone ;
- Composé n°30 : 4-{2-[4-(1H-Indol-3-yl)-3,6-dihydro-2H-pyridin-1-yl]-2-oxo-éthyl}-1-(5-trifluorométhyl-pyridin-2-yl)-pipérazin-2-one ;
- Composé n°31 : 4-[2-(4-Benzo[b]thiophèn-7-yl-3,6-dihydro-2H-pyridin-1-yl)-2-oxo-éthyl]-1-(5-trifluorométhyl-pyridin-2-yl)-pipérazin-2-one ;
- Composé n°32 : 4-[2-(4-Benzofuran-7-yl-3,6-dihydro-2H-pyridin-1-yl)-2-oxo-éthyl]-1-(5-trifluorométhyl-pyridin-2-yl)-pipérazin-2-one ;
- Composé n°33 : 1-(4-Benzofuran-7-yl-3,6-dihydro-2H-pyridin-1-yl)-2-[4-(5-trifluorométhyl-pyridin-2-yl)-pipérazin-1-yl]-éthanone ;
- Composé n°34 : 1-(4-Benzofuran-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°35 : 1-(4-Benzofuran-7-yl-3,6-dihydro-2H-pyridin-1-yl)-2-((2S,6R)-2,6-diméthyl-4-pyrimidin-5-yl-pipérazin-1-yl)-éthanone ;
- Composé n°36 : 1-(4-Benzofuran-7-yl-3,6-dihydro-2H-pyridin-1-yl)-2-[(2S,6R)-4-(5-fluoro-pyrimidin-2-yl)-2,6-diméthyl-pipérazin-1-yl]-éthanone ;
- Composé n°37 : 1-(4-Benzofuran-7-yl-3,6-dihydro-2H-pyridin-1-yl)-2-[(2S,6R)-2,6-diméthyl-4-(6-trifluorométhyl-pyridin-3-yl)-pipérazin-1-yl]-éthanone ;
- Composé n°38 : 2-[(2S,6R)-4-(5-Fluoro-pyrimidin-2-yl)-2,6-diméthyl-pipérazin-1-yl]-1-[4-(2-méthyl-benzo[b]thiophèn-5-yl)-3,6-dihydro-2H-pyridin-1-yl]-éthanone ;
- Composé n°39 : 2-[(2S,6R)-2,6-Diméthyl-4-(6-trifluorométhyl-pyridin-3-yl)-pipérazin -1-yl]-1-[4-(2-méthyl-benzo[b]thiophèn-5-yl)-3,6-dihydro-2H-pyridin-1-yl]-éthanone ;
- Composé n°40 : 2-((2S,6R)-2,6-Diméthyl-4-pyrimidin-5-yl-pipérazin-1-yl)-1-[4-(2-méthyl-benzo[b]thiophèn-5-yl)-3,6-dihydro-2H-pyridin-1-yl]-éthanone ;
- Composé n°41 : 1-(4-Benzo[b]thiophèn-5-yl-3,6-dihydro-2H-pyridin-1-yl)-2-((2S,6R)-2,6-diméthyl-4-pyrimidin-5-yl-pipérazin-1-yl)-éthanone ;
- Composé n°42 : 1-(4-Benzo[b]thiophèn-5-yl-3,6-dihydro-2H-pyridin-1-yl)-2-[(2S,6R)-4-(5-fluoro-pyrimidin-2-yl)-2,6-diméthyl-pipérazin-1-yl]-éthanone ;
- Composé n°43 : 4-[2-(4-Benzo[b]thiophèn-5-yl-3,6-dihydro-2H-pyridin-1-yl)-2-oxo-éthyl]-1-(5-trifluorométhyl-pyridin-2-yl)-pipérazin-2-one ;
- Composé n°44 : 1-[4-(2,3-Dihydro-benzo[1,4]dioxin-6-yl)-3,6-dihydro-2H-pyridin-1-yl]-2-[(2S,6R)-4-(5-fluoro-pyrimidin-2-yl)-2,6-diméthyl-pipérazin-1-yl]-éthanone ;
- Composé n°45 : 1-[4-(2,3-Dihydro-benzo[1,4]dioxin-6-yl)-3,6-dihydro-2H-pyridin-1-yl]-2-[(2S,6R);-2,6-diméthyl-4-(6-trifluorométhyl-pyridin-3-yl)-pipérazin-1-yl]-éthanone ;
- Composé n°46 : 1-(4-Benzo[b]thiophèn-5-yl-3,6-dihydro-2H-pyridin-1-yl)-2-[4-(5-trifluorométhyl-pyridin-2-yl)-pipérazin-1-yl]-éthanone ;
- Composé n°47 : 1-(4-Benzo[b]thiophèn-5-yl-3,6-dihydro-2H-pyridin-1-yl)-2-[(2S,6R)-2,6-diméthyl-4-(6-trifluorométhyl-pyridin-3-yl)-pipérazin-1-yl]-éthanone ;
- Composé n°48 : Acide 5-{(3S,5R)-4-[2-(4-Benzo[b]thiophèn-4-yl-3,6-dihydro-2H-pyridin-1-yl)-2-oxo-éthyl]-3,5-diméthyl-pipérazin-1-yl}-pyridine-2-carboxylique ;
- Composé n°49 : 4-{2-[4-(2,3-Dihydro-benzo[1,4]dioxin-6-yl)-3,6-dihydro-2H-pyridin-1-yl]-2-oxo-éthyl}-1-(6-trifluorométhyl-pyridin-3-yl)-pipérazin-2-one ;
- Composé n°50 : 1-(4-Benzo[b]thiophèn-7-yl-3,6-dihydro-2H-pyridin-1-yl)-2-[4-(6-trifluorométhyl-pyridin-3-yl)-pipérazin-1-yl]-éthanone ;
- Composé n°51 : Méthyl ester de l'acide 5-{(3S,5R)-4-[2-(4-Benzofuran-7-yl-3,6-dihydro-2H-pyridin-1-yl)-2-oxo-éthyl]-3,5-diméthyl-pipérazin-1-yl}-pyridine-2-carboxylique ;
- Composé n°52 : Méthyl ester de l'acide 6-{(3S,5R)-4-[2-(4-Benzo[b]thiophèn-5-yl-3,6-dihydro-2H-pyridin-1-yl)-2-oxo-éthyl]-3,5-diméthyl-pipérazin-1-yl}-nicotinique ; Composé n°53 : 1-(4-Benzo[b]thiophèn-7-yl-3,6-dihydro-2H-pyridin-1-yl)-2-[(2S,6R)-4-(5-fluoro-pyrimidin-2-yl)-2,6-diméthyl-pipérazin-1-yl]-éthanone ;
- Composé n°54 : 1-(4-Benzo[b]thiophèn-7-yl-3,6-dihydro-2H-pyridin-1-yl)-2-[(2S,6R)-2,6-diméthyl-4-(6-trifluorométhyl-pyridin-3-yl)-pipérazin-1-yl]-éthanone ;
- Composé n°55 : 1-(4-Benzo[b]thiophèn-7-yl-3,6-dihydro-2H-pyridin-1-yl)-2-((2S,6R)-2,6-diméthyl-4-pyrimidin-5-yl-pipérazin-1-yl)-éthanone ;
- Composé n°56 : Acide 6-{(3S,5R)-4-[2-(4-Benzo[b]thiophèn-5-yl-3,6-dihydro-2H-pyridin-1-yl)-2-oxo-éthyl]-3,5-diméthyl-pipérazin-1-yl}-nicotinique ;
- Composé n°57 : Acide 6-{(3S,5R)-4-[2-(4-Benzofuran-7-yl-3,6-dihydro-2H-pyridin-1-yl)-2-oxo-éthyl]-3,5-diméthyl-pipérazin-1-yl}-nicotinique ;
- Composé n°58 : Méthyl ester de l'acide 6-{(3S,5R)-4-[2-(4-Benzo[b]thiophèn-7-yl-3,6-dihydro-2H-pyridin-1-yl)-2-oxo-éthyl]-3,5-diméthyl-pipérazin-1-yl}-nicotinique ;
- Composé n°59 : Acide 6-{(3S,5R)-4-[2-(4-Benzo[b]thiophèn-7-yl-3,6-dihydro-2H-pyridin-1-yl)-2-oxo-éthyl]-3,5-diméthyl-pipérazin-1-yl}-nicotinique ;
- Composé n°60 : 4-[2-(4-Benzofuran-5-yl-3,6-dihydro-2H-pyridin-1-yl)-2-oxo-éthyl]-1-(5-trifluorométhyl-pyridin-2-yl)-pipérazin-2-one ;
- Composé n°61 : Méthyl ester de l'acide 6-((3S,5R)-3,5-Diméthyl-4-{2-[4-(2-méthyl-benzo[b]thiophèn-5-yl)-3,6-dihydro-2H-pyridin-1-yl]-2-oxo-éthyl}-pipérazin-1-yl)-nicotinique ;
- Composé n°62 :Acide 6-((3S,5R)-3,5-Diméthyl-4-{2-[4-(2-méthyl-benzo[b]thiophèn-5-yl)-3,6-dihydro-2H-pyridin-1-yl]-2-oxo-éthyl}-pipérazin-1-yl)-nicotinique ;
- Composé n°63 : 1-(5-Benzofuran-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°64 : Ethyl ester de l'acide 6-{(3S,5R)-4-[2-(4-Benzofuran-7-yl-3,6-dihydro-2H-pyridin-1-yl)-2-oxo-éthyl]-3,5-diméthyl-pipérazin-1-yl}-nicotinique ;
- Composé n°65 :Méthyl ester de l'acide 6-{3-[2-(4-Benzofuran-7-yl-3,6-dihydro-2H-pyridin-1-yl)-2-oxo-éthyl]-3,8-diaza-bicyclo[3.2.1]oct-8-yl}-nicotinique;
- Composé n°66 : Méthyl ester de l'acide 7-(1-{2-[(2S,6R)-2,6-Diméthyl-4-(5-trifluorométhyl-pyridin-2-yl)-pipérazin-1-yl]-acétyl}-1,2,3,6-tétrahydro-pyridin-4-yl)-benzo[b]thiophène-2-carboxylique ;
- Composé n°67 : Méthyl ester de l'acide 5-(1-{2-[(2S,6R)-2,6-Diméthyl-4-(5-trifluorométhyl-pyridin-2-yl)-pipérazin-1-yl]-acétyl}-1,2,3,6-tétrahydro-pyridin-4-yl)-benzo[b]thiophène-2-carboxylique ;
- Composé n°68 : 2-[(2S,6R)-2,6-Diméthyl-4-(5-trifluorométhyl-pyridin-2-yl)-pipérazin -1-yl]-1-[4-(2-propyl-benzo[b]thiophèn-5-yl)-3,6-dihydro-2H-pyridin-1-yl]-éhanone ;
- Composé n°69 : Méthtyl ester de l'acide 6-{3-[2-(4-Benzo[b]thiophèn-7-yl-3,6-dihydro-2H-pyridin-1-yl)-2-oxo-éthyl]-3,8-diaza-bicyclo[3.2.1]oct-8-yl}-nicotinique ;
- Composé n°70 : Méthyl ester de l'acide 6-{3-[2-(4-Benzo[b]thiophèn-5-yl-3,6-dihydro-2H-pyridin-1-yl)-2-oxo-éthyl]-3,8-diaza-bicyclo[3.2.1]oct-8-yl}-nicotinique ;
- Composé n°71 : Acide 6-{3-[2-(4-Benzo[b]thiophèn-7-yl-3,6-dihydro-2H-pyridin-1-yl)-2-oxo-éthyl]-3,8-diaza-bicyclo[3.2.1]oct-8-yl}-nicotinique ;
- Composé n°72 : 2-[(2S,6R)-2,6-Diméthyl-4-(5-trifluorométhyl-pyridin-2-yl)-pipérazin -1-yl]-1-[4-(7-fluoro-benzofuran-5-yl)-3,6-dihydro-2H-pyridin-1-yl]-éthanone ;
- Composé n°73 : 1-[4-(2,3-Diméthyl-benzofuran-6-yl)-3,6-dihydro-2H-pyridin-1-yl]-2-[(2S,6R)-2,6-diméthyl-4-(5-trifluorométhyl-pyridin-2-yl)-pipérazin-1-yl]-éthanone ;
- Composé n°74 : 2-[(2S,6R)-2,6-Diméthyl-4-(5-trifluorométhyl-pyridin-2-yl)-pipérazin -1-yl]-1-(4-quinolein-2-yl-3,6-dihydro-2H-pyridin-1-yl)-éthanone ;
- Composé n°75 : 1-(4-Benzofuran-5-yl-3,6-dihydro-2H-pyridin-1-yl)-2-(2,3,5,6-tétrahydro-[1,2']bipyrazinyl-4-yl)-éthanone ;
- Composé n°76 : Méthyl ester de l'acide 6-{3-[2-Oxo-2-(4-thiéno[3,2-c]pyridin-4-yl-3,6-dihydro-2H-pyridin-1-yl)-éthyl]-3,8-diaza-bicyclo[3.2.1]oct-8-yl}-nicotinique ;
- Composé n°77 : Méthyl ester de l'acide 6-(3-{2-[4-(2-Méthyl-benzo[b]thiophèn-5-yl)-3,6-dihydro-2H-pyridin-1-yl]-2-oxo-éthyl}-3,8-diaza-bicyclo[3.2.1]oct-8-yl)-nicotinique ;
- Composé n°78 : 1-(4-Benzofuran-7-yl-3,6-dihydro-2H-pyridin-1-yl)-2-(4-pyridin-3-yl-[1,4]diazepan-1-yl)-éthanone ;
- Composé n°79 : Acide 6-{3-[2-Oxo-2-(4-thiéno[3,2-c]pyridin-4-yl-3,6-dihydro-2H-pyridin-1-yl)-éthyl]-3,8-diaza-bicyclo[3.2.1]oct-8-yl}-nicotinique ;
- Composé n°80 : Acide 6-(3-{2-[4-(2-Méthyl-benzo[b]thiophèn-5-yl)-3,6-dihydro-2H-pyridin-1-yl]-2-oxo-éthyl}-3,8-diaza-bicyclo[3.2.1]oct-8-yl)-nicotinique ;
- Composé n°81 : Méthyl ester de l'acide 6-{3-[2-(3-Benzofuran-7-yl-2,5-dihydro-pyrrol-1-yl)-2-oxo-éthyl]-3,8-diaza-bicyclo[3.2.1]oct-8-yl}-nicotinique ;
- Composé n°82 : Méthyl ester de l'acide 6-{3-[2-(3-Benzo[b]thiophèn-7-yl-2,5-dihydro-pyrrol-1-yl)-2-oxo-ethyl]-3,8-diaza-bicyclo[3.2.1]oct-8-yl}-nicotinique ;
- Composé n°83 : 4-[2-(3-Benzofuran-7-yl-2,5-dihydro-pyrrol-1-yl)-2-oxo-éthyl]-1-(5-trifluorométhyl-pyridin-2-yl)-pipérazin-2-one ;
- Composé n°84 : acide 6-{3-[2-(4-Benzofuran-7-yl-2,3-dihydro-pyrrol-1-yl)-2-oxo-ethyl]-3,8-diaza-bicyclo[3.2.1]oct-8-yl}-nicotinique;
- Composé n°85 : acide 6-{3-[2-(5-Benzo[b]thiophen-7-yl-3,4-dihydro-2H-pyridin-1-yl)-2-oxo-ethyl]-3,8-diaza-bicyclo[3.2.1]oct-8-yl}-nicotinique;
- Composé n°86 : acide 6-{3-[2-(4-Benzo[b]thiophen-7-yl-2,3-dihydro-pyrrol-1-yl)-2-oxo-ethyl]-3,8-diaza-bicyclo[3.2.1]oct-8-yl}-nicotinique ;
- Composé n°87 : méthyl ester de l'acide 6-(3-{2-[4-(2,3-Dihydro-benzo[1,4]dioxin-6-yl)-3,6-dihydro-2H-pyridin-1-yl]-2-oxo-ethyl}-3,8-diaza-bicyclo[3.2.1]oct-8-yl)-nicotinique;
- Composé n°88 : acide 2-{(3S,5R)-4-[2-(4-Benzo[b]thiophen-7-yl-3,6-dihydro-2H-pyridin-1-yl)-2-oxo-ethyl]-3,5-dimethyl-piperazin-1-yl}-pyrimidine-5-carboxylique;
- Composé n°89: 3-(6-{3-[2-(4-Benzofuran-7-yl-3,6-dihydro-2H-pyridin-1-yl)-2-oxo-ethyl]-3,8-diaza-bicyclo[3.2.1]oct-8-yl}-pyridin-3-yl)-4H-[1,2,4]oxadiazol-5-one ;
- Composé n°90 : 3-(6-{3-[2-(4-Benzo[b]thiophen-7-yl-3,6-dihydro-2H-pyridin-1-yl)-2-oxo-ethyl]-3,8-diaza-bicyclo[3.2.1]oct-8-yl}-pyridin-3-yl)-4H-[1,2,4]oxadiazol-5-one ;
- Composé n°91 : 6-(3-{2-[4-(2-Methyl-benzo[b]thiophen-5-yl)-3,6-dihydro-2H-pyridin -1-yl]-2-oxo-ethyl}-3,8-diaza-bicyclo[3.2.1]oct-8-yl)-nicotinonitrile ;
- Composé n°92 : 1-(4-Benzofuran-7-yl-3,6-dihydro-2H-pyridin-1-yl)-2-[(2S,6R)-4-(3-chloro-5-trifluoromethyl-pyridin-2-yl)-2,6-dimethyl-piperazin-1-yl]-ethanone ;
- Composé n°93 : 1-(4-Benzofuran-7-yl-3,6-dihydro-2H-pyridin-1-yl)-2-(8-pyridin-3-yl-3,8-diaza-bicyclo[3.2.1]oct-3-yl)-ethanone ;
- Composé n°94 : 6-{3-[2-(4-Benzofuran-7-yl-3,6-dihydro-2H-pyridin-1-yl)-2-oxo-ethyl]-3,8-diaza-bicyclo[3.2.1]oct-8-yl}-nicotinonitrile ;
- Composé n°95 : 1-(4-Benzofuran-7-yl-3,6-dihydro-2H-pyridin-1-yl)-2-[5-(5-trifluoromethyl-pyridin-2-yl)-2,5-diaza-bicyclo[2.2.1]hept-2-yl]-ethanone ;
- Composé n°96 : cyclobutyl ester de l'acide 6-(3-{2-[4-(2-Methyl-benzo[b]thiophen-5-yl)-3,6-dihydro-2H-pyridin-1-yl]-2-oxo-ethyl}-3,8-diaza-bicyclo[3.2.1]oct-8-yl)-nicotinique ;
- Composé n°97 : 2-[(2S,6R)-2,6-Dimethyl-4-(5-trifluoromethyl-pyridin-2-yl)-piperazin -1-yl]-1-[4-(1H-indol-3-yl)-3,6-dihydro-2H-pyridin-1-yl]-ethanone ;
- Composé n°98 : 1-[4-(1H-Indol-3-yl)-3,6-dihydro-2H-pyridin-1-yl]-2-(4-quinolin-2-yl-piperazin-1-yl)-ethanone ;
- Composé n°99 : 1-(4-Benzofuran-7-yl-3,6-dihydro-2H-pyridin-1-yl)-2-[4-(7-chloro-quinolin-4-yl)-piperazin-1-yl]-ethanone ;
- Composé n°100 : 2-[(2S,6R)-4-(5-Fluoro-pyrimidin-2-yl)-2,6-dimethyl-piperazin-1-yl]-1-[4-(1H-indol-3-yl)-3,6-dihydro-2H-pyridin-1-yl]-ethanone ;
- Composé n°101 : 1-(4-Benzo[b]thiophen-7-yl-3,6-dihydro-2H-pyridin-1-yl)-2-[(2S,6R)- 4-(5-chloro-pyridin-2-yl)-2,6-dimethyl-piperazin-1-yl]-ethanone ;
- Composé n°102 : 1-(4-Benzo[b]thiophen-7-yl-3,6-dihydro-2H-pyridin-1-yl)-2-((2R,5S)-2,5-dimethyl-2,3,5,6-tetrahydro-[1,2']bipyrazinyl-4-yl)-ethanone ;
- Composé n°103 : 1-(4-Benzo[b]thiophen-7-yl-3,6-dihydro-2H-pyridin-1-yl)-2-[8-(5-fluoro-pyrimidin-2-yl)-3,8-diaza-bicyclo[3.2.1]oct-3-yl]-ethanone ;
- Composé n°104 : 1-(4-Benzo[b]thiophen-7-yl-3,6-dihydro-2H-pyridin-1-yl)-2-[8-(6-trifluoromethyl-pyridin-2-yl)-3,8-diaza-bicyclo[3.2.1]oct-3-yl]-ethanone ;
- Composé n°105 : 1-(4-Benzo[b]thiophen-7-yl-3,6-dihydro-2H-pyridin-1-yl)-2-(4-quinolin-2-yl-piperazin-1-yl)-ethanone ;
- Composé n°106 : 1-(4-Benzo[b]thiophen-7-yl-3,6-dihydro-2H-pyridin-1-yl)-2-[4-(7-chloro-quinolin-4-yl)-piperazin-1-yl]-ethanone ;
- Composé n°107 : 1-(4-Benzo[b]thiophen-7-yl-3,6-dihydro-2H-pyridin-1-yl)-2-[4-(5-chloro-pyridin-2-yl)-piperazin-1-yl]-ethanone ;
- Composé n°108 : 2-[4-(6-Chloro-pyridin-2-yl)-piperazin-1-yl]-1-[4-(1H-indol-3-yl)-3,6-dihydro-2H-pyridin-1-yl]-ethanone ;
- Composé n°109 : 1-[4-(1H-Indol-3-yl)-3,6-dihydro-2H-pyridin-1-yl]-2-[4-(5-trifluoromethyl-pyridin-2-yl)-piperazin-1-yl]-ethanone ;
- Composé n°110 : 2-((2S,6R)-2,6-Dimethyl-4-quinolin-2-yl-piperazin-1-yl)-1-[4-(1H-indol-3-yl)-3,6-dihydro-2H-pyridin-1-yl]-ethanone ;
- Composé n°111 : 1-(4-Benzo[b]thiophen-7-yl-3,6-dihydro-2H-pyridin-1-yl)-2-(4-pyridin-3-yl-[1,4]diazepan-1-yl)-ethanone ;
- Composé n°112 : 1-(4-Benzo[b]thiophen-7-yl-3,6-dihydro-2H-pyridin-1-yl)-2-[4-(5,6-dichloro-pyridin-2-yl)-piperazin-1-yl]-ethanone ;
- Composé n°113 : 1-(4-Benzo[b]thiophen-7-yl-3,6-dihydro-2H-pyridin-1-yl)-2-[4-(6-bromo-pyridin-2-yl)-piperazin-1-yl]-ethanone ;
- Composé n°114 : 1-[4-(2-Methyl-benzo[b]thiophen-5-yl)-3,6-dihydro-2H-pyridin-1-yl]-2-(4-quinolin-2-yl-piperazin-1-yl)-ethanone ;
- Composé n°115 : 1-(4-Benzo[b]thiophen-7-yl-3,6-dihydro-2H-pyridin-1-yl)-2-[5-(6-trifluoromethyl-pyridazin-3-yl)-2,5-diaza-bicyclo[2.2.1]hept-2-yl]-ethanone ;
- Composé n°116 : 1-[4-(2-Methyl-benzo[b]thiophen-5-yl)-3,6-dihydro-2H-pyridin-1-yl]-2-[4-(6-trifluoromethyl-pyridin-2-yl)-piperazin-1-yl]-ethanone ;
- Composé n°117 : 2-[4-(7-Chloro-quinolin-4-yl)-piperazin-1-yl]-1-[4-(2-methyl-benzo[b]thiophen-5-yl)-3,6-dihydro-2H-pyridin-1-yl]-ethanone ;
- Composé n°118 : 4-[2-Oxo-2-(4-thieno[3,2-c]pyridin-4-yl-3,6-dihydro-2H-pyridin-1-yl)-ethyl]-1-(5-trifluoromethyl-pyridin-2-yl)-piperazin-2-one ;
- Composé n°119 : 1-(4-Benzofuran-7-yl-3,6-dihydro-2H-pyridin-1-yl)-2-[(2S,6R)-2,6-dimethyl-4-(5-thiazol-2-yl-pyridin-2-yl)-piperazin-1-yl]-ethanone ;
- Composé n°120 : 1-(4-Benzofuran-7-yl-3,6-dihydro-2H-pyridin-1-yl)-2-{(2S,6R)-2,6-dimethyl-4-[5-(2-methyl-2H-tetrazol-5-yl)-pyridin-2-yl]-piperazin-1-yl}-ethanone ;
- Composé n°121 : 2-[(2S,6R)-2,6-Dimethyl-4-(5-trifluoromethyl-pyridin-2-yl)-piperazin-1-yl]-1-(4-quinolin-2-yl-3,6-dihydro-2H-pyridin-1-yl)-ethanone ;
- Composé n°122 : 4-[2-Oxo-2-(4-quinolin-2-yl-3,6-dihydro-2H-pyridin-1-yl)-ethyl]-1-(5-trifluoromethyl-pyridin-2-yl)-piperazin-2-one ;
- Composé n°123 : 1-(4-Quinolin-2-yl-3,6-dihydro-2H-pyridin-1-yl)-2-[8-(5-trifluoromethyl-pyridin-2-yl)-3,8-diaza-bicyclo[3.2.1]oct-3-yl]-ethanone ;
- Composé n°124 : 1-(4-Benzofuran-7-yl-3,6-dihydro-2H-pyridin-1-yl)-2-{8-[5-(1-methyl-1H-tetrazol-5-yl)-pyridin-2-yl]-3,8-diaza-bicyclo[3.2.1]oct-3-yl}-ethanone ;
- Composé n°125 : 1-(4-Benzofuran-7-yl-3,6-dihydro-2H-pyridin-1-yl)-2-[(2S,6R)-4-(5-methanesulfonyl-pyridin-2-yl)-2,6-dimethyl-piperazin-1-yl]-ethanone ;
à l'état de base ou de sel d'addition à un acide.

Dans ce qui suit, on entend par "groupe protecteur Pg" un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans Protective Groups in Organic Synthesis, Green et al., 2nd Edition (John Wiley & Sons, Inc., New York).

Conformément à l'invention, on peut préparer les composés de formule générale (I) selon le procédé qui suit.

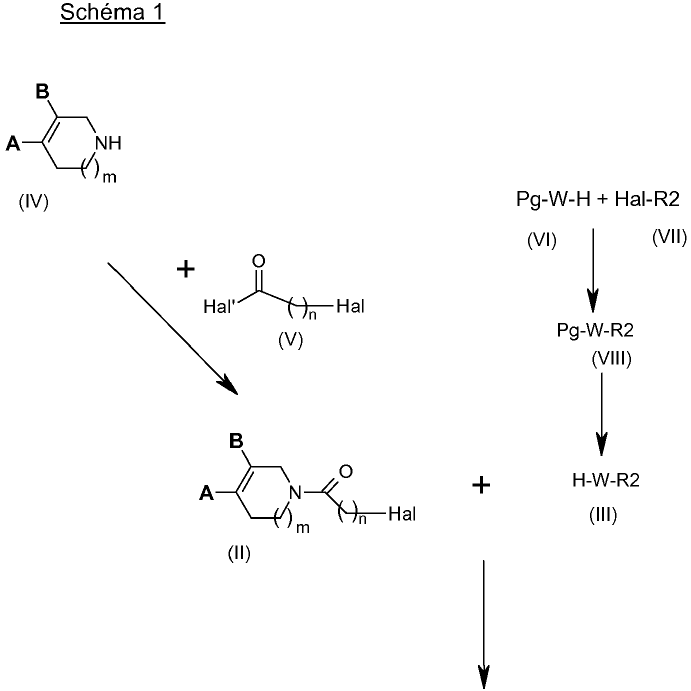

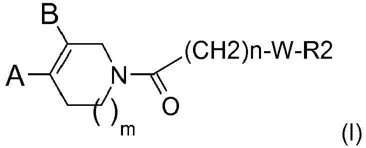

Plus précisément, le procédé de préparation des composés de formule générale (I) dans laquelle A, B, m, n, W et R2 sont tels que définis précédemment comprend la réaction d'un composé de formule (II) : 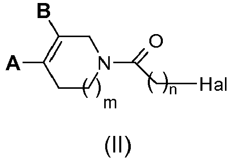 dans laquelle A, B, m et n sont définis comme en formule générale (I) et Hal représente un atome d'halogène, par exemple le chlore, et d'un composé de formule générale (III) :

H-W-R2 (III)

dans laquelle W et R2 sont définis comme en formule générale (I), selon des méthodes connues de l'homme du métier, par exemple en présence d'une base, dans un solvant tel que décrit dans WO 03/104225. Ainsi, à titre de base, on peut citer les bases organiques telles que la triéthylamine, la N,N-diisopropylamine, la Diisopropyl-éthylamine (DPEA) ou la N-méthyl-morpholine ou les carbonates ou bicarbonates de métal alcalin tel que le carbonate de potassium, le carbonate de sodium ou le bicarbonate de sodium et en l'absence ou en présence d'un iodure de métal alcalin tel que l'iodure de potassium ou l'iodure de sodium. La réaction s'effectue dans un solvant tel que l'acétonitrile, le N,N-diméthylformamide (DMF), I'N-méthyl-pyrrodinone, le toluène ou le propan-2-ol et à une température comprise entre la température ambiante et la température de reflux du solvant. Par température ambiante, on entend une température comprise entre 5 et 25°C. A titre d'exemple la réaction peut s'effectuer en présence de bicarbonate de sodium, d'iodure de sodium dans un solvant tel que le DMF. Cettes réactions peuvent aussi être conduites dans un réacteur à micro-ondes.
Dans les produits de formule générale (I) ainsi obtenus, R, R1, R3, R4, R5 et R7 peuvent être modifiés par des traitements communément utilisés par l'homme du métier, comme par exemple par hydrolyse d'un groupe ester pour donner un groupe carboxylique ou d'un cyano pour obtenir un groupe tétrazole.

Généralement, les sels d'addition acide des composés de formule générale (I) peuvent être obtenus par addition de l'acide approprié, tel que l'acide chlorhydrique, l'acide bromhydrique, l'acide oxalique.

Les composés de formule (III), éventuellement sous forme de sels peuvent être préparés à partir des composés correspondants de formule (VIII) :

Pg-W-R2 (VIII)

dans laquelle W et R2 sont tels que définis en formule (I) et Pg représente un groupe protecteur d'un atome d'azote de W. De préférence, Pg est un groupe benzyle et la déprotection s'effectue selon des méthodes classiques, bien connues de l'homme du métier, par exemple par hydrogénation catalytique sur Pd/C ou par traitement avec des chloroformiates puis hydrolyse en milieu acide.

Les composés de formule (VIII) peuvent être préparés à partir des composés de formule (VI) :

Pg-W-H (VI)

et (VII) :

Hal-R2 (VII)

dans lesquelles Pg, W, R2 sont définis comme précédemment et Hal représente un atome d'halogène, de préférence le chlore. Cette réaction s'effectue généralement dans les mêmes conditions que la réaction de préparation des composés de formule (I) à partir des composés de formule (II) et (III).

Alternativement, les composés de formule (VIII) peuvent être préparés par la méthode de couplage de Buchwald en présence d'un catalysateur au Palladium et d'une phosphine opportunément choisis, en utilisant comme solvant des solvants inertes tels que le toluène ou le xylène, à une température comprise entre la température ambiante et 110°C.
Dans les composés de formule générale (VIII) ainsi obtenus, R7 et R8, peuvent être modifiés par des traitements communément utilisés par l'homme du métier, comme par exemple la synthèse d'un groupe oxadiazole à partir d'un groupe cyano, ou bien par la formation d'un intermédiaire boronique et par couplage de Suzuki comme décrit dans le schéma ci-dessous.

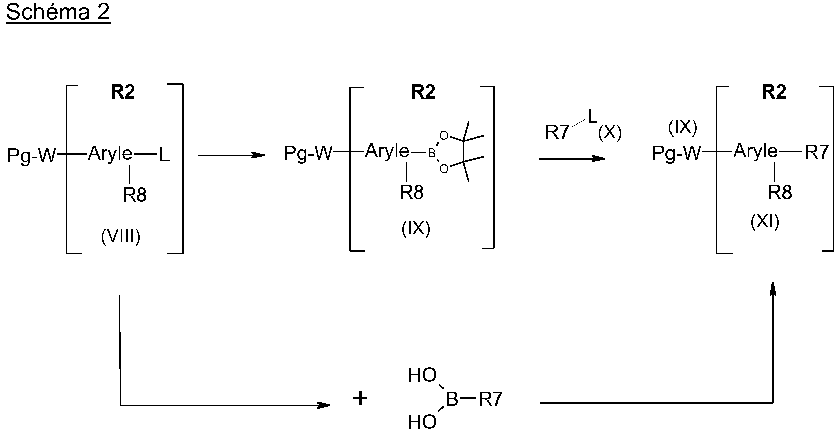

Dans le schéma 2 ci-dessus, L réprésente un groupe partant comme le lodo, le bromo ou le trifluorométhanesulphonate, R7 représente des hétérocycles tels que décrits dans la formule générale (I), R8 est tel que défini dans la formule générale (I) et de B est l'atome de Bore.

Des exemples des telles réactions sont décrits dans la partie expérimentale.

Les composés de formule (III), éventuellement sous forme de sels, quant W représente une Oxo-pipérazine sont disponibles dans le commerce ou décrits dans la littérature, ou bien peuvent être préparés, à partir des composés correspondants de formule (VIII), selon des méthodes qui sont décrites ou connues de l'homme du métier.

Des exemples des telles préparations sont décrits dans la partie expérimentale.

Les composés de formule (II) peuvent être obtenus par réaction d'un composé correspondant de formule (IV) : 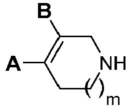 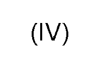 dans laquelle A, B et m sont définis comme en formule générale (I) ; éventuellement sous forme de sel d'addition acide, et d'un composé de formule (V) : 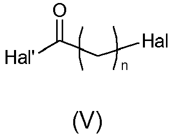 dans laquelle Hal et n sont tels que définis en formule (II) et Hal' représente un atome d'halogène, identique ou différent de Hal. De préférence, Hal' représente un atome de chlore.

Cette réaction s'effectue généralement en présence d'une base, telle que la triéthylamine, la N,N-diisopropyléthylamine ou la N-méthylmorpholine, dans un solvant tel que le dichlorométhane, le chloroforme, le tétrahydrofuranne, le dioxane ou un mélange de ces solvants et à une température comprise entre 0°C et la température ambiante. Les composés de formule (V) sont généralement disponibles commercialement.

Eventuellement, le procédé selon l'invention comprend l'étape ultérieure consistant à isoler le produit désiré obtenu.

Les produits de formules (IV) (V), (VI), (VII), et les réactifs, quand leur mode de préparation n'est pas décrit, sont disponibles dans le commerce ou décrits dans la littérature, ou bien peuvent être préparés selon des méthodes qui sont décrites ou connues de l'homme du métier.

De façon alternative, les composés de formule (I) peuvent être preparés selon le procédé qui suit :

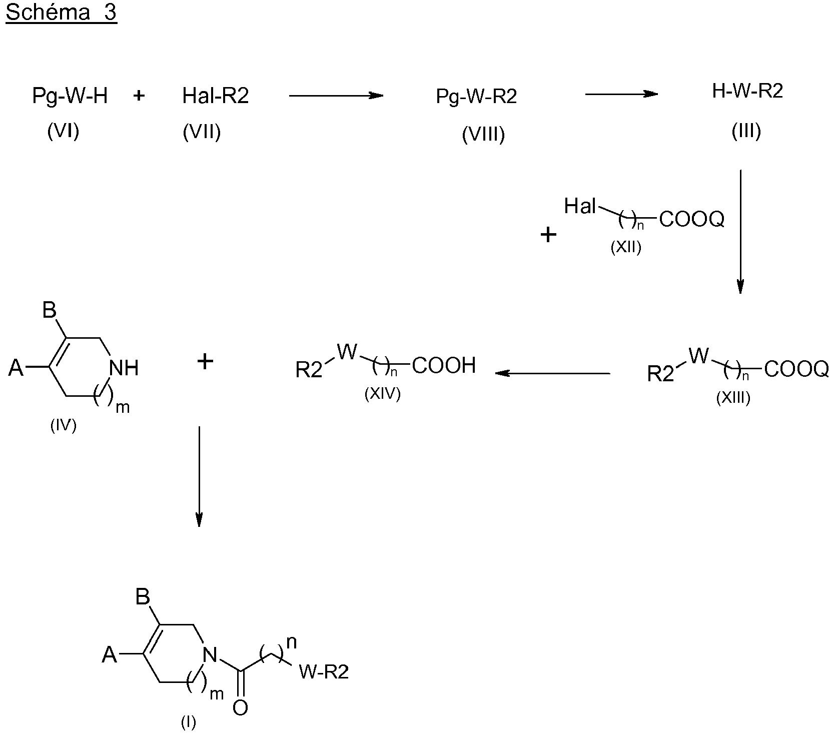

Plus précisément, le procédé de préparation des composés de formule générale (I) dans laquelle A, B, R2, m et n sont tels que définis précédemment comprend la réaction d'un composé de formule (XIV) : 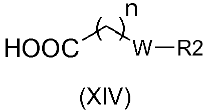 dans laquelle R2, W et n sont définis comme en formule générale (I) et d'un composé de formule générale (IV) 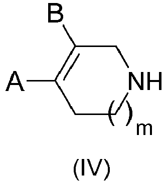 dans laquelle A, B et m sont définis comme en formule générale (I), selon des méthodes connues de l'homme du métier, par exemple , dans un solvant tel que le dichlorométhane, le DMF ou le THF, en présence d'une base telle que la pyridine, la triéthylamine, la N,N-diisopropylamine, la Diisopropyl-éthylamine (DPEA) et d'un agent condensant tel que le BOP, le DBU ou le DCC. La réaction s'effectue à une température comprise entre la température ambiante et la température de reflux du solvant. Par température ambiante, on entend une température comprise entre 5 et 25°C. A titre d'exemple la réaction peut s'effectuer en présence de bicarbonate de sodium, d'iodure de sodium dans un solvant tel que le DMF. Ces réactions peuvent aussi être conduites dans un réacteur à micro-ondes.
Dans les composés de formule générale (I) ainsi obtenus, R, R1, R3, R4, R5, R6, R7 et R8 peuvent être modifiés par des traitements communément utilisés par l'homme du métier, comme par exemple par hydrolyse d'un groupe ester pour donner un groupe carboxylique ou d'un cyano à obtenir un groupe tétrazole.

Généralement, les sels d'addition acide des composés de formule générale (I) peuvent être obtenus par addition de l'acide approprié, tel que l'acide chlorhydrique, l'acide bromhydrique, l'acide oxalique.

Les composés de formule (XIV) peuvent être obtenus à partir de composés de formule (XIII) 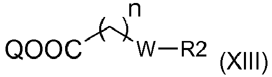 dans laquelle R2, W et n sont définis comme en formule générale (I) et Q répresente un résidu adpte à former un ester, tel que le méthyl, l'éthyl ou le benzyl, par hydrolyse de la liaison ester, selon des méthodes connues de l'homme du métier, par exemple par un traitement en milieu acqueux acide ou basique, ou bien par réduction dans un solvant polaire tel qu'un alcool ou le THF, sous flux d' hydrogène.

Les composés de formule (XIII) peuvent être obtenus à partir de composés de formule (III)

H-W-R2 (III)

dans laquelle R2 et W sont définis comme en formule générale (I) ; éventuellement sous forme de sel d'addition acide, et d'un composé de formule (XII) : 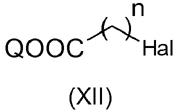 dans laquelle Q réprésente un résidu apte à former un ester tel que le méthyl, l'éthyl ou le benzyl, Hal représente un atome d'halogène, de préférence un atome de chlore, et n est tel que défini dans la formule générale (I).

Cette réaction s'effectue généralement en présence d'une base, telle que la triéthylamine, la N,N-diisopropyléthylamine ou la N-méthylmorpholine, dans un solvant tel que le dichlorométhane, le chloroforme, le tétrahydrofurane, le dioxane ou un mélange de ces solvants et à une température comprise entre 0°C et la température ambiante. Les composés de formule (XII) sont généralement disponibles commercialement.

Les composés de formule (III), éventuellement sous forme de sels peuvent être préparés à partir des composés correspondants de formule (VIII) :

Pg-W-R2 (VIII)

dans laquelle W et R2 sont tels que définis en formule (I) et Pg représente un groupe protecteur d'un atome d'azote de W. De préférence, Pg est un groupe benzyle et la déprotection s'effectue selon des méthodes classiques, bien connues de l'homme du métier, par exemple par hydrogénation catalytique sur Pd/C ou par traitement avec des chloroformiates puis hydrolyse en milieu acide.

Les composés de formule (VIII) peuvent être préparés à partir des composés de formule (VI) :

Pg-W-H (VI)

et (VII) :

Hal-R2 (VII)

dans lesquelles Pg, W et R2 sont définis comme précédemment et Hal représente un atome d'halogène, de préférence le chlore. Cette réaction s'effectue généralement dans les mêmes conditions que la réaction de préparation des composés de formule (I) à partir des composés de formule (II) et (III).

Alternativement, les composés de formule (VIII) peuvent être préparés par la méthode de couplage de Buchwald en présence d'un catalysateur au Palladium et d'une phosphine opportunément choisie, en utilisant comme solvant des solvants inertes tels que le toluène ou le xylène, à une température comprise entre la température ambiante et 110°C.
Dans les composés de formule générale (VIII) ainsi obtenus, R7 et R8, peuvent être modifiés par des traitements communément utilisés par l'homme du métier, comme par exemple la synthèse d'un groupe oxadiazole à partir d'un groupe cyano, ou bien par des couplages de Suzuki comme déjà décrit dans le schéma 2 déjà exposé ci-dessus.

Les composés de formule (III), éventuellement sous forme de sels, quant W représente une Oxo-pipérazine, sont disponibles dans le commerce ou décrits dans la littérature, ou bien peuvent être préparés, à partir des composés correspondants de formule (VII), selon des méthodes qui sont décrites ou connues de l'homme du métier.

Des exemples des telles préparations sont décrits dans la partie expérimentale.

Eventuellement, le procédé selon l'invention comprend l'étape ultérieure consistant à isoler le produit désiré obtenu.

Les produits de formules (IV), (VI), (VII), (XII) et les réactifs, quand leur mode de préparation n'est pas décrit, sont disponibles dans le commerce ou décrits dans la littérature, ou bien peuvent être préparés selon des méthodes qui sont décrites ou connues de l'homme du métier.

Des exemples des telles préparations sont décrits dans la partie expérimentale. Des composés de formule (II) sont également décrits 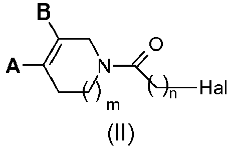 dans laquelle A, B, n et m sont définis comme en formule générale (I) et Hal représente un atome d'halogène, de préférence le chlore ; éventuellement sous forme de sel d'addition acide. Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les mesures physico-chimiques ont été effectuées de la façon suivante :

Les points de fusion ont été mesurés avec un appareil Buchi B540.

Les spectres de résonnance magnétique nucléaire du proton (RMN 1 H) ont été enregistrés dans les conditions suivantes:
a) à 500 MHz sur un appareil Bruker équipé avec une console Avance III ;
b) à 400MHz sur un appareil Bruker équipé avec une console Avance I.

Les déplacements chimiques sont rapportés en ppm par rapport à la fréquence TMS.

Les spectres ont étés enregistrés dans lec conditions de température suivantes :
Temp. A : 40°C
Temp. B : 30°C

Les abréviations utilisées pour caractériser les signaux sont les suivantes: s = singulet, bs =singulet large, m = multiplet, bm= multiplet large, d = doublet, bd= doublet large, t = triplet, q = quadruplet.
* = non intégrable à cause de l'interférence avec un pic large dû à l'eau.
** = non intégrable à cause de l'interférence avec un pic dû au solvant RMN.
2Xs= deux singulet partiellement superposés.
2Xbs= deux singulet large partiellement superposés.
2Xm= deux multiplets partiellement superposés.

Les composés sont analysés par couplage HPLC-UV-MS (chromatographie liquide -détection UV et détection masse).

L'appareil utilisé est composé d'une chaîne chromatographique Thermo Surveyor équipé d'un détecteur à barrette de diodes Thermo et d'un spectromètre de masse Thermo Deca XPMax à trappe d'ions.

Les conditions analytiques sont les suivantes :

### Conditions HPLC

Différentes conditions HPLC ont été utilisées selon les composés :

### Méthode A

Eluant A: H₂O + TFA 0,005% + CH₃CN 5%
Eluant B: CH₃CN
Gradient:

| Temps (min) | % B |
|---|---|
| 0 | 5 |
| 17 | 90 |
| 22 | 90 |
| 23 | 5 |
| 30 | 5 |

Température colonne: 30°C
Débit: 0,3 ml/min
Détection: λ = 220 nm

### Méthode B

Eluant A: H₂O + TFA 0,005%
Eluant B: CH₃CN
Gradient:

| Temps (min) | % B |
|---|---|
| 0 | 5 |
| 22 | 90 |
| 29 | 90 |
| 30 | 5 |
| 40 | 5 |

Température colonne: non contrôlée
Débit: 0,3 ml/min
Détection: λ = 220 nm

### Méthode C

Eluant A: AcONH₄ 5mM à pH 6,5
Eluant B: CH₃CN
Gradient:

| Temps (min) | % B |
|---|---|
| 0 | 5 |
| 25 | 90 |
| 30 | 90 |
| 32 | 5 |
| 40 | 5 |

Température colonne: non contrôlée
Débit: 0,3 ml/min
Détection: λ = 220 nm

### Méthode D

Eluant A: AcONH₄ 5mM à pH 6,5
Eluant B: CH₃CN
Gradient:

| Temps (min) | % B |
|---|---|
| 0 | 5 |
| 17 | 90 |
| 22 | 90 |
| 23 | 5 |
| 30 | 5 |

Température colonne: non contrôlée
Débit: 0,3 ml/min
Détection: λ = 220 nm

### Méthode E

Eluant A: H₂O + TFA 0,01%
Eluant B: CH₃CN
Gradient:

| Temps (min) | % B |
|---|---|
| 0 | 10 |
| 18 | 95 |
| 20 | 95 |
| 21 | 10 |
| 25 | 10 |

Température colonne: 40°C
Débit: 0,5 ml/min
Détection: λ = 220 nm

### Méthode F

Eluant A: H₂O + TFA 0,005%
Eluant B: CH₃CN
Gradient:

| Temps (min) | % B |
|---|---|
| 0 | 10 |
| 18 | 60 |
| 20 | 60 |
| 21 | 10 |
| 25 | 10 |

Température colonne: non contrôlée
Débit: 0,3 ml/min
Détection: λ = 220 nm

### Méthode G

Eluant A: AcONH₄ 5mM à pH 6,5
Eluant B: CH₃CN
Gradient:

| Temps (min) | % B |
|---|---|
| 0 | 40 |
| 25 | 40 |

Température colonne: non contrôlée
Débit: 0,3 ml/min
Détection: λ = 220 nm

### Méthode H

Eluant A: H₂O + TFA 0,05%
Eluant B: CH₃CN + TFA 0,035%
Gradient:

| Temps (min) | % B |
|---|---|
| 0 | 10 |
| 22 | 90 |
| 29 | 90 |
| 30 | 10 |
| 40 | 10 |

Température colonne: 40°C
Débit: 0,3 ml/min
Détection: λ = 220 nm

### Méthode I

Eluant A: H₂O + TFA 0,01 %
Eluant B: CH₃CN
Gradient:

| Temps (min) | % B |
|---|---|
| 0 | 2 |
| 10 | 95 |
| 15 | 95 |
| 16 | 2 |
| 20 | 2 |

Température colonne: 40°C
Débit: 0,5 ml/min
Détection: λ = 220 nm

Les colonnes utilisées sont des C18 avec une granulométrie entre 2 et 5 µm de préférence de 3,5 µm.

Conditions de spectrométrie de masse

L'enregistrement des spectres de masse est effectué en mode électrospray (ESI) positif ou négatif, afin d'observer les ions issus de la protonation de composés analysés (MH⁺ ou MH⁻), ou de la formation d'adduits avec d'autres cations tels que Na⁺, K⁺, etc...

La chromatographie en couche mince a été effectuée sur des plaques CCM de gel de silice Merck Silica gel 60. Le gel de silice pour la chromatographie sur colonne flash est commercialisé par Biotage ou Supelco.

Tous les solvants utilisés sont de pureté "reagent grade" ou "HPLC grade".

### Préparation 1

### (3R,5S)-3,5-Diméthyl-1-(5-trifluorométhyl-pyridin-2-yl)-pipérazine

On charge 0,8 g de 2-chloro-5(trifluorométhyl)pyridine (composé de formule (VII)), 0,5g de cis-2,6-diméthyl pipérazine (composé de formule (VI)), 0,67 g de carbonate de potassium et 0,3 g de Nal dans 8 mL de DMF. La réaction est conduite dans un initiateur à micro-ondes CEMdiscover pendant 30 mn à 160°C. Puis, on verse dans une solution aqueuse saturée en chlorure de sodium et on extrait avec de l'acétate d'éthyle. La phase organique est séchée sur Na₂SO₄, filtrée et évaporée sous vide. On isole 1,1 g d'une matière huileuse correspondant au produit du titre.

### Préparation 2

### (3R,5S)-2,5-Diméthyl-1-(5-trifluorométhyl-pyridin-2-yl)-pipérazine

En opérant comme décrit dans la préparation 1, mais en utilisant la 2,5-trans-diméthyl-pipérazine au lieu de la cis-2,6-diméthyl pipérazine, on obtient le composé du titre sous forme d'une matière huileuse.

### Préparation 3

### Chlorhydrate de 2-[8-(5-fluoro-pyrimidin-2-yl)-3,8-diaza-bicyclo[3.2.1]octane

On charge 1,44 g de 2-chloro-5-fluoropyrimidine (composé de formule (VII)), 2,2 g de 1-benzyl-3,8-diaza-bicyclo[3.2.1]octane (composé de formule (VI)), 1,7 g de carbonate de potassium et 0,73 g de Nal dans 27 mL de N-méthylpyrrolidone. On chauffe à 110°C pendant 5 heures. Puis on verse dans une solution aqueuse saturée en chlorure de sodium et on extrait avec de l'acétate d'éthyle. La phase organique est séchée sur Na₂SO₄, filtrée et évaporée sous vide. On isole 3,2 g d'une matière huileuse que l'on purifie par flash chromatographie sur colonne Biotage®, au moyen de l'éluant cyclohexane 95/acétate d'éthyle 5. On isole 1,4 g de solide blanc que l'on dissous dans 35 ml de 1,2-dichloroéthane. On ajoute à 0°C 0,72 ml de 1-chloroéthylchloroformate et laisse agiter sous flux d'azote pendant 10 minutes à 0°C et ensuite 3 heures à 85°C. On évapore le solvant et on ajoute 35 ml de méthanol. On chauffe pendant 30 minutes à la température de reflux. On évapore le solvant et on traite le résidu avec de l'isopropanol. On obtient un solide blanc que l'on filtre et on isole 900 mg de produit du titre. Pf 236-239°C

### Préparation 4

### (3R,5S)-3,5-Diméthyl-1-(6-trifluorométhyl-pyridin-2-yl)-pipérazine

On charge 2,2 g de 2-trifluorométhyl-5-bromo-pyridine (composé de formule (VII)), 1,1g de cis-2,6-diméthyl pipérazine (composé de formule (VI)), 0,22 g de palladium acétate, 0,28 g de sodium t-butoxyde et 1,3 g de tri-t-butyl phosphine dans 16 mL de o-xilène. On chauffe à 120°C pendant 6 heures. On filtre sur célyte et on évapore le solvant. On isole 1,8 g d'une matière huileuse correspondant au produit du titre.

### Préparation 5

### Chlorhydrate du méthyl ester de l'acide 3,8-diaza-bicyclo[3.2.1]oct-8-yl-nicotinique

On charge 0,42 g de 6-chloronicotinate de méthyle(composé de formule (VII)), 0,5g de 1-benzyl-3,8-diaza-bicyclo[3.2.1]octane (composé de formule (VI)), 0,4 g de carbonate de potassium et 0,17 g de Nal dans 7 mL de N-méthylpyrrolidone. On chauffe pendant 7 heures à 110°C. Puis on verse dans une solution aqueuse saturée en chlorure de sodium et on extrait avec de l'acétate d'éthyle. La phase organique est séchée sur Na₂SO₄, filtrée et évaporée sous vide. On isole 1,1 g d'une matière huileuse que l'on purifie par flash chromatographie sur colonne Biotage®, au moyen de l'éluant cyclohexane 8/acétate d'éthyle 2. On isole 520 mg d'une huile claire. On hydrogène à 40°C sous pression atmosphérique pendant 2 heures le produit obtenu à l'étape précédente, dans 20 mL d'éthanol, 2 mL d'isopropanol/HCl, en présence de 0,22 g de Pd/C à 10%. On filtre, on évapore sous vide et on isole 440 mg du produit du titre, sous forme de solide blanc correspondent au produit du titre.

### Préparation 6

### Chlorhydrate du 1-(5-trifluorométhyl-pyridin-2-yl)-pipérazine-2-one

On chauffe à 135° C pendant 6 heures dans un ballon, 10 g de 2-chloro-5-(trifluorométhyl)-pyridine et 40,5 ml de N-benzyléthylèndiamine. On verse dans l'eau et on extrait avec de l'acétate d'éthyle. On sèche et on évapore sous vide ; le brut ainsi obtenu est purifié par flash chromatographie. Le produit isolé (composé de formule (VIII)), 14 g, est solubilisé dans 200 ml d'une solution 2N de HCl. On ajoute 30 g de dihydrate de glyoxal trimérique et laisse agiter à température ambiante pendant 72 heures. On extrait avec de l'acétate d'éthyle. On sèche et évapore sous vide; le brut ainsi obtenu est purifié par flash chromatographie. Le produit isolé, 10 g, est solubilisé dans 450 ml d'éthanol, puis on ajoute 15 ml d'une solution d'isopropanol saturé avec HCl et 3 g de Pd/C à 10%. On laisse à réagir sous flux d'hydrogène pendant 4 heures à la température de 40°C. On filtre et on évapore sous vide et on obtient 3 g du composé du titre Pf 205-207°C.

### Préparation 7

### 2-Chloro-1-(2-phényl-6,7-dihydro-4H-thiazolo[4,5-c]pyridin-5-yl)-éthanone

### Etape a) préparation de la 1-(t-butoxycarboyl)-4-(7-benzo(b)thiophène)-4-hydroxy pipéridine:

On dissous dans 135 ml de THF, 45 g de 7-bromobenzothiophène et la solution ainsi obtenu est ajouté goutte à goutte à une suspension de 5,13 g de Magnesium dans 10 ml de THF dans un ballon sous azote. On ajoute de l'iode en quantité catalytique et on chauffe à la température de reflux pendant 3 heures. On refroidit à température ambiante et on ajoute une solution de 36 g de 1-(t-butoxycarbonyl)-4-pipéridone dans 80 ml de THF. On agite pendant 3 heures à température ambiante et on ajoute une solution saturée du chlorure d'ammonium.

On extrait avec de l'acétate d'éthyle. On sèche et on évapore sous vide ; le brut ainsi obtenu est purifié par flash chromatographie au moyen de l'éluant hexane 95/acétate d'éthyle 5. On isole 47,5 g d'un solide blanc avec un point de fusion de 130-131°C.

### Etape b) préparation du chlorhydrate de la 4-(7-benzo(b)thiophène)-4-hydroxy pipéridine:

On dissous dans 650 ml d'acétate d'éthyle 40 g du produit de l'étape a). On ajoute lentement 88 ml de HCl 37% et on agite à température ambiante pendant 30 minutes. On évapore les solvants et le résidu est traité avec de l'acétone. On filtre et on obtient 34 g d'un solide blanc avec un point de fusion de 232-233°C.

### Etape c) préparation du chlorhydrate de la 4-(7-benzo(b)thiophène)-1,2,3,6-tétrahydropyridine :

On dissous dans 437 ml d'acide acétique 34 g du produit de l'étape b).

On ajoute 20 ml d'acide sulphorique 96% et on chauffe à la température de 60°C pendant 2 heures. On verse dans un mélange eau/glace et on rend le pH basique avec de la soude 40%. On extrait avec de l'acétate d'éthyle. On sèche et on évapore sous vide et on obtient 30 g de produit sous forme d'une matière huileuse. On obtient la formation du chlorhydrate dans de l'isopropanol à l'aide d'une solution d'isopropanol saturé avec du HCl. On filtre on obtient 25,8 g d'un solide blanc avec un point de fusion de 226-227°C.

### Etape d) préparation du 2-Chloro-1-(2-phényl-6,7-dihydro-4H-thiazolo[4,5-c]pyridin-5-yl)-éthanone :

Dans un ballon muni d'un agitateur magnétique, on met en suspension 2,8 g du produit de l'étape c) dans 50 mL de dichlorométhane. On ajoute 2,8 mL de triéthylamine et on porte à 0°C. A 0°C, on coule goutte à goutte 1,5 mL de chloroacétylchlorure, c'est-à-dire le composé de formule générale (V) dans laquelle Hal=Hal'=Cl et n=1. On laisse réagir pendant 1 heure et demie et on verse dans de l'eau. On extrait au dichlorométhane. La phase organique est séchée sur Na₂SO₄, filtrée et évaporée sous vide. On isole 4,1 g d'une matiere huileuse que l'on purifie par flash chromatographie sur colonne Biotage®, au moyen de l'éluant cyclohexane 9/acétate d'éthyle 1. On isole 1,1 mg du produit du titre sous forme d'une huile claire.

### Préparation 8

### 1-(4-Benzofuran-7-yl-3,6-dihydro-2H-pyridin-1-yl)-2-chloro-éthanone

En opérant comme décrit dans la préparation 1, mais en utilisant le 7-bromobenzofurane au lieu du 7-bromo benzothiophène, on obtient le composé du titre.

### Préparation 9

### 1-(5-Benzofuran-7-yl-3,6-dihydro-2H-pyridin-1-yl)-2-chloro-éthanone

En opérant comme décrit dans la préparation 1, mais en utilisant le 7-bromobenzofurane au lieu du 7-bromo benzothiophène et le 1-(t-butoxycarbonyl)-3-pipéridone au lieu du 1-(t-butoxycarbonyl)-4-pipéridone, on obtient le composé du titre.

### Préparation 10

### Méthyl ester de l'acide5-[1-(2-Chloro-acétyl)-1,2,3,6-tétrahydro-pyridin-4-yl]-benzo[b]thiophène-3-carboxylique

### Etape a) préparation du tert-butyl ester de l'acide 4-(2-Méthoxycarbonyl-benzo[b]thiophèn-5-yl)-3,6-dihydro-2H-pyridine-1-carboxylique :

On charge dans un ballon sous flux d'azote 1,1 g de tert-butyl ester de l'acide 4-(4,4,5,5-Tétraméthyl-[1,3,2]dioxaborolan-2-yl)-3,6-dihydro-2H-pyridine-1-carboxylique (pureté 70%), 0,7 g du méthyl ester de l'acide 5-Bromo-benzo[b]thiophène-2-carboxylique, 0,15 g de PalladiumTetrakis (PdP(Ph3)4), 5,5 ml d'une solution de carbonate de sodium 2M, 0,42 g de chlorure de litium, et 30 ml de DME. On chauffe à la température de reflux pendant 3 heures.On évapore le solvant et on dissous dans 35 ml d'acétate d'éthyle. On lave avec une solution de carbonate de sodium 2M. La phase organique est séchée sur Na₂SO₄, filtrée et évaporée sous vide. On isole 1,6 g d'une matiere huileuse que l'on purifie par flash chromatographie sur colonne Biotage®, au moyen de l'éluant cyclohexane 98/acétate d'éthyle 2. On isole 0,45 g de solide jaunâtre.

### Etape b) préparation du chlorhydrate de méthyl ester de l'acide 5-(1,2,3,6-tétrahydro-pyridin-4-yl)-benzo[b]thiophène-2-carboxylique) :

On dissous dans 25 ml d'acétate d'éthyle 0,45 g du produit de l'étape a). On ajoute lentement 50 ml d'une solution d'acétate d'éthyl saturé avec HCl .et on agite à température ambiante pendant 3 heures. On évapore les solvants et le résidu est traité avec de l'acétone. On filtre et on obtient 0,35 g d'un solide blanchâtre.

### Etape c) préparation du Méthyl ester de l'acide-5-[1-(2-Chloro-acétyl)-1,2,3,6-tétrahydro-pyridin-4-yl]-benzo[b]thiophène-3-carboxylique)

En opérant comme décrit dans l'étape d) de la préparation 7, mais en utilisant le produit de l'étape b) au lieu du produit de l'étape c) de la préparation 7 on obtient 0,4 g du produit du titre sous forme d'une huile claire.

### Préparation 11

### 2-Chloro-1-(4-quinolin-8-yl-3,6-dihydro-2H-pyridin-1-yl)-éthanone

En opérant comme décrit dans la préparation 10, mais en utilisant le 8-bromoquinoléine au lieu du méthyl ester de l'acide 5-Bromo-benzo[b]thiophène-2-carboxylique, on obtient le composé du titre.

### Préparation 12

### 2-Chloro-1-[4-(2,3-dihydro-benzo[1,4]dioxin-6-yl)-3,6-dihydro-2H-pyridin-1-yl]-éthanone

En opérant comme décrit dans la préparation 1, mais en utilisant le 6-bromobenzodioxane au lieu du 7-bromo benzothiophène on obtient le composé du titre.

### Préparation 13

### 1-(3-Benzofuran-7-yl-2,5-dihydro-pyrrol-1-yl)-2-chloro-éthanone

En opérant comme décrit dans la préparation 7, mais en utilisant le 1-(t-butoxycarbonyl)-3-pyrrolidone au lieu du 1-(t-butoxycarbonyl)-4-pipéridone et le le 7-bromobenzofurane au lieu du 7-bromo benzothiophène, on obtient le composé du titre.

### Préparation 14

### Methyl ester de l'acide 6-((3S,5R)-3,5-Dimethyl-piperazin-1-yl)-nicotinique

En opérant comme décrit dans la préparation 1 mais en utilisant le méthyl ester de l'acide 6-chloro-nicotinique au lieu de la 2-trifluorométhyl-5-bromo-pyridine on obtient le composé du titre sous forme d'un huile claire.

### Préparation 15

### Chlorhydrate du 3-[6-(3,8-Diaza-bicyclo[3.2.1]oct-8-yl)-pyridin-3-yl]-4H-[1,2,4]oxadiazol-5-one

### Etape a) Chlorhydrate du 6-(3,8-Diaza-bicyclo[3.2.1]oct-8-yl)-nicotinonitrile

En opérant comme décrit dans la préparation 3 mais en utilisant le 6-Chloro-nicotinonitrile au lieu de la 2-chloro-5-fluoropyrimidine on obtient le composé du titre sous forme d'un solid blanc.

### Etape b) Tert-butyl ester de l'acide 8-(5-Cyano-pyridin-2-yl)-3,8-diaza-bicyclo[3.2.1]octane-3-carboxylique

On dissous dans 83 ml de DMF 7 g du produit de l'étape a). On ajoute 11,7 ml de triéthylamine. On ajoute à 0°C 6,7g de (BOC)₂O et on agite à température ambiante pendant 1 heure. On évapore sous vide. On lave avec l'eau et on extrait avec de l'acétate d'éthyle. La phase organique est séchée sur Na₂SO₄, filtrée et évaporée sous vide. On isole 12 g d'une matiere huileuse que l'on purifie par flash chromatographie sur colonne Biotage® automatique, au moyen de l'éluant cyclohexane 8/acétate d'éthyle 2 en gradient jusqu'à acétate d'éthyle 100%. On isole 7,6 g du composé du titre sous forme d'un solid blanc.

### Etape c) Tert-butyl ester de l'acide 8-[5-(N-Hydroxycarbamimidoyl)-pyridin-2-yl]-3,8-diaza-bicyclo[3.2.1]octane-3-carboxylique

On dissous le produit de l'étape b) (7,6 g) dans 75 ml d'éthanol et on ajoute une solution de 3,36 g d'hydroxylamine chlorhydrate dissous dans 38 ml d'eau puis 5 g de carbonate de sodium. On agite à 90°C pendant 4 heures. On laisse refroidir et on filtre. On obtient 8 g du composé du titre sous forme d'un solide blanc.

### Etape d) Tert-butyl ester de l'acide 8-[5-(5-Oxo-4,5-dihydro-[1,2,4]oxadiazol-3-yl)-pyridin-2-yl]-3,8-diaza-bicyclo[3.2.1]octane-3-carboxylique

On charge dans un ballon sous flux d'azote 1 g de composé de l'étape c), on ajoute 10mL de DMF et 0,3mL de pyridine. On ajoute à 0°C 0,23 ml de méthylchloroformate et on agite à température ambiante pendant 3 heures. On lave avec l'eau et on extrait avec de l'acétate d'éthyle. La phase organique est séchée sur Na₂SO₄, filtrée et évaporée sous vide. On ajoute 30 ml de toluène et on agite à reflux pendant 4 heures. On évapore le solvant et on obtient 0,8 g du composé du titre.

### Etape e) Chlorhydrate du 3-[6-(3,8-Diaza-bicyclo[3.2.1]oct-8-yl)-pyridin-3-yl]-4H-[1,2,4]oxadiazol-5-one

On dissous le produit dans 20 ml d'acétate d'éthyle, on ajoute une solution d'acétate d'éthyle saturée avec du HCl et on agite à température ambiante pendant 2 heures. On évapore le solvant et le résidu est traité avec de l'isopropanol. On filtre et on obtient 0,65 g du composé du titre sous forme d'un solide jaune.

### Préparation 16

### Acide [(2R,6S)-2,6-Dimethyl-4-(5-trifluoromethyl-pyridin-2-yl)-piperazin-1-yl]-acetique

### Etape a) Ethyl ester de l'acide [(2R,6S)-2,6-Dimethyl-4-(5-trifluoromethyl-pyridin-2-yl)-piperazin-1-yl]-acétique

Dans un ballon muni d'un agitateur magnétique, on charge : 3,6 g du produit de la préparation 1, 100 ml de THF, 1,9 ml d'éthyl-bromoacétate, 4,7 ml de triéthylamine. On laisse réagir pendant 7 heures à 80°C. On évapore le solvant, on lave avec l'éther éthylique et on filtre. On purifie l'eau de filtrage par flash chromatographie sur colonne Biotage® automatique au moyen de l'éluant cyclohexane 8/acétate d'éthyle 2 en gradient jusqu'à acétate d'éthyle 7/méthanol 3. On isole 2,5 g du produit du titre sous forme de solide.

### Etape b) Acide [(2R,6S)-2,6-Dimethyl-4-(5-trifluoromethyl-pyridin-2-yl)-piperazin-1-yl]-acetique

2,5 g de produit de l'étape précedente sont solubilisés dans 22 ml d'éthanol, puis on ajoute 5 ml d'une solution acqueuse 40% de NaOH. On laisse réagir pendant 3 heures à 70°C. On règle le pH à 6 à l'aide d'une solution 1 N de HCl. On extrait à l'acetate d'éthyle. La phase organique est séchée sur Na₂SO₄, filtrée et évaporée sous vide. On isole 1,6 g du produit du titre sous forme d'un solide blanc.

### Préparation 17

### Chlorhydrate de 2-(1,2,3,6-Tetrahydro-pyridin-4-yl)-quinoline

En opérant comme décrit dans la préparation 10 mais en utilisant le quinolin-2-yl ester de l'acide trifluoro-méthanesulfonique au lieu du méthyl ester de l'acide 5-Bromo-benzo[b]thiophène-2-carboxylique on obtient le composé du titre sous forme d'un solide blanc.

### Préparation 18

### (3S,5R)-3,5-Dimethyl-1-(5-thiazol-2-yl-pyridin-2-yl)-piperazine

### Etape a) (3S,5R)-3,5-Diméthyl-1-(5-Iodo-2-yl-pyridin-2-yl)-pipérazine

En opérant comme décrit dans la préparation 1, mais en utilisant le 2-fluoro-5-iodo-pyridine au lieu du 2-chloro-5(trifluorométhyl)-pyridine, on obtient le composé du titre sous forme d'huile.

### Etape b) Tert-butyl ester de l'acide (2S,6R)-2,6-Diméthyl-4-(5-iodo-2-yl-pyridin-2-yl)-piperazine-1-carboxylique

On charge sous flux d'azote à 0°C : 0,35 g de composé de l'étape a), 0,26 g de (Boc)₂O, 0,46 mL de tryéthylamine et 5 ml de DMF. On chauffe à la température de 140°C pendant 4 heures. On évapore le solvant. On isole 0,49 g de brut. Le résidu est purifié par flash chromatographie sur colonne Biotage®, au moyen de l'éluant éthyle acétate. On isole 0,43 g du composé du titre sous forme d'une huile jaune pâle.

### Etape c) Tert-butyl ester de l'acide (2S,6R)-2,6-Dimethyl-4-[5-(4,4,5,5-tetramethyl-[1,3,2]dioxaborolan-2-yl)-pyridin-2-yl]-piperazine-1-carboxylique

On charge dans un ballon sous flux d'azote 0,43 g composé de l'étape b), 0,29 g de bis(pinacole)diboron, 0,026 g de PalladiumCl₂ (dppf)₂·CH₂Cl₂, 0,31 g de potassium acétate, 10 ml de DMSO. On chauffe à 85°C pendant 2 heures. On verse dans une solution acqueuse saturée en NaCl et on extrait avec de l'acétate d'éthyle. La phase organique est séchée sur Na₂SO₄, filtrée et évaporée sous vide. On isole 0,32 g d'une matière huileuse que l'on purifie par flash chromatographie sur colonne Biotage®, au moyen de l'éluant cyclohexane 9/acétate d'éthyle 1. On isole 0,28 g de solide jaunâtre.

### Etape d) tert-butyl ester de l'acide 2S,6R-2,6-Dimethyl-4-(5-thiazol-2-yl-pyridin-2-yl)-piperazine-1-carboxylique

On charge dans un ballon sous flux d'azote 0,28 g de de l'étape c) 0,092 g de 2-bromothyazole, 0,032 g de PalladiumTetrakis (PdP(Ph3)4), 0,094 g de bicarbonate de sodium, 20 ml de DME et 3 ml d'eau. On chauffe à la température de reflux pendant 7 heures. On verse dans une solution acqueuse saturée en NaCl et on extrait avec de l'acétate d'éthyle. La phase organique est séchée sur Na₂SO₄, filtrée et évaporée sous vide. On isole 0,36 g d'une matière huileuse que l'on purifie par flash chromatographie sur colonne Biotage®, au moyen de l'éluant cyclohexane 9/acétate d'éthyle 1. On isole 0,2 g d'une huile jaunâtre.

### Etape e) Trifluoroacétate de (3S,5R)-3,5-Dimethyl-1-(5-thiazol-2-yl-pyridin-2-yl)-piperazine

Le composé de l'étape d) (0,2 g) est dissous lentement dans 5 ml d'acide trifluoroacétique à 0°C. On laisse ensuite sous agitation pendant 2 heures à température ambiante. On évapore sous vide l'acide trifluoroacétique et on obtient 0,15 g du composé du titre sous forme d'un solide beige.

### Préparation 19

### Acide [3-Oxo-4-(3-trifluoromethyl-phenyl)-piperazin-1-yl]-acétique

### Etape a) Benzyl ester de l'acide [3-Oxo-4-(3-trifluoromethyl-phényl)-pipérazin-1-yl]-acétique

Dans un ballon muni d'un agitateur magnétique, on charge: 1,12 g de 1-(5-Trifluoromethyl-pyridin-2-yl)-piperazin-2-one, 40 ml de THF, 0,87 ml de benzyl-bromoacétate, 1,5 ml de triéthylamine. On laisse réagir sous flux d'azote pendant une nuit à température ambiante. On évapore le solvant et on purifie par flash chromatographie sur colonne Biotage®, au moyen de l'éluant hexane 1/acétate d'éthyle 1. On isole 2,9 g du produit du titre sous forme d'un solide blanc.

### Etape b) Acide [3-Oxo-4-(3-trifluoromethyl-phenyl)-piperazin-1-yl]-acétique

1 g du produit de l'étape précédente est solubilisé dans 150 ml d'éthanol, puis on ajoute 0,15 g de Pd/C à 10%. On laisse réagir sous flux d'hydrogène pendant 4 heures à la température de 40°C. On filtre et on évapore sous vide et on obtient 0,74 g du composé du titre sous forme d'un solide blanc.

### EXEMPLE 1

### - Composé n 34 :

### 1-(4-Benzofuran-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone

On fait réagir 0,18 g du composé obtenu à la préparation 8 (composé de formule (II)), 0,17 g du composé obtenu à la préparation 1 (composé de formule (III)), 0,1 g de carbonate de potassium et 0,04 g de Nal dans 3 mL de DMF. La réaction est conduite au moyen d'un initiateur CEMdiscover à micro-ondes pendant 30 mn à 160°C. On verse dans l'eau et on extrait à l'acétate d'éthyle. La phase organique est séchée sur Na₂SO₄, filtrée et évaporée sous vide. On isole 0,36 g d'une matière huileuse. On purifie sur colonne par flash chromatographie au moyen d'une colonne Biotage® que l'on élue avec un mélange cyclohexane 6/acétate d'éthyle 4. On isole 0,180 g d'un solide jaune pâle que l'on crystalise avec de l'éther éthylique. On filtre et on obtient 0,08 g du produit du titre sous forme d'un solide blanc.
Pf : (138-139)°C
RMN Appareil b). δ (ppm, dmso-d6): 1,06 (m, 6H); 2,56-2,79 (m, 4H); 3,19 (m, *), 3,73 (m, 4H); 4,18 (m, 3H); 4,31 (m, 1H); 6,57 (m, 1H); 6,95 (d, J= 9,2Hz, 1H); 6,99 (d, J= 1,8Hz, 1 H); 7,27 (m, 2H); 7,58 (d, J= 7,2Hz, 1 H); 7,76 (dd, J= 9,2 et 2,2Hz, 1 H); 8,01 (d, J= 1,7Hz, 1 H); 8,39 (bs, 1 H).

### EXEMPLE 2

### - Composé n°1 :

### 1-(4-Benzo[b]thiophèn-7-yl-3,6-dihydro-2H-pyridin-1-yl)-2-[(2S,6R)-2,6-diméthyl-4-(5-trifluorométhyl-pyridin-2-yl)-piperazin-1-yl]-éthanone et son oxalate

En opérant comme décrit dans l'exemple 1, mais en utilisant le composé de la préparation 7 au lieu du composé de la préparation 8, on obtient le composé du titre.

On dissous dans de l'acétone et on ajoute une solution d'acide oxalique dans l'acétone et on obtient l'oxalate sous forme d'un solide blanc.
Pf: (60-61)°C
RMN :(Appareil b). δ (ppm, dmso-d6): 1,17 (m, 6H), 2,60 + 2,68 (2 x m, 2H); 2,97 (m, *); 3,36 (m, *); 3,76 (m, *); 4,00 (m, *); 4,14 - 4,43 (m, *); 6,30 (bs, 1 H); 7,03 (d, J= 9,0Hz, 1 H); 7,32 (d, J= 7.1 Hz, 1 H); 7,42 (t, J= 7,6Hz, 1 H); 7,51 (d, J= 5.3Hz, 1 H); 7,77 (d, J= 5,5Hz, 1 H); 7,83 (m, 2H); 8,43 (bs, 1 H).

### EXEMPLE 3

### - Composé n 14 :

### 1-(4-Benzofuran-7-yl-3,6-dihydro-2H-pyridin-1-yl)-2-[(2R,5S)-2,5-diméthyl-4-(5-trifluorométhyl-pyridin-2-yl)-pipérazin-1-yl]-éthanone et son oxalate

En opérant comme décrit dans l'exemple 1, mais en utilisant le composé de la préparation 2 au lieu du composé de la préparation 1 et le composé de la préparation 8 au lieu du composé de la préparation 7, on obtient le composé du titre.

On dissous dans de l'acétone et on ajoute une solution d'acide oxalique dans l'acétone et on obtient l'oxalate sous forme d'un solide blanc.
Pf :(130-131)°C
RMN (Appareil b). δ (ppm, dmso-d6): 1,02 (m, 3H); 1,15 - 1,32 (m, 3H); 2,64 (m, 2H); 2,78 (m, 1 H); 2,92 (m, 1 H); 2,90 (m, 1 H); 3,26 (m, *); 3,34 - 3,67 (m, *); 3,66 -3,99 (m, *); 4,07 - 4,54 (m, *); 4,64 (m, *); 6,58 (m, 1 H); 6,90 (m, 1 H); 6,98 (d, J= 1,9Hz, 1 H); 7,20 - 7,33 (m, 2H); 7,58 (d, J= 7,2Hz, 1 H); 7,76 (m, 1 H); 8,00 (d, J= 1,8Hz, 1 H); 8,39 (m, 1 H).

### EXEMPLE 4

### - Composé n°69 :

### Méthyl ester de l'acide 6-{3-[2-(4-Benzo[b]thiophèn-7-yl-3,6-dihydro-2H-pyridin-1-yl)-2-oxo-éthyl]-3,8-diaza-bicyclo[3.2.1]oct-8-yl}-nicotinique

On met à réagir 0,47 g du composé obtenu à la préparation 7 (composé de formule (II)), 0,45 g du composé obtenu à la préparation 5 (composé de formule (III)), 0,57 ml de diisopropyl-éthyl-amine et 18 mL de DMF. On chauffe pendant 3 heures à 100°C. On verse dans l'eau et on extrait à l'acétate d'éthyle. La phase organique est séchée sur Na₂SO₄, filtrée et évaporée sous vide. On isole 0,58 g d'une matière solide. On purifie sur colonne par flash chromatographie au moyen d'une colonne que l'on élue avec un mélange hexane 1/acétate d'éthyle 1. On isole 0,24 g du produit du titre. On traite avec du diéthyl éther, on filtre et on obtient 0,21 g d'un solide blanc.
Pf : (153-154)°C
RMN (Appareil a). δ (ppm, dmso-d6): 1,87 (m, 2H), 1,98 (m, 2H); 2,40 (m, 2H); 2,56 (m, 1 H); 2,71 (m, 3H); 3,17 + 3,20 (2 x s, 2H); 3,69 - 3,88 (m, 5H); 4,16 (s, 1 H); 4,42 (s, 1 H); 4,67 (bs, 2H); 6,29 + 6,32 (2 x m, 1 H); 6,78 (m, 1 H); 7,31 (d, J= 7,2Hz, 1 H); 7,42 (t, J= 7,6Hz, 1 H); 7,50 (d, J= 54Hz, 1 H); 7,77 (d, J=5,3Hz, 1 H); 7,82 (d, J= 7,5Hz, 1 H); 7,93 (d, J= 8,7Hz, 1 H); 8,64 (bs, 1 H).

### EXEMPLE 5

### - Composé n 36 :

### 1-(4-Benzofuran-7-yl-3,6-dihydro-2H-pyridin-1-yl)-2-[8-(5-fluoro-pyrimidin-2-yl)-3,8-diaza-bicyclo[3.2.1]oct-3-yl]-éthanone

En opérant comme décrit dans l'exemple 4, mais en utilisant le composé de la préparation 8 au lieu du composé de la préparation 7 et le composé préparation 4 au lieu du composé de la préparation 5 on obtient le composé du titre sous forme d'un solide blanc.
Pf : (174-175)°C
RMN (Appareil a). δ (ppm, dmso-d6): 1,71 - 2,03 (m, 4H); 2,39 (m, 2H); 2,57-2,80 (m, 4H); 3,16 + 3,19 (2 x s, 2H); 3,73 + 3,82 (2 x m, 2H); 4,17 (bs, 1H); 4,42 (bs, 1 H); 4,60 (m, 2H); 6,56 + 6,59 (2 x m, 1 H); 6,98 (d, J= 2,3Hz, 1 H); 7,27 (m, 2H); 7,58 (dd, J= 7,35 et 1,8Hz, 1 H); 8,01 (d, J= 2,1 Hz, 1 H); 8,44 (m, 2H).

### EXEMPLE 6

### - Composé n°71 :

### Acide 6-{3-[2-(4-Benzo[b]thiophèn-7-yl-3,6-dihydro-2H-pyridin-1-yl)-2-oxo-éthyl]-3,8-diaza-bicyclo[3.2.1]oct-8-yl}-nicotinique

On dissous 0,147 g du composé de l'exemple 4 dans 2 ml de solution aqueuse de HCl 3N. On chauffe à la température de reflux pendant 2 heures. On ajoute 2 ml de solution aqueuse de HCl 3N. On chauffe à la température de reflux pendant 1 heure. On lave avec de l'éther éthylique. On ajuste le pH à 6 avec une solution de NaHCO3 et on extrait avec de l'acétate d'éthyle. On sèche et on évapore la phase organique et on obtient 200 mg d'une matière hulieuse. On traite avec du diéthyl éther, on filtre et on obtient 0,015 g d'un solide jaune pâle correspondant au produit du titre.
Pf : (121-122)°C
RMN (Appareil b). δ (ppm, dmso-d6): 1,75 - 2,06 (m, 4H); 2,41 (m, 2H); 2,56 (m, **); 2,70 (m, 3H); 3,12 - 3,33 (m, *); 3,68 - 3,90 (m, 2H); 4,16 (bs, 1H); 4,42 (bs, 1H); 4,66 (bs, 2H); 6,29 (m, 1H); 6,78 (m, 1H); 7,31 (d, J= 7Hz, 1H); 7,42 (dd → t, J= ∼8Hz, 1 H); 7,50 (d, J= 5,6Hz, 1 H); 7,77 (d, J= 5,4Hz, 1 H); 7,82 (d, J= 8Hz, 1 H); 7,91 (m, 1 H); 8,63 (bs, 1 H); 11,72 - 12,49 (bs, 1 H).

### EXEMPLE 7

### - Composé n°32 :

### 4-[2-(4-Benzofuran-7-yl-3,6-dihydro-2H-pyridin-1-yl)-2-oxo-éthyl]-1-(5-trifluorométhyl-pyridin-2-yl)-pipérazin-2-one

En opérant comme décrit dans l'exemple 4, mais en utilisant le composé de la préparation 8 au lieu du composé de la préparation 7 et le composé de la préparation 6 au lieu du composé de la préparation 5 on obtient le composé du titre sous forme d'un solide blanc.
Pf :(151-152)°C
RMN (Appareil a). δ (ppm, dmso-d6): 2,64 (m, 0,9H); 2,73 (m, 1,1 H); 2,99 (m, 2H); 3,51 (s, 3H); 3,77 (m, 2H); 3,98 (m, 2H); 4,22 (s, 1,1 H); 4,33 (s, 0,9H); 6,57 (bs, 1 H); 6,98 (d, J=2,2Hz, 1H); 7,22 - 7,34 (m, 2H); 7,58 (bd, J= 7,5Hz, 1H); 7,99 (bs, 1 H); 8,22 (m, 2H); 8,84 (m, 1 H).

### EXEMPLE 8

### - Composé n°20 :

### 1-(4-Quinolin-8-yl-3,6-dihydro-2H-pyridin-1-yl)-2-[4-(5-trifluorométhyl-pyridin-2-yl)-pipérazin-1-yl]-éthanone et son oxalate

En opérant comme décrit dans l'exemple 4, mais en utilisant le composé de la préparation 11 au lieu du composé de la préparation 7 et la (5-trifluorométhyl-pyridin-2-yl)-pipérazine au lieu du composé de la préparation 5 on obtient le composé du titre.

On dissous dans de l'acétone et on ajoute une solution d'acide oxalique dans l'acétone et on obtient l'oxalate sous forme d'un solide amorphe beige.

RMN (Appareil b). δ (ppm, dmso-d6): 2,77 + 2,87 (2 x m, 2H); 3,02 (m, 4H); 3,53 - 3,98 (m, *); 4,22 (m, 2H); 5,96 (m, 1 H); 7,03 (d, J= 9,1 Hz, 1 H); 7,50 - 7,65 (m, 3H); 7,86 (dd, J= 9,0 et 2,1 Hz, 1 H); 7,92 (m, 1 H); 8,38 (dd, J= 8,2 et 1,8 Hz, 1 H); 8,46 (bs, 1H); 8,90 (m, 1 H).

### EXEMPLE 9

### - Composé n°45 :

### 1-[4-(2,3-Dihydro-benzo[1,4]dioxin-6-yl)-3,6-dihydro-2H-pyridin-1-yl]-2-[(2S,6R)-2,6-diméthyl-4-(6-trifluorométhyl-pyridin-3-yl)-pipérazin-1-yl]-éthanone

En opérant comme décrit dans l'exemple 1, mais en utilisant le composé de la préparation 12 au lieu du composé de la préparation 7 on obtient le composé du titre sous forme d'un solide blanc.
Pf :(200-201)°C
RMN (Appareil a). δ (ppm, dmso-d6): 1,05 (m, 6H); 2,39 + 2,50 (2 x m, **); 2,60 (m, 2H); 3,21 - 3,32 (m, *); 3,56 - 3,73 (m, 4H); 3,77 (m, 2H); 4,05 + 4,18 (2 x m, 2H); 4,24 (s, 4H); 6,05 (m, 1 H); 6,82 (d, J= 8,4Hz, 1 H); 6,89 - 6,96 (m, 2H); 7,39 (dd, J= 8,8 et 2,6Hz, 1 H); 7,60 (d, J= 8,8Hz, 1 H); 8,39 (d, J= 2,7Hz, 1 H).

### EXEMPLE 10

### - Composé n°63 :

### 1-(5-Benzofuran-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone

En opérant comme décrit dans l'exemple 1, mais en utilisant le composé de la préparation 9 au lieu du composé de la préparation 7 on obtient le composé du titre sous forme d'un solide blanc.
Pf :(129-130)°C
RMN (Appareil a). δ (ppm, dmso-d6): 1,06 (m, 6H); 2,33 + 2,44 (2 x m, 2H); 2,71 (m, 2H); 3,19 (m, *); 3,68 (m, 2H); 3,73 (s, 2H); 4,17 (m, 2H); 4,47 + 4,58 (2 x bs, 2H); 6,63 (bs, 1 H); 6,94 (m, 1H); 6,70 (bs, 1H); 7,22 - 7,39 (m, 2H); 7,59 (d, J= 7,4Hz, 1 H); 7,75 (bd, J= 9,0Hz, 1 H); 8,02 (bs, 1 H); 8,38 (bs, 1 H).

### EXEMPLE 11

### - Composé n°67 :

### Méthyl ester de l'acide 5-(1-{2-[(2S,6R)-2,6-Diméthyl-4-(5-trifluorométhyl-pyridin-2-yl)-pipérazin-1-yl]-acétyl}-1,2,3,6-tétrahydro-pyridin-4-yl)-benzo[b] thiophène -2-carboxylique et son oxalate

En opérant comme décrit dans l'exemple 1, mais en utilisant le composé de la préparation 10 au lieu du composé de la préparation 7 on obtient le composé du titre.

On le dissous dans de l'acétone et on ajoute une solution d'acide oxalique dans l'acétone et on obtient l'oxalate sous forme d'un solide amorphe beige.
RMN (Appareil b). δ (ppm, dmso-d6): 1,15 (m, 6H); 2,58 + 2,68 (2 x m, 2H); 2,95 (m, 2H); 3,35 (m, 2H); 3,74 (m, *); 3,82 - 4,08 (m, *); 4,12 - 4,40 (m, *); 6,31 (m, 1 H); 7,03 (d, J= 9,0Hz, 1 H); 7,70 (m, 1 H); 7,83 (bd, J= 9,1 Hz, 1 H); 8,01 - 8,11 (m, 2H); 8,20 (bs, 1 H); 8,43 (bs, 1 H).

### EXEMPLE 12

### - Composé n°83 :

### 4-[2-(3-Benzofuran-7-yl-2,5-dihydro-pyrrol-1-yl)-2-oxo-éthyl]-1-(5-trifluorométhyl-pyridin-2-yl)-piperazin-2-one

En opérant comme décrit dans l'exemple 4, mais en utilisant le composé de la préparation 13 au lieu du composé de la préparation 7 et le composé de la préparation 6 au lieu du composé de la préparation 5 on obtient le composé du titre sous forme d'un solide blanc.
Pf : (184-186)°C
RMN (Appareil b). δ (ppm, dmso-d6): 2,89 - 3,12 (m, 4H); 3,45 - 3,59 (m, 4H); 3,80 - 4,02 (m, 4H); 4,74 + 4,83 (2 x s, 2H); 7,51 (m, 3H); 8,20 (m, 2H), 8,37 (m, 2H); 8,65 - 8,88 (m, 2H).

### EXEMPLE 13

### - Composé n°89:

### 3-(6-{3-[2-(4-Benzofuran-7-yl-3,6-dihydro-2H-pyridin-1-yl)-2-oxo-ethyl]-3,8-diaza-bicyclo[3.2.1]oct-8-yl}-pyridin-3-yl)-4H-[1,2,4]oxadiazol-5-one ;

En opérant comme décrit dans l'exemple 4, mais en utilisant le composé de la préparation 8 au lieu du composé de la préparation 7 et le composé de la préparation 15 au lieu du composé de la préparation 5 on obtient le composé du titre sous forme d'un solide blanc.
Pf : (230-232)°C
RMN :(Temp. B). δ (ppm, dmso-d6): 1.76 - 2.04 (m, 4H), 2.41 (m, 2H), 2.56-2.81 (m, 4H), 3.17 + 3.21 (2Xs, 2H), 3.73 (m, 1H), 3.81 (m, 1H), 4.17 (m, 1H), 4.42 (m, 1 H), 4.64 (m, 2H), 6.56 (m, 0.5H), 6.61 (m, 0.5H), 6.89 (m, 1H), 6.99 (d, J= 2.2Hz, 1 H), 7.24 - 7.32 (m, 2H), 7.58 (dd, J= 7.3 et 1.4Hz, 1 H), 7.82 (m, 1 H), 8.02 (m, 1 H), 8.48 (m, 1 H), 12.69 (bs, 1 H).

### EXEMPLE 14

### - Composé n°51:

### Methyl ester de l'acide 6-{(3R,5S)-4-[2-(4-Benzofuran-7-yl-3,6-dihydro-2H-pyridin-1-yl)-2-oxo-ethyl]-3,5-dimethyl-piperazin-1-yl}-nicotinique

En opérant comme décrit dans l'exemple 1, mais en utilisant le composé de la préparation 8 au lieu du composé de la préparation 7 et le composé de la préparation 14 au lieu du composé de la préparation 1 on obtient le composé du titre.

On le dissous dans de l'acétone, on ajoute une solution d'acide oxalique dans l'acétone et on obtient l'oxalate sous forme d'un solide amorphe blanc.
RMN : (Appareil b, Temp. A). δ (ppm, dmso-d6): 1.19 (m, 6H), 2.67 + 2.76 (2Xm, 2H), 3.04 (m, **), 3.43 (m, **), 3.58- 4.58 (m, **), 6.58 (m, 1 H), 6.88 - 7.12 (m, 2H), 7.28 (m, 2H), 7.59 (m, 1 H), 7.90 - 8.12 (m, 2H), 8.68 (m, 1 H).

### EXEMPLE 15

### - Composé n°57:

### Acide 6-{(3S,5R)-4-[2-(4-Benzofuran-7-yl-3,6-dihydro-2H-pyridin-1-yl)-2-oxo-éthyl]-3,5-diméthyl-pipérazin-1-yl}-nicotinique

En opérant comme décrit dans l'exemple 6, mais en utilisant le composé de l'exemple 13 au lieu du composé de l'exemple 4 on obtient le composé du titre sous forme de matière huileuse. On le traite avec du diéthyl éther, on filtre et on obtient un solide jaune pâle correspondant au produit du titre.
Pf :(272-274)°C
RMN : (Temp. B). δ (ppm, dmso-d6): 1.06 (m, 6H), 2.55 - 2.76 (m, 4H), 3.17 (m, 2H), 3.72 (m, 4H), 4.18 (m, 3H), 3.41 (s, 1 H), 6.57 (m, 1 H), 6.81 (d, J= 9.0Hz, 1 H), 7.00 (d, J= 2.1 Hz, 1 H), 7.22 - 7.32 (m, 2H), 7.58 (d, J= 7.4Hz, 1H), 7.93 (d, J= 9.0Hz, 1 H), 8.02 (s,1 H), 8.62 (m, 1 H).

### EXEMPLE 16

### - Composé n°118

### 4-[2-Oxo-2-(4-thieno[3,2-c]pyridin-4-yl-3,6-dihydro-2H-pyridin-1-yl)-ethyl]-1-(5-trifluoromethyl-pyridin-2-yl)-piperazin-2-one

0,11 g du composé de la préparation 19 sont mis en suspension dans un ballon muni d'un agitateur magnétique, dans 13 mL de dichlorométhane. On ajoute 0,1 g de 4-(1,2,3,6-Tetrahydro-pyridin-4-yl)-thieno[3,2-c]pyridine, 0,19 mL de triéthylamine et 0,15 g de BOP. On laisse réagir pendant 1 heure à température ambiante. Puis, on verse dans l'eau et on extrait avec du dichlorométhane. La phase organique est séchée sur Na₂SO₄, filtrée et évaporée sous vide. On isole 0,22 g d'une matière hulieuse. On purifie sur colonne par flash chromatographie au moyen d'une colonne Biotage® que l'on élue avec de l'éthyl acétate. On isole 0,7 g d'un solide blanc.
Pf :(145-148)°C
RMN : (Appareil a, Temp. B). δ (ppm, dmso-d6): 2.69 (m, 0.9H), 2.79 (m, 1.1 H), 3.00 (m, 2H), 3.51 (m, 4H), 3.77 (m, 2H), 3.97 (m, 2H), 4.25 (m, 1.1 H), 4.35 (m, 0.9H), 6.34 + 6.37 (2Xm, 1 H), 7.75 (m, 1 H), 7.84 + 7.88 (2Xm, 1 H), 7.97 (m, 1 H), 8.22 (m, 2H), 8.40 (m, 1 H), 8.84 (m, 1 H).

### EXEMPLE 17

### - Composé n°119

### Oxalate du 1-(4-Benzofuran-7-yl-3,6-dihydro-2H-pyridin-1-yl)-2-[(2S,6R)-2,6-dimethyl-4-(5-thiazol-2-yl-pyridin-2-yl)-piperazin-1-yl]-ethanone

En opérant comme décrit dans l'exemple 1, mais en utilisant le composé de la préparation 8 au lieu du composé de la préparation 7 et le composé de la préparation 18 au lieu du composé de la préparation 1, on obtient le composé du titre sous forme de base libre. On le dissous dans de l'acétone puis on ajoute une solution d'acide oxalique dans l'acétone. On obtient le produit du titre sous forme de solide blanc.
Pf : (140-145)°C
RMN : (Appareil a, Temp.B). (ppm, dmso-d6): 1.21 (m, 6H), 2.67 (m, *), 2.77 (m, *), 2.91 - 3.95 (m, *), 4.04 - 4.47 (m, *), 6.59 (m, 1 H), 7.00 (d, J= 2.1 Hz, 1 H), 7.05 (m, 1 H), 7.27 (m, 1 H), 7.32 (m, 1 H), 7.59 (d, J= 7.4Hz, 1 H), 7.68 (bd, J= 3.2Hz, 1 H), 7.86 (bd, J=3.2Hz, 1 H), 8.03 (bd, 1 H), 8.08 (m, 1 H), 8.71 (bs, 1 H)

### EXEMPLE 18

### - Composé n°121

### 2-[(2S,6R)-2,6-Dimethyl-4-(5-trifluoromethyl-pyridin-2-yl)-piperazin-1-yl]-1-(4-quinolin-2-yl-3,6-dihydro-2H-pyridin-1-yl)-ethanone

En opérant comme décrit dans l'exemple 16, mais en utilisant le composé de la préparation 16 au lieu du composé de la préparation 19 et le composé de la préparation 17 au lieu de la 4-(1,2,3,6-Tetrahydro-pyridin-4-yl)-thieno[3,2-c]pyridine, on obtient le composé du titre sous forme d'un solide blanc.
Pf : (94-95)°C
RMN: (Appareil a, Temp.B). (ppm, dmso-d6): 1.06 (m, 6H), 2.66 - 2.78 (m, 3H), 2.85 (m, 1 H), 3.20 (m, 2H), 3.66- 3.79 (m, 4H), 4.20 (m, 3H), 4.35 (m, 1 H), 6.89 (m, 1 H), 6.95 (d, J= 9.1 Hz, 1 H), 7.56 (m, 1 H), 7.72 - 7.79 (m, 2H), 7.87 (d, J= 8.7Hz, 1 H), 7.92 - 7.99 (m, 2H), 8.33 (m, 1 H), 8.39 (m, 1 H).

Le tableau suivant décrit les exemples obtenus par application et/ou adaptation de méthodes décrites au moyen des réactifs et produits de départ appropriés :

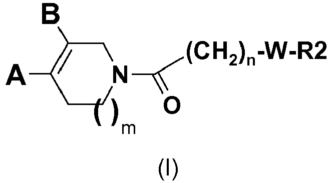

| N | **A** | **B** | **m** | **W** | **R2** | **n** | **Sel** | **PF °C** | **LCMS** |
|---|---|---|---|---|---|---|---|---|---|
| **1** | 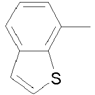 | H | 1 | 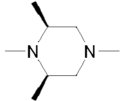 | 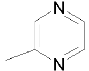 | 1 | oxalate | 60-61 | MH+ 448 r.t. 8.4' Méthode A |
| **2** | 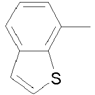 | H | 1 | 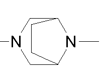 | 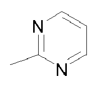 | 1 | HCl | 111-112 | MH+ 446 r.t. 8.4' Méthode A |
| **3** | 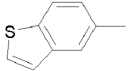 | H | 1 | 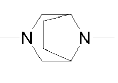 | 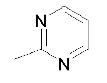 | 1 | -- | | MH+ 446 r.t. 9.5' Méthode A |
| **4** | 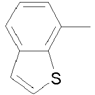 | H | 1 | 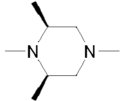 | 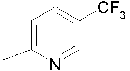 | 1 | oxalate | | MH+ 515 r.t. 19.1' Méthode A |
| **5** | 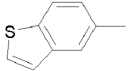 | H | 1 | 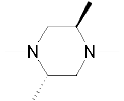 | 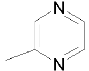 | 1 | oxalate | 101-102 | MH+ 448 r.t. 16.4' Méthode G |
| **6** | 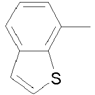 | H | 1 | 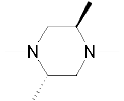 | 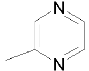 | 1 | oxalate | 96-97 | MH+ 448 r.t. 8.6' Méthode A |
| **7** | 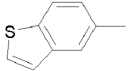 | H | 1 | 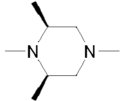 | 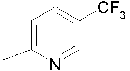 | 1 | -- | | MH+ 515 r.t. 22.7' Méthode C |
| **8** | 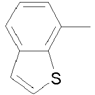 | H | 1 | 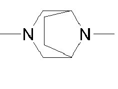 | 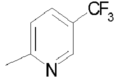 | 1 | -- | 162-163 | MH+ 513 r.t. 14.6' Méthode B |
| **9** | 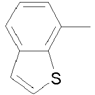 | H | 1 | 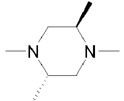 | 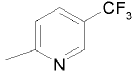 | 1 | oxalate | 107-108 | MH+ 515 r.t. 22.2' Méthode C |
| **10** | 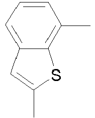 | H | 1 | 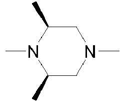 | 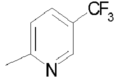 | 1 | oxalate | 93-94 | MH+ 529 r.t. 12.7' Méthode B |
| **11** | 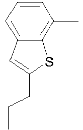 | H | 1 | 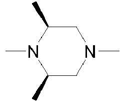 | 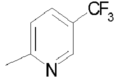 | 1 | oxalate | 90-91 | MH+ 557 r.t. 17.0' Méthode D |
| **12** | 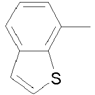 | H | 1 | 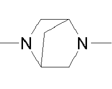 | 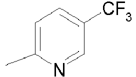 | 1 | oxalate | 105-106 | MH+ 499 r.t. 12.7' Méthode D |
| **13** | 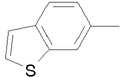 | H | 1 | 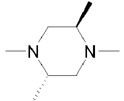 | 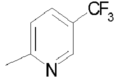 | 1 | oxalate | 141-143 | MH+ 515 r.t. 6.3' Méthode A |
| **14** | 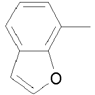 | H | 1 | 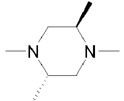 | 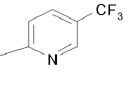 | 1 | oxalate | 130-131 | MH+ 499 r.t. 8.6' Méthode B |
| **15** | 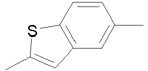 | H | 1 | 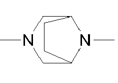 | 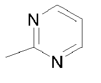 | 1 | -- | 171-173 | MH+ 460 r.t. 5.7' Méthode A |
| **16** | 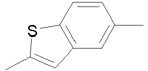 | H | 1 | 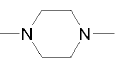 | 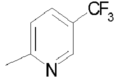 | 1 | -- | 159 | MH+ 5.1 r.t. 7.6' Méthode A |
| **17** | 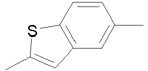 | H | 1 | 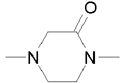 | 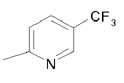 | 1 | -- | 141-143 | MH+ 515 r.t. 8.2' Méthode A |
| **18** | 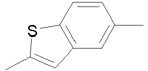 | H | 1 | 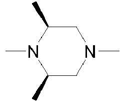 | 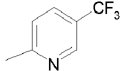 | 1 | -- | 162-163 | MH+ 529 r.t. 6.3' Méthode A |
| **19** | 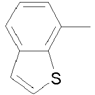 | H | 1 | 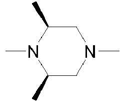 | 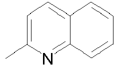 | 1 | HCl | 192-193 | MH+ 497 r.t. 5.5' Méthode A |
| **20** | 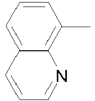 | H | 1 | 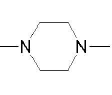 | 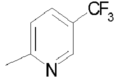 | 1 | oxalate | -- | MH+ 482 r.t. 4.7' Méthode A |
| **21** | 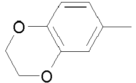 | H | 1 | 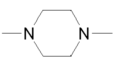 | 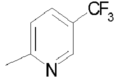 | 1 | -- | 142-143 | MH+ 489 r.t. 5.3' Méthode A |
| **22** | 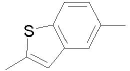 | H | 1 | 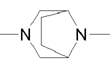 | 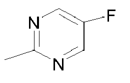 | 1 | -- | 177-179 | MH+ 478 r.t. 6.7' Méthode A |
| **23** | 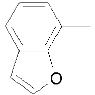 | H | 1 | 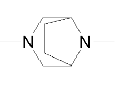 | 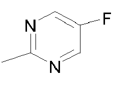 | 1 | -- | 157-158 | MH+ 448 r.t. 5.6' Méthode C |
| **24** | 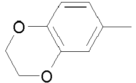 | H | 1 | 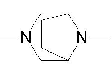 | 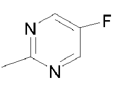 | 1 | -- | 163-165 | MH+ 466 r.t. 5.0' Méthode A |
| **25** | 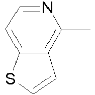 | H | 1 | 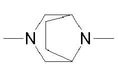 | 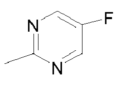 | 1 | -- | 167-168 | MH+ 465 r.t.3.8' Méthode A |
| **26** | 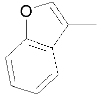 | H | 1 | 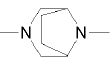 | 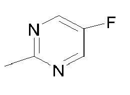 | 1 | -- | 207-210 | MH+ 448 r.t. 5.6' Méthode A |
| **27** | 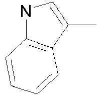 | H | 1 | 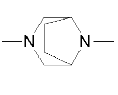 | 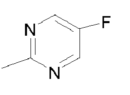 | 1 | oxalate | 128-130 | MH+ 447 r.t. 4.8' Méthode A |
| **28** | 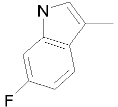 | H | 1 | 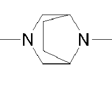 | 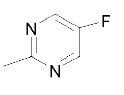 | 1 | oxalate | 95-96 | MH+ 465 r.t. 5.3' Méthode A |
| **29** | 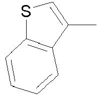 | H | 1 | 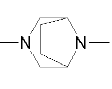 | 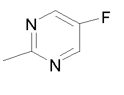 | 1 | -- | 172-174 | MH+ 464 r.t. 5.5' Méthode A |
| **30** | 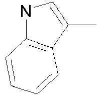 | H | 1 | 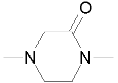 | 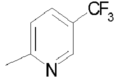 | 1 | -- | 230-233 | MH+ 484 r.t. 6.8' Méthode A |
| **31** | 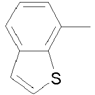 | H | 1 | 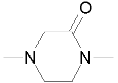 | 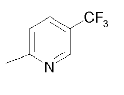 | 1 | -- | 143-146 | MH+ 501 r.t. 7.7' Méthode A |
| **32** | 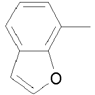 | H | 1 | 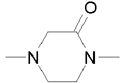 | 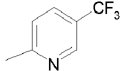 | 1 | -- | 151-152 | MH+ 485 r.t. 7.5' Méthode A |
| **33** | 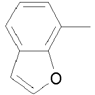 | H | 1 | 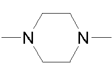 | 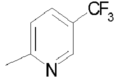 | 1 | -- | 155-156 | MH+ 471 r.t. 5.7' Méthode A |
| **34** | 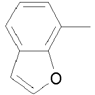 | H | 1 | 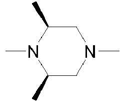 | 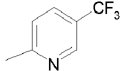 | 1 | -- | 138-139 | MH+ 499 r.t. 5.8' Méthode A |
| **35** | 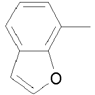 | H | 1 | 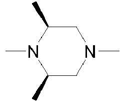 | 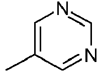 | 1 | - | 142-143 | MH+ 432 r.t. 4.6' Méthode A |
| **36** | 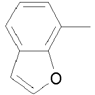 | H | 1 | 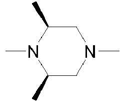 | 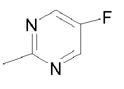 | 1 | HCl | 174-175 | MH+ 450 r.t. 5.4' Méthode A |
| **37** | 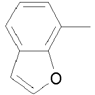 | H | 1 | 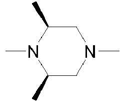 | 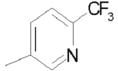 | 1 | - | 119-120 | MH+ 499 r.t. 5.7' Méthode A |
| **38** | 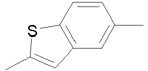 | H | 1 | 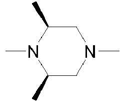 | 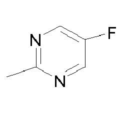 | 1 | -- | 205-206 | MH+ 480 r.t. 5.1' Méthode A |
| **39** | 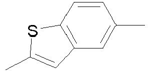 | H | 1 | 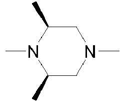 | 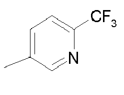 | 1 | -- | 217-218 | MH+ 529 r.t. 5.4' Méthode A |
| **40** | 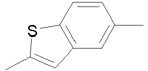 | H | 1 | 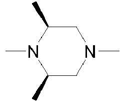 | 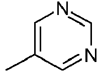 | 1 | -- | 191-192 | MH+ 462 r.t. 4.5' Méthode A |
| **41** | 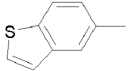 | H | 1 | 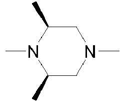 | 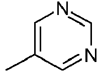 | 1 | -- | 218-220 | MH+ 448 r.t. 4.5' Méthode A |
| **42** | 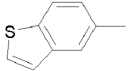 | H | 1 | 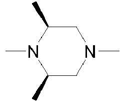 | 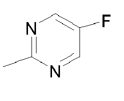 | 1 | oxalate | -- | MH+ 466 r.t. 5.6' Méthode E |
| **43** | 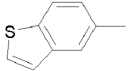 | H | 1 | 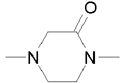 | 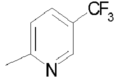 | 1 | -- | 169-170 | MH+ 501 r.t. 7.0' Méthode A |
| **44** | 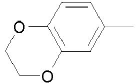 | H | 1 | 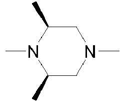 | 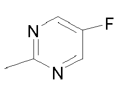 | 1 | oxalate | 90-91 | MH+ 468 r.t. 4.3' Méthode A |
| **45** | 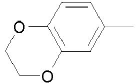 | H | 1 | 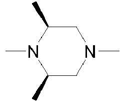 | 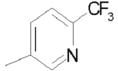 | 1 | -- | 200-201 | MH+ 517 r.t. 4.5' Méthode A |
| **46** | 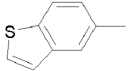 | H | 1 | 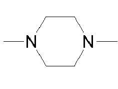 | 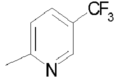 | 1 | -- | 170-171 | MH+ 487 r.t. 5.3' Méthode A |
| **47** | 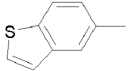 | H | 1 | 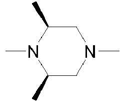 | 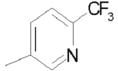 | 1 | -- | 136-137 | MH+ 515 r.t. 6.3' Méthode E |
| **48** | 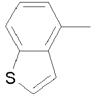 | H | 1 | 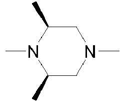 | 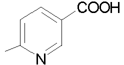 | 1 | -- | - | MH+ 491 r.t. 4.4' Méthode A |
| **49** | 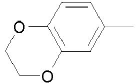 | H | 1 | 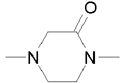 | 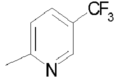 | 1 | -- | 161-162 | MH+ 503 r.t. 6.2' Méthode A |
| **50** | 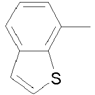 | H | 1 | 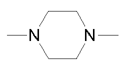 | 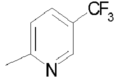 | 1 | -- | 120-121 | MH+ 487 r.t. 5.7' Méthode A |
| **51** | 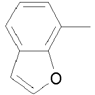 | H | 1 | 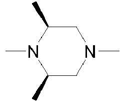 | 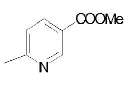 | 1 | oxalate | -- | MH+ 489 r.t. 5.1' Méthode A |
| **52** | 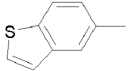 | H | 1 | 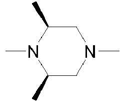 | 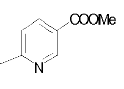 | 1 | -- | 130-132 | MH+ 505 r.t. 6.7' Méthode E |
| **53** | 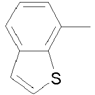 | H | 1 | 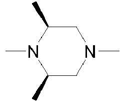 | 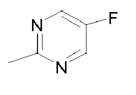 | 1 | -- | -- | MH+ 466 r.t. 5.2' Méthode A |
| **54** | 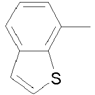 | H | 1 | 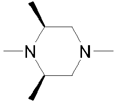 | 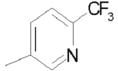 | 1 | HCl | 225-226 | MH+ 515 r.t. 5.6' Méthode A |
| **55** | 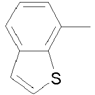 | H | 1 | 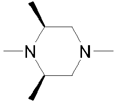 | 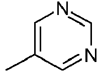 | 1 | -- | 195-196 | MH+ 448 r.t. 4.6' Méthode A |
| **56** | 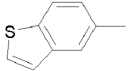 | H | 1 | 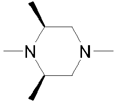 | 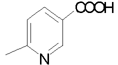 | 1 | -- | -- | MH+ 491 r.t. 6.1' Méthode A |
| **57** | 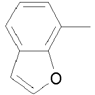 | H | 1 | 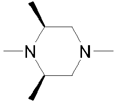 | 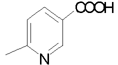 | 1 | -- | 272-274 | MH+ 475 r.t. 4.7' Méthode A |
| **58** | 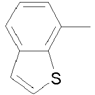 | H | 1 | 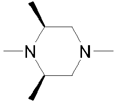 | 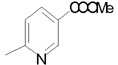 | 1 | HCl | 208-210 | MH+ 505 r.t. 6.6' Méthode E |
| **59** | 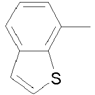 | H | 1 | 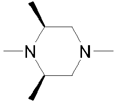 | 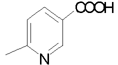 | 1 | -- | -- | MH+ 491 r.t. 6.2' Méthode F |
| **60** | 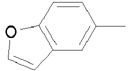 | H | 1 | 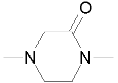 | 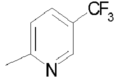 | 1 | -- | 154-155 | MH+ 485 r.t. 7.3' Méthode A |
| **61** | 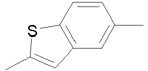 | H | 1 | 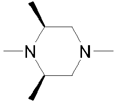 | 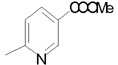 | 1 | -- | 148-150 | MH+ 519 r.t. 8.2' Méthode A |
| **62** | 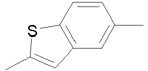 | H | 1 | 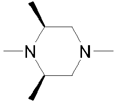 | 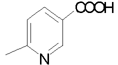 | 1 | -- | -- | MH+ 505 r.t. 5.4' Méthode A |
| **63** | H | 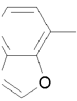 | 1 | 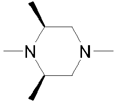 | 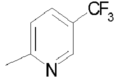 | 1 | -- | 129-130 | MH+ 499 r.t.5.8' Méthode A |
| **64** | 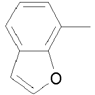 | H | 1 | 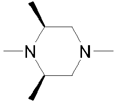 | 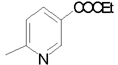 | 1 | HCl | -- | MH+ 503 r.t. 5.5' Méthode A |
| **65** | 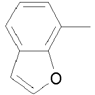 | H | 1 | 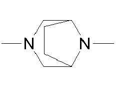 | 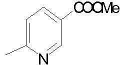 | 1 | -- | 158-159 | MH+ 487 r.t. 5.1' Méthode A |
| **66** | 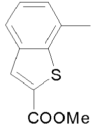 | H | 1 | 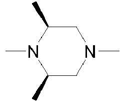 | 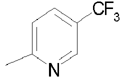 | 1 | HCl | 138-141 | MH+ 573 r.t. 6.0' Méthode A |
| **67** | 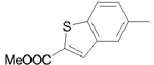 | H | 1 | 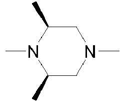 | 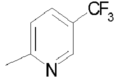 | 1 | oxalate | -- | MH+ 573 r.t. 6.0' Méthode A |
| **68** | 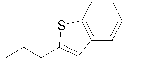 | H | 1 | 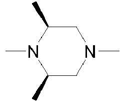 | 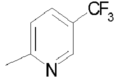 | 1 | -- | 157-159 | MH+ 557 r.t. 6.8' Méthode A |
| **69** | 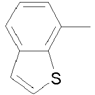 | H | 1 | 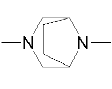 | 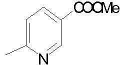 | 1 | -- | 153-154 | MH+ 503 r.t. 5.5' Méthode A |
| **70** | 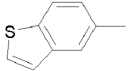 | H | 1 | 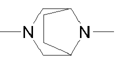 | 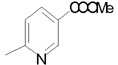 | 1 | -- | 150-151 | MH+ 503 r.t. 5.5' Méthode A |
| **71** | 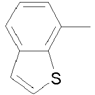 | H | 1 | 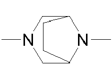 | 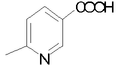 | 1 | -- | 121-122 | MH+ 489 r.t. 5.0' Méthode A |
| **72** | 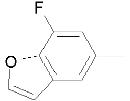 | H | 1 | 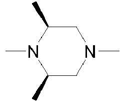 | 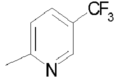 | 1 | oxalate | -- | MH+ 517 r.t. 5.6' Méthode A |
| **73** | 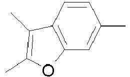 | H | 1 | 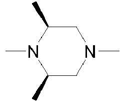 | 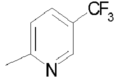 | 1 | oxalate | -- | MH+ 527 r.t. 6.1' Méthode A |
| **74** | 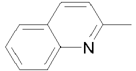 | H | 1 | 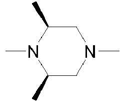 | 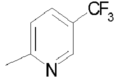 | 1 | -- | -- | MH+ 510 r.t. 4.5' Méthode A |
| **75** | 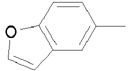 | H | 1 | 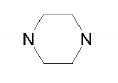 | 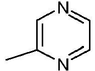 | 1 | -- | | MH+ 404 r.t. 11.5' Méthode H |
| **76** | 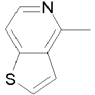 | H | 1 | 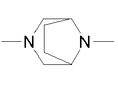 | 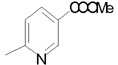 | 1 | -- | 170-171 | MH+ 504 r.t. 3.7' Methode A |
| **77** | 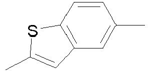 | H | 1 | 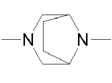 | 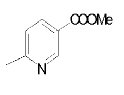 | 1 | -- | 179-180 | MH+ 517 r.t. 5.8' Méthode A |
| **78** | 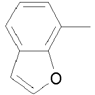 | H | 1 | 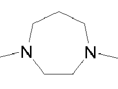 | 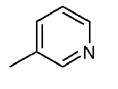 | 1 | oxalate | 100-101 | MH+ 417 r.t. 3.4' Méthode A |
| **79** | 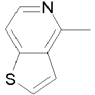 | H | 1 | 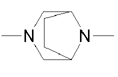 | 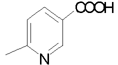 | 1 | -- | 159-160 | MH+ 490 r.t. 3.8' Méthode A |
| **80** | 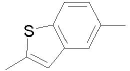 | H | 1 | 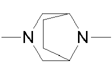 | 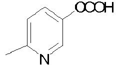 | 1 | -- | 160-161 | MH+ 503 r.t. 8.5' Méthode A |
| **81** | 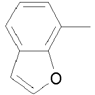 | H | 0 | 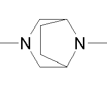 | 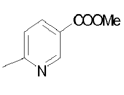 | 1 | -- | 169-170 | MH+ 473 r.t. 5.0' Méthode A |
| **82** | 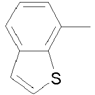 | H | 0 | 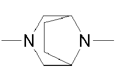 | 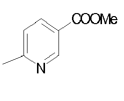 | 1 | -- | 172-173 | MH+ 489 r.t. 5.17' Méthode A |
| **83** | 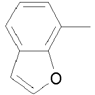 | H | 0 | 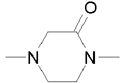 | 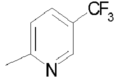 | 1 | -- | 184-186 | MH+ 471 r.t. 10.9' Méthode A |
| **84** | 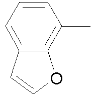 | H | 0 | 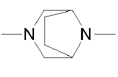 | 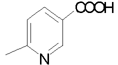 | 1 | -- | 188-189 | MH+ 459 r.t. 4,81' Méthode A |
| **85** | 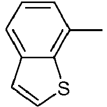 | H | 1 | 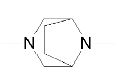 | 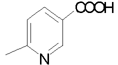 | 1 | -- | 191-192 | MH+ 489 r.t. 5,22' Méthode A |
| **86** | 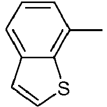 | H | 0 | 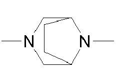 | 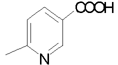 | 1 | -- | 189-190 | MH+ 475 r.t. 4,97' Méthode A |
| **87** | 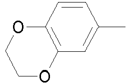 | H | 1 | 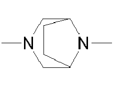 | 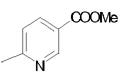 | 1 | -- | 172-174 | MH+ 505 r.t. 4,77' Méthode A |
| **88** | 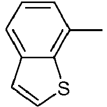 | H | 1 | 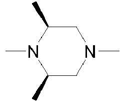 | 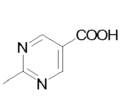 | 1 | HCl | 275-278 | MH+ 492 r.t. 4,65' Méthode A |
| **89** | 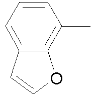 | H | 1 | 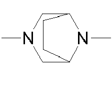 | 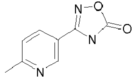 | 1 | -- | 230-232 | MH+ 513 r.t. 4,86' Méthode A |
| **90** | 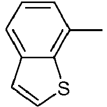 | H | 1 | 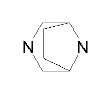 | 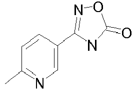 | 1 | -- | 215-217 | MH+ 529 r.t. 5,07' Méthode A |
| **91** | 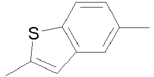 | H | 1 | 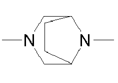 | 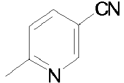 | 1 | -- | 171-172 | MH+ 484 r.t. 5,87' Méthode A |
| **92** | 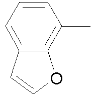 | H | 1 | 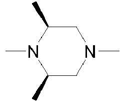 | 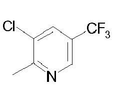 | 1 | -- | 130-131 | MH+ 533 r.t. 5,75' Méthode A |
| **93** | 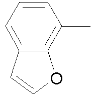 | H | 1 | 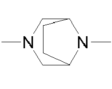 | 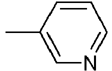 | 1 | -- | 121-123 | MH+ 429 r.t. 4,06' Méthode A |
| **94** | 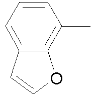 | H | 1 | 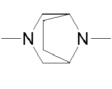 | 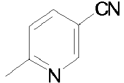 | 1 | -- | 184-185 | MH+ 454 r.t. 5,35' Méthode A |
| **95** | 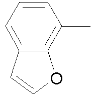 | H | 1 | 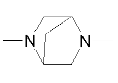 | 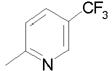 | 1 | HCl | -- | MH+ 483 r.t. 5,05' Méthode A |
| **96** | 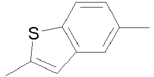 | H | 1 | 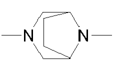 | 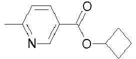 | 1 | -- | 153-154 | MH+ 557 r.t. 6,5' Méthode A |
| **97** | 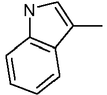 | H | 1 | 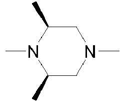 | 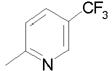 | 1 | -- | 198-200 | MH+ 498 r.t. 5,27' Méthode |
| **98** | 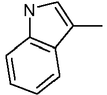 | H | 1 | 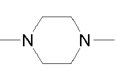 | 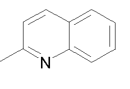 | 1 | -- | 213-215 | MH+ 452 r.t. 4,19' Méthode I |
| **99** | 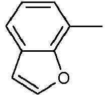 | H | 1 | 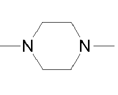 | 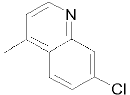 | 1 | oxalate | 99-100 | MH+ 487 r.t. 4,18' Méthode I |
| **100** | 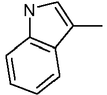 | H | 1 | 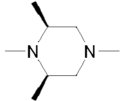 | 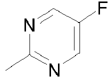 | 1 | -- | 235-237 | MH+ 449 r.t. 4,6' Méthode I |
| **101** | 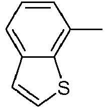 | H | 1 | 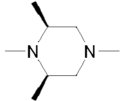 | 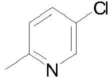 | 1 | -- | 157-158 | MH+ 481 r.t. 5,46' Méthode I |
| **102** | 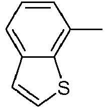 | H | 1 | 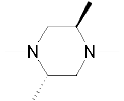 | 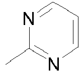 | 1 | -- | -- | MH+ 448 r.t. 4.78 Méthode I |
| **103** | 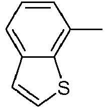 | H | 1 | 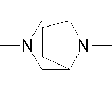 | 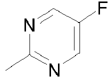 | 1 | -- | 112-113 | MH+ 464 r.t. 5.17' Méthode I |
| **104** | 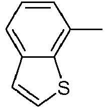 | H | 1 | 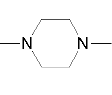 | 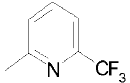 | 1 | -- | 74-75 | MH+ 487 r.t. 5.67' Méthode I |
| **105** | 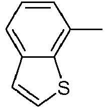 | H | 1 | 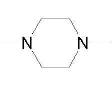 | 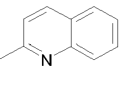 | 1 | -- | 177-178 | MH+ 469 r.t. 4.8' Méthode I |
| **106** | 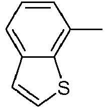 | H | 1 | 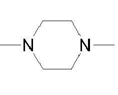 | 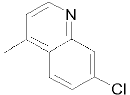 | 1 | -- | 90-91 | MH+ 503 r.t. 4.39' Méthode I |
| **107** | 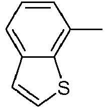 | H | 1 | 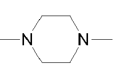 | 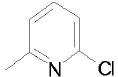 | 1 | -- | 92-93 | MH+ 453 r.t. 5.26' Méthode I |
| **108** | 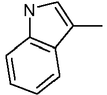 | H | 1 | 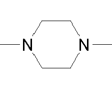 | 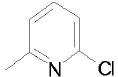 | 1 | -- | 202-204 | MH+ 436 r.t. 4.67' Méthode I |
| **109** | 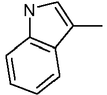 | H | 1 | 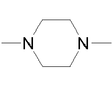 | 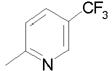 | 1 | -- | 220-222 | MH+ 470 r.t. 4.93' Méthode I |
| **110** | 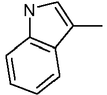 | H | 1 | 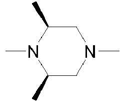 | 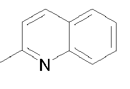 | 1 | -- | 198-200 | MH+ 480 r.t. 4.45' Méthode I |
| **111** | 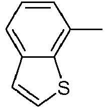 | H | 1 | 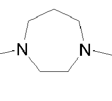 | 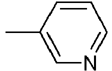 | 1 | -- | -- | MH+ 433 r.t. 3.62' Méthode I |
| **112** | 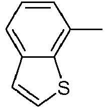 | H | 1 | 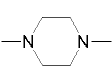 | 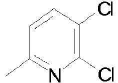 | 1 | -- | 89-90 | MH+ 487 r.t. 5.59' Méthode I |
| **113** | 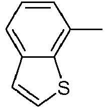 | H | 1 | 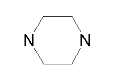 | 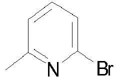 | 1 | -- | 80-81 | MH+ 499 r.t. 5.3' Méthode I |
| **114** | 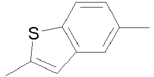 | H | 1 | 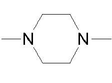 | 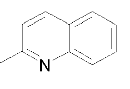 | 1 | -- | 189-190 | MH+ 483 r.t. 5.11 Méthode I |
| **115** | 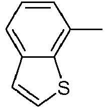 | H | 1 | 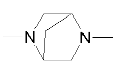 | 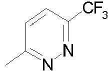 | 1 | -- | -- | MH+ 500 r.t. 4.83' Méthode I |
| **116** | 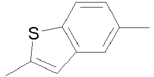 | H | 1 | 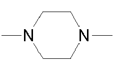 | 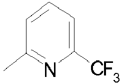 | 1 | -- | 121-123 | MH+ 501 r.t. 5.94' Méthode I |
| **117** | 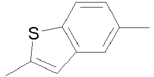 | H | 1 | 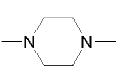 | 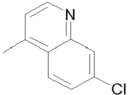 | 1 | -- | 90-93 | MH+ 517 r.t. 4.86' Méthode I |
| **118** | 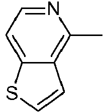 | H | 1 | 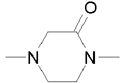 | 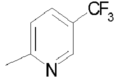 | 1 | -- | 145-148 | MH+ 502 r.t. 4.13' Méthode I |
| **119** | 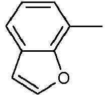 | H | 1 | 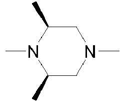 | 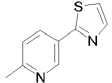 | 1 | oxalate | 140-145 | MH+ 514 r.t. 5.19' Méthode I |
| **120** | 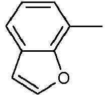 | H | 1 | 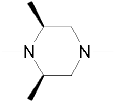 | 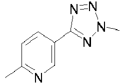 | 1 | oxalate | 165-170 | MH+ 513 r.t. 4.97' Méthode I |
| **121** | 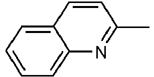 | H | 1 | 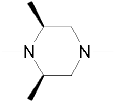 | 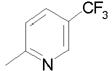 | 1 | -- | 94-95 | MH+ 510 r.t. 4.95' Méthode I |
| **122** | 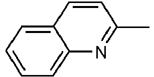 | H | 1 | 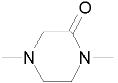 | 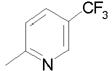 | 1 | -- | 135-136 | MH+ 496 r.t. 5.24' Méthode I |
| **123** | 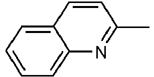 | H | 1 | 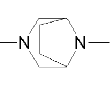 | 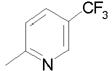 | 1 | -- | 101-102 | MH+ 508 r.t. 5.1' Méthode I |
| **124** | 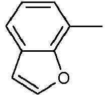 | H | 1 | 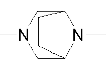 | 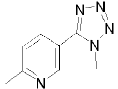 | 1 | -- | 178-180 | MH+ 511 r.t. 4.72' Méthode I |
| **125** | 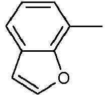 | H | 1 | 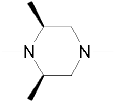 | 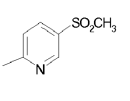 | 1 | -- | -- | MH+ 509 r.t. 4.71' Méthode I |

Les composés selon l'invention ont fait l'objet d'études biochimiques.

### Culture cellulaire :

La souche SH-SY-5Y (neuroblastome humain) est cultivée classiquement dans un milieu de culture DMEM (de l'anglais Dulbecco's Modified Eagle's Medium) (Gibco BRL, France) contenant du SVF (5%) (sérum de veau foetal) (Boehringer Mannheim, Allemagne), du pyruvate de sodium (1 mM) et de la glutamine (4 mM) dans des flacons de culture recouvert de collagène (Becton Dickinson, France).

La souche mère SK-N-BE (neuroblastome humain) et le clone Bep 75, exprimant de manière stable la forme entière du récepteur p75^{NTR} humain (SK-N-BE Bep 75) sont cultivés classiquement dans un milieu de culture RPMI contenant du SVF (5%), du pyruvate de sodium (1 mM) et de la glutamine (4 mM). Pour les cellules SK-N-BE Bep 75, on ajoute de l'hygromycine (200 µl/20ml milieu) comme agent de sélection.

### Etude de la dimérisation du récepteur p75^{NTR} indépendamment de son ligand

L'étude de la dimérisation du récepteur p75^{NTR} est réalisée sur une suspension cellulaire de la souche SK-N-BE Bep 75. Les cellules (2,5 10⁴ cellules/puits) sont disposées dans des puits (plaque de 96 puits) pendant 24 h, puis pré-incubées pendant 1h à 37°C en présence ou non des composés selon l'invention. On ajoute ensuite du surnageant, issu de culture de cellules humaines d'origine rénale HEK293 exprimant, après 48h de transfection, et sécrétant une forme soluble du récepteur p75^{NTR} (partie extracellulaire du récepteur) couplée à une phosphatase alcaline, ce à la concentration finale de 10 nM. La quantification de la liaison spécifique du récepteur soluble p75^{NTR} au récepteur présent sur cellules SK-N-BE Bep 75 est déterminée par la mesure de l'activité enzymatique de la phosphatase alcaline après incubation des cellules pendant 1 heure à 37°C en présence du surnageant. Après filtration et transfert des filtres dans des plaques de 24 puits, l'activité de la phosphatase alcaline est déterminée par ajout de substrat chimioluminescent CDP-Star (ready-to-use, Roche). Les concentrations inhibitrices de 50 %(CI₅₀) de la dimérisation du récepteur p75^{NTR} des composés selon l'invention sont faibles et varient de10⁻⁶ à 10⁻¹⁰¹M.

Les composés de formule (I) présentent une activité dans ce test avec des CI₅₀ qui varient de 10⁻⁶ à 10⁻¹¹M.

Par exemple, les composés n° 1, 14, 34 et 36 ont montré respectivement une CI₅₀ de 0,08 nM, 1,09 nM, 0,94 nM et 20 nM.

### Mesure de l'apoptose

Les cellules (souches de neuroblastomes humains SH-SY-5Y et SK-N-BE Bep 75) sont installées dans des boîtes de Pétri de 35 mm de diamètre (Biocoat collagenI, (10⁵ cellules/puits)) dans un milieu de culture approprié contenant 5 % de SVF durant 24H. Le milieu de culture est ensuite éliminé, les cellules sont rincées avec du PBS (de l'anglais Dulbecco's Phosphate buffered saline), puis on ajoute soit du milieu frais contenant 5% de SVF, soit du milieu contenant du NGF (à la concentration de 10 ng/ml), soit du peptide beta-amyloïde (Aβ1-40) (à la concentration de 10 µM), ceci en présence ou non des composés selon l'invention. Les taux d'apoptose sont mesurés 48 heures après les traitements dans le cas de la souche SH-SY-5Y, et 24 heures après dans le cas de la souche SK-N-BE Bep 75 par quantification des histones cytoplasmiques associés aux fragments d'ADN (cell death détection ELISA, Boehringer Mannheim, Allemagne). Les taux d'apoptose sont exprimés en quantité d'oligonucléosomes/10⁵ cellules. Chaque valeur correspond à la moyenne de 9 points expérimentaux répartis dans 3 expériences indépendantes.

Les composés de formule (I) présentent une activité dans ce test avec des CI₅₀ qui varient de 10⁻⁶ à 10⁻¹¹M.

Par exemple, les composés n° 19, 14, et 34 ont montré respectivement une CI₅₀ de 1,07 nM, 1,33 nM, et 3,39 nM.

Ainsi, la fixation des composés selon l'invention au récepteur p75^{NTR} se traduit, d'une part, au niveau biochimique par l'inhibition de la dimérisation du récepteur induit par les neurotrophines, ou indépendamment du ligand, et, d'autre part, au niveau cellulaire par l'inhibition de l'effet proapoptotique médié par le récepteur p75^{NTR}.

Ainsi, selon un des objets de la présente invention, les composés de formule (I) présentent une activité très intéressante d'inhibition de la dimérisation du récepteur p75^{NTR} indépendamment de son ligand.

Les composés selon l'invention peuvent donc être utilisés pour la préparation de médicaments, en particulier de médicaments destinés à prévenir ou à traiter toute pathologie où le récepteur p75^{NTR} est impliqué, plus particulièrement celles indiquées ci-après.

Les composés selon l'invention peuvent également être utilisés pour prévenir ou traiter toute pathologie où le récepteur p75^{NTR} est impliqué, plus particulièrement celles indiquées ci-après.

Ainsi, selon un autre de ses aspects, l'invention a pour objet des médicaments qui comprennent un composé de formule (I), ou un sel d'addition de ce dernier à un acide pharmaceutiquement acceptable.

Ainsi, les composés selon l'invention peuvent être utilisés, chez l'homme ou chez l'animal, dans le traitement ou la prévention de différentes affections p75^{NTR} dépendantes telles que les maladies neurodégénératives centrales et périphériques comme la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire; la sclérose latérale amyotrophique, la sclérose en plaques ; les affections cardiovasculaires comme les dommages cardiaques post-ischémiques, les cardiomyopathies, l'infarctus du myocarde, l'insuffisance cardiaque, l'ischémie cardiaque, l'infarctus cérébral ; les neuropathies périphériques (d'origine diabétique, traumatisme ou iatrogène) ; les dommages au nerf optique et à la rétine (dégénérescence du pigment rétinien, glaucome) ; l'ischémie rétinale ; la dégénérescence maculaire ; les traumatismes de la moelle épinière et les traumatismes crâniens ; l'athérosclérose ; les sténoses ; les désordres de la cicatrisation ; l'alopécie.

Les composés selon l'invention peuvent également être utilisés dans le traitement de la pancréatite, et de la fibrose hépatique.

Les composés selon l'invention peuvent également être utilisés dans le traitement des cancers comme celui du poumon, de la thyroïde, du pancréas, de la prostate, de l'intestin grêle et du colon, du sein, dans le traitement des tumeurs, des métastases et des leucémies.

Les composés selon l'invention peuvent également être utilisés dans le traitement des désordres respiratoires comme l'inflammation pulmonaire, l'allergie et l'asthme, la broncho-pneumopathie chronique obstructive.

Les composés selon l'invention peuvent aussi être utilisés dans le traitement de la douleur cutanée (de la peau, des tissus sous-cutanés et organes associés), somatique, viscérale (au niveau du système circulatoire, respiratoire, gastro-intestinal, ou génito-urinaire), et neurologiques.

Les composés selon l'invention peuvent être utilisés dans le traitement des douleurs chroniques neuropathiques et inflammatoires et dans le traitement des maladies auto-immunes comme la polyarthrite rhumatoïde.

Les composés selon l'invention peuvent aussi être utilisés dans le traitement des maladies comme la spondylarthrite ankylosante, le rhumatisme psoriasique, le psoriasis en plaques.

Les composés selon l'invention peuvent également être utilisés dans le traitement des fractures osseuses, dans le traitement ou la prévention des maladies osseuses comme l'ostéoporose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel peut être administré sous forme unitaire d'administration, en mélange avec des excipients pharmaceutiques classiques, aux animaux et aux êtres humains pour la prévention ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La dose de principe actif administrée par jour peut atteindre 0,01 à 100 mg/kg, en une ou plusieurs prises, préférentiellement 0,02 à 50 mg/kg. En général, la dose journalière du composé de l'invention sera la dose efficace la plus faible du composé capable de produire un effet thérapeutique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I) : 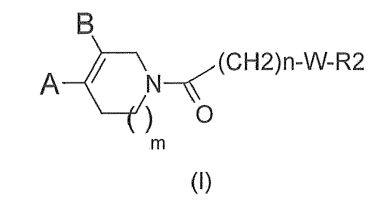 dans laquelle :
- n représente 1 ou 2 ;
- m représente 0 ou 1 ;
- A représente un groupe hétérocyclique condensé de formule (Y) 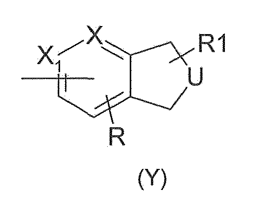
et B représente un atome d'hydrogène ;
ou
A représente un atome d'hydrogène ;
et B représente un groupe hétérocyclique condensé de formule (Y) 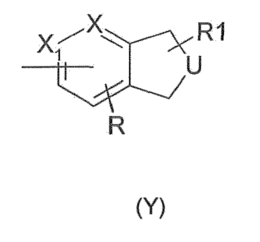 L'hétérocycle condensé de formule Y pouvant être rattaché au reste de la molécule par l'un quelconque des atomes de carbone disponibles et dans lequel :
- U complète :
- soit un noyau à 6 atomes, aromatique ou saturé, contenant 1 ou 2 atomes d'azote, le noyau pouvant être substitué par un ou deux atomes d'halogène, un ou deux groupes (C1-C4)alkyle ou (C1-C4)alcoxy, un ou deux radicaux perfluoroalkyle;
- soit un noyau à 5 atomes, aromatique ou saturé, contenant un atome d'azote, d'oxygène ou de soufre, le noyau pouvant être substitué par un ou deux groupes (C1-C4) alkyle ;
- X et X1 représentent CH ou N ;
- R et R1 situés sur l'une quelconque des positions disponibles, représentent indépendamment un atome d'hydrogène, un atome d'halogène, un groupe (C1-C4)alkyle, (C1-C4)alcoxy, un radical perfluoroalkyle , trifluorométhoxy, un cyano, un groupe COOH, COOalkyle, CONR3R4 ou NHCOR3 ;
- -W- est un hétérocycle azoté choisi parmi :
-W-= 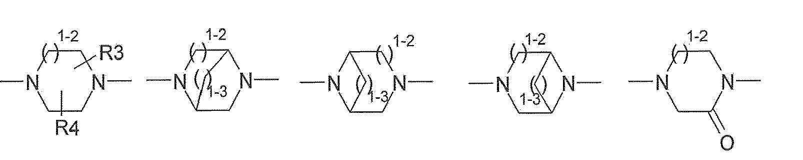
- 1-2 représente 1 ou 2 ;
- 1-3 représente 1, 2 ou 3 ;
- R2 représente un groupe de formule : 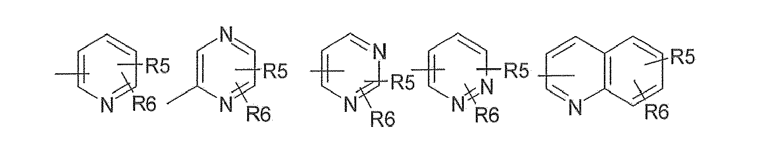
- dans lequel R5 et R6, situés sur l'une quelconque des positions disponibles, représentent indépendamment un atome d'hydrogène, un atome d'halogène, un groupe (C1-C4)alkyle, (C1-C4)alcoxy, un radical trifluorométhyle, trifluorométhoxy, un cyano, un groupe COOH, COOalkyle, COOcycloalkyle, SOalkyle, SO₂alkyle, CONR3R4, NR3R4 ou NHCOR3 ;
ou un des R5 et R6 peut aussi représenter un hétérocycle choisi parmi : 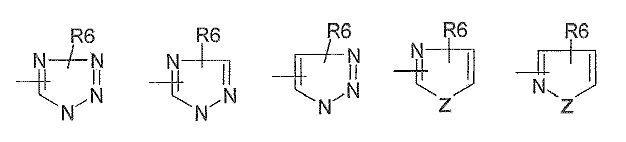 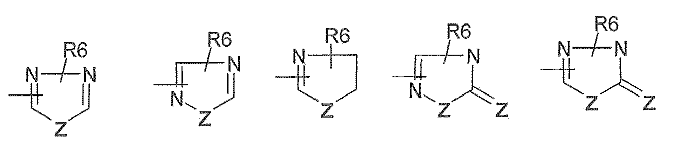
- Z représente un atome d'oxygène ou de soufre ;
- R3 et R4 représentent un hydrogène ou un groupe (C1-C6)alkyle ;
à l'état de base ou de sel d'addition à un acide.

2. Composé selon la revendication 1 tel que
- A représente un groupe hétérocyclique condensé de formule (Y) 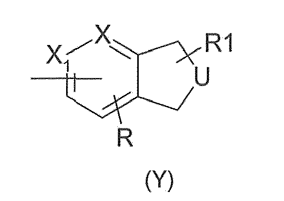 et B représente un atome d'hydrogène ;
ou
A représente un atome d'hydrogène ;
et B représente un groupe hétérocyclique condensé de formule (Y) 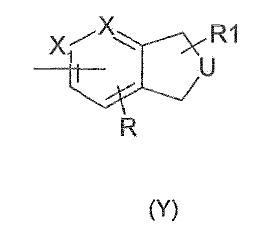 l'hétérocycle condensé de formule Y pouvant être rattaché au reste de la molécule par l'un quelconque des atomes de carbone disponibles du noyau benzénique ;
à l'état de base ou de sel d'addition à un acide.

3. Composé selon l'une des revendications 1 à 2 tel que R et R1 situés sur l'une quelconque des positions disponibles, représentent indépendamment un atome d'hydrogène, un atome d'halogène, un groupe (C₁-C₄) alkyle, COOalkyle; à l'état de base ou de sel d'addition à un acide.

4. Composé selon l'une des revendications 1 à 3 tel que :
- -W- est un hétérocycle azoté choisi parmi :
-W-= 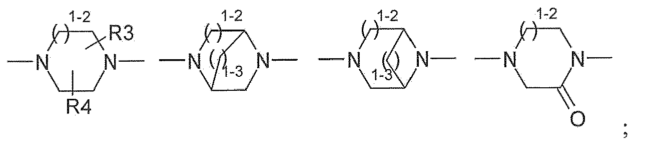
- R3 et R4 représentent un hydrogène ou un groupe méthyle ; à l'état de base ou de sel d'addition à un acide.

5. Composé selon l'une des revendications 1 à 4 tel que :
- R2 représente un groupe de formule : 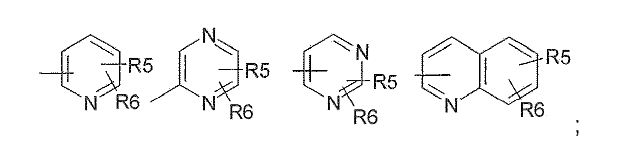
- R5 et R6 situés sur l'une quelconque des positions disponibles, représentent indépendamment un atome d'hydrogène, un radical trifluorométhyle, un groupe COOH, COOalkyle ou COOcycloalkyle; ou
un des R5 et R6 peut aussi représenter un hétérocycle choisi parmi : 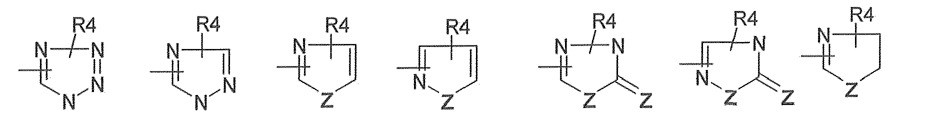
- Z représente un atome d'oxygène ou de soufre ;
- R3 et R4 représentent un hydrogène ou un groupe méthyle ;
à l'état de base ou de sel d'addition à un acide.

6. Composé selon l'une quelconque des revendications 1 à 5 choisi parmi :
- Composé n°1: 1-(4-Benzo[b]thiophèn-7-yl-3,6-dihydro-2H-pyridin-1-yl)-2-(3,5-diméthyl-2,3,5,6-tétrahydro-[1,2']bipyrazinyl-4-yl)-éthanone ;
- Composé n° 2: 1-(4-Benzo[b]thiophèn-7-yl-3,6-dihydro-2H-pyridin-1-yl)-2-(8-pyrimidin-2-yl-3,8-diaza-bicyclo[3.2.1]oct-3-yl)-éthanone ;
- Composé n°3: 1-(4-Benzo[b]thiophèn-5-yl-3,6-dihydro-2H-pyridin-1-yl)-2-(8-pyrimidin -2-yl-3,8-diaza-bicyclo[3.2.1]oct-3-yl)-éthanone ;
- Composé n°4 : 1-(4-Benzo[b]thiophèn-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°5 : 1-(4-Benzo[b]thiophèn-5-yl-3,6-dihydro-2H-pyridin-1-yl)-2-((2S,5R)-2,5-diméthyl-2,3,5,6-tétrahydro-[1,2']bipyrazinyl-4-yl)-éthanone ;
- Composé n°6 : 1-(4-Benzo[b]thiophèn-7-yl-3,6-dihydro-2H-pyridin-1-yl)-2-((2S,5R)-2,5-diméthyl-2,3,5,6-tétrahydro-[1,2']bipyrazinyl-4-yl)-éthanone ;
- Composé n°7 : 1-(4-Benzo[b]thiophèn-5-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°8 : 1-(4-Benzo[b]thiophèn-7-yl-3,6-dihydro-2H-pyridin-1-yl)-2-[8-(5-trifluorométhyl-pyridin-2-yl)-3,8-diaza-bicyclo[3.2.1]oct-3-yl]-éthanone ;
- Composé n°9 : 1-(4-Benzo[b]thiophèn-7-yl-3,6-dihydro-2H-pyridin-1-yl)-2-[(2R,5S)-2,5-diméthyl-4-(5-trifluorométhyl-pyridin-2-yl)-pipérazin-1-yl]-éthanone ;
- Composé n°10 : 2-[(2S,6R)-2,6-Diméthyl-4-(5-trifluorométhyl-pyridin-2-yl)-pipérazin -1-yl]-1-[4-(2-méthyl-benzo[b]thiophèn-7-yl)-3,6-dihydro-2H-pyridin-1-yl]-éthanone ;
- Composé n°11 : 2-[(2S,6R)-2,6-Diméthyl-4-(5-trifluorométhyl-pyridin-2-yl)-pipérazin -1-yl]-1-[4-(2-propyl-benzo[b]thiophèn-7-yl)-3,6-dihydro-2H-pyridin-1-yl]-éthanone ;
- Composé n°12: 1-(4-Benzo[b]thiophèn-7-yl-3,6-dihydro-2H-pyridin-1-yl)-2-[5-(5-trifluorométhyl-pyridin-2-yl)-2,5-diaza-bicyclo[2.2.1]hept-2-yl]-éthanone ;
- Composé n°13: 1-(4-Benzo[b]thiophèn-6-yl-3,6-dihydro-2H-pyridin-1-yl)-2-[(2R,5S) -2,5-diméthyl-4-(5-trifluorométhyl-pyridin-2-yl)-pipérazin-1-yl]-éthanone ;
- Composé n°14 : 1-(4-Benzofuran-7-yl-3,6-dihydro-2H-pyridin-1-yl)-2-[(2R,5S)-2,5-diméthyl-4-(5-trifluorométhyl-pyridin-2-yl)-pipérazin-1-yl]-éthanone ;
- Composé n°15: 1-[4-(2-Méthyl-benzo[b]thiophèn-5-yl)-3,6-dihydro-2H-pyridin-1-yl]-2-(8-pyrimidin-2-yl-3,8-diaza-bicyclo[3.2.1]oct-3-yl)-éthanone ;
- Composé n°16: 1-[4-(2-Méthyl-benzo[b]thiophèn-5-yl)-3,6-dihydro-2H-pyridin-1-yl]-2-[4-(5-trifluorométhyl-pyridin-2-yl)-pipérazin-1-yl]-éthanone ;
- Composé n°17 : 4-{2-[4-(2-Méthyl-benzo[b]thiophèn-5-yl)-3,6-dihydro-2H-pyridin-1-yl]-2-oxo-éthyl}-1-(5-trifluorométhyl-pyridin-2-yl)-pipérazin-2-one ;
- Composé n°18 : 2-[(2S,6R)-2,6-Diméthyl-4-(5-trifluorométhyl-pyridin-2-yl)-pipérazin -1-yl]-1-[4-(2-méthyl-benzo[b]thiophèn-5-yl)-3,6-dihydro-2H-pyridin-1-yl]-éthanone ;
- Composé n°19 : 1-(4-Benzo[b]thiophèn-7-yl-3,6-dihydro-2H-pyridin-1-yl)-2-((2S,6R) -2,6-diméthyl-4-quinolein-2-yl-pipérazin-1-yl)-éthanone ;
- Composé n°20: 1-(4-Quinolin-8-yl-3,6-dihydro-2H-pyridin-1-yl)-2-[4-(5-trifluorométhyl-pyridin-2-yl)-pipérazin-1-yl]-éthanone ;
- Composé n°21 : 1-[4-(2,3-Dihydro-benzo[1,4]dioxin-6-yl)-3,6-dihydro-2H-pyridin-1-yl]-2-[4-(5-trifluorométhyl-pyridin-2-yl)-pipérazin-1-yl]-éthanone ;
- Composé n°22 : 2-[8-(5-Fluoro-pyrimidin-2-yl)-3,8-diaza-bicyclo[3.2.1]oct-3-yl]-1-[4-(2-méthyl-benzo[b]thiophèn-5-yl)-3,6-dihydro-2H-pyridin-1-yl]-éthanone ;
- Composé n°23: 1-(4-Benzofuran-7-yl-3,6-dihydro-2H-pyridin-1-yl)-2-[8-(5-fluoro-pyrimidin-2-yl)-3,8-diaza-bicyclo[3.2.1]oct-3-yl]-éthanone ;
- Composé n°24: 1-[4-(2,3-Dihydro-benzo[1,4]dioxin-6-yl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°25: 2-[8-(5-Fluoro-pyrimidin-2-yl)-3,8-diaza-bicyclo[3.2.1]oct-3-yl]-1-(4-thieno[3,2-c]pyridin-4-yl-3,6-dihydro-2H-pyridin-1-yl)-éthanone ;
- Composé n°26: 1-(4-Benzofuran-3-yl-3,6-dihydro-2H-pyridin-1-yl)-2-[8-(5-fluoro-pyrimidin-2-yl)-3,8-diaza-bicyclo[3.2.1]oct-3-yl]-éthanone ;
- Composé n°27 : 2-[8-(5-Fluoro-pyrimidin-2-yl)-3,8-diaza-bicyclo[3.2.1]oct-3-yl]-1-[4-(1H-indol-3-yl)-3,6-dihydro-2H-pyridin-1-yl]-éthanone ;
- Composé n°28 : 1-[4-(6-Fluoro-1H-indol-3-yl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°29: 1-(4-Benzo[b]thiophèn-3-yl-3,6-dihydro-2H-pyridin-1-yl)-2-[8-(5-fluoro-pyrimidin-2-yl)-3,8-diaza-bicyclo[3.2.1]oct-3-yl]-éthanone ;
- Composé n°30 : 4-{2-[4-(1H-Indol-3-yl)-3,6-dihydro-2H-pyridin-1-yl]-2-oxo-éthyl}-1-(5-trifluorométhyl-pyridin-2-yl)-pipérazin-2-one ;
- Composé n°31 : 4-[2-(4-Benzo[b]thiophèn-7-yl-3,6-dihydro-2H-pyridin-1-yl)-2-oxo-éthyl]-1-(5-trifluorométhyl-pyridin-2-yl)-pipérazin-2-one ;
- Composé n°32: 4-[2-(4-Benzofuran-7-yl-3,6-dihydro-2H-pyridin-1-yl)-2-oxo-éthyl]-1-(5-trifluorométhyl-pyridin-2-yl)-pipérazin-2-one ;
- Composé n°33: 1-(4-Benzofuran-7-yl-3,6-dihydro-2H-pyridin-1-yl)-2-[4-(5-trifluorométhyl-pyridin-2-yl)-pipérazin-1-yl]-éthanone ;
- Composé n°34 : 1-(4-Benzofuran-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°35 : 1-(4-Benzofuran-7-yl-3,6-dihydro-2H-pyridin-1-yl)-2-((2S,6R)-2,6-diméthyl-4-pyrimidin-5-yl-pipérazin-1-yl)-éthanone ;
- Composé n°36 : 1-(4-Benzofuran-7-yl-3,6-dihydro-2H-pyridin-1-yl)-2-[(2S,6R)-4-(5-fluoro-pyrimidin-2-yl)-2,6-diméthyl-pipérazin-1-yl]-éthanone ;
- Composé n°37 : 1-(4-Benzofuran-7-yl-3,6-dihydro-2H-pyridin-1-yl)-2-[(2S,6R)-2,6-diméthyl-4-(6-trifluorométhyl-pyridin-3-yl)-pipérazin-1-yl]-éthanone ;
- Composé n°38 : 2-[(2S,6R)-4-(5-Fluoro-pyrimidin-2-yl)-2,6-diméthyl-pipérazin-1-yl]-1-[4-(2-méthyl-benzo[b]thiophèn-5-yl)-3,6-dihydro-2H-pyridin-1-yl]-éthanone ;
- Composé n°39 : 2-[(2S,6R)-2,6-Diméthyl-4-(6-trifluorométhyl-pyridin-3-yl)-pipérazin -1-yl]-1-[4-(2-méthyl-benzo[b]thiophèn-5-yl)-3,6-dihydro-2H-pyridin-1-yl]-éthanone ;
- Composé n°40 : 2-((2S,6R)-2,6-Diméthyl-4-pyrimidin-5-yl-pipérazin-1-yl)-1-[4-(2-méthyl-benzo[b]thiophèn-5-yl)-3,6-dihydro-2H-pyridin-1-yl]-éthanone ;
- Composé n°41 : 1-(4-Benzo[b]thiophèn-5-yl-3,6-dihydro-2H-pyridin-1-yl)-2-((2S,6R) -2,6-diméthyl-4-pyrimidin-5-yl-pipérazin-1-yl)-éthanone ;
- Composé n°42 : 1-(4-Benzo[b]thiophèn-5-yl-3,6-dihydro-2H-pyridin-1-yl)-2-[(2S,6R) -4-(5-fluoro-pyrimidin-2-yl)-2,6-diméthyl-pipérazin-1-yl]-éthanone ;
- Composé n°43 : 4-[2-(4-Benzo[b]thiophèn-5-yl-3,6-dihydro-2H-pyridin-1-yl)-2-oxo-éthyl]-1-(5-trifluorométhyl-pyridin-2-yl)-pipérazin-2-one ;
- Composé n°44 : 1-[4-(2,3-Dihydro-benzo[1,4]dioxin-6-yl)-3,6-dihydro-2H-pyridin-1-yl]-2-[(2S,6R)-4-(5-fluoro-pyrimidin-2-yl)-2,6-diméthyl-pipérazin-1-yl]-éthanone ;
- Composé n°45: 1-[4-(2,3-Dihydro-benzo[1,4]dioxin-6-yl)-3,6-dihydro-2H-pyridin-1-yl]-2-[(2S,6R);-2,6-diméthyl-4-(6-trifluorométhyl-pyridin-3-yl)-pipérazin-1-yl]-éthanone ;
- Composé n°46 : 1-(4-Benzo[b]thiophèn-5-yl-3,6-dihydro-2H-pyridin-1-yl)-2-[4-(5-trifluorométhyl-pyridin-2-yl)-pipérazin-1-yl]-éthanone ;
- Composé n°47 : 1-(4-Benzo[b]thiophèn-5-yl-3,6-dihydro-2H-pyridin-1-yl)-2-[(2S,6R)-2,6-diméthyl-4-(6-trifluorométhyl-pyridin-3-yl)-pipérazin-1-yl]-éthanone ;
- Composé n°48 : Acide 5-{(3S,5R)-4-[2-(4-Benzo[b]thiophèn-4-yl-3,6-dihydro-2H-pyridin-1-yl)-2-oxo-éthyl]-3,5-diméthyl-pipérazin-1-yl}-pyridine-2-carboxylique ;
- Composé n°49 : 4-{2-[4-(2,3-Dihydro-benzo[1,4]dioxin-6-yl)-3,6-dihydro-2H-pyridin-1-yl]-2-oxo-éthyl}-1-(6-trifluorométhyl-pyridin-3-yl)-pipérazin-2-one ;
- Composé n°50 : 1-(4-Benzo[b]thiophèn-7-yl-3,6-dihydro-2H-pyridin-1-yl)-2-[4-(6-trifluorométhyl-pyridin-3-yl)-pipérazin-1-yl]-éthanone ;
- Composé n°51 : Méthyl ester de l'acide 5-{(3S,5R)-4-[2-(4-Benzofuran-7-yl-3,6-dihydro-2H-pyridin-1-yl)-2-oxo-éthyl]-3,5-diméthyl-pipérazin-1-yl}-pyridine-2-carboxylique ;
- Composé n°52 : Méthyl ester de l'acide 6-{(3S,5R)-4-[2-(4-Benzo[b]thiophèn-5-yl-3,6-dihydro-2H-pyridin-1-yl)-2-oxo-éthyl]-3,5-diméthyl-pipérazin-1-yl}-nicotinique ;
Composé n°53 : 1-(4-Benzo[b]thiophèn-7-yl-3,6-dihydro-2H-pyridin-1-yl)-2-[(2S,6R) -4-(5-fluoro-pyrimidin-2-yl)-2,6-diméthyl-pipérazin-1-yl]-éthanone ;
- Composé n°54 : 1-(4-Benzo[b]thiophèn-7-yl-3,6-dihydro-2H-pyridin-1-yl)-2-[(2S,6R) -2,6-diméthyl-4-(6-trifluorométhyl-pyridin-3-yl)-pipérazin-1-yl]-éthanone ;
- Composé n°55 : 1-(4-Benzo[b]thiophèn-7-yl-3,6-dihydro-2H-pyridin-1-yl)-2-((2S,6R)-2,6-diméthyl-4-pyrimidin-5-yl-pipérazin-1-yl)-éthanone ;
- Composé n°56 : Acide 6-{(3S,5R)-4-[2-(4-Benzo[b]thiophèn-5-yl-3,6-dihydro-2H-pyridin-1-yl)-2-oxo-éthyl]-3,5-diméthyl-pipérazin-1-yl}-nicotinique ;
- Composé n°57 : Acide 6-{(3S,5R)-4-[2-(4-Benzofuran-7-yl-3,6-dihydro-2H-pyridin-1-yl)-2-oxo-éthyl]-3,5-diméthyl-pipérazin-1-yl}-nicotinique ;
- Composé n°58 : Méthyl ester de l'acide 6-{(3S,5R)-4-[2-(4-Benzo[b]thiophèn-7-yl-3,6-dihydro-2H-pyridin-1-yl)-2-oxo-éthyl]-3,5-diméthyl-pipérazin-1-yl}-nicotinique ;
- Composé n°59 : Acide 6-{(3S,5R)-4-[2-(4-Benzo[b]thiophèn-7-yl-3,6-dihydro-2H-pyridin-1-yl)-2-oxo-éthyl]-3,5-diméthyl-pipérazin-1-yl}-nicotinique ;
- Composé n°60 : 4-[2-(4-Benzofuran-5-yl-3,6-dihydro-2H-pyridin-1-yl)-2-oxo-éthyl]-1-(5-trifluorométhyl-pyridin-2-yl)-pipérazin-2-one ;
- Composé n°61 : Méthyl ester de l'acide 6-((3S,5R)-3,5-Diméthyl-4-{2-[4-(2-méthyl-benzo[b]thiophèn-5-yl)-3,6-dihydro-2H-pyridin-1-yl]-2-oxo-éthyl}-pipérazin-1-yl)-nicotinique ;
- Composé n°62 :Acide 6-((3S,5R)-3,5-Diméthyl-4-{2-[4-(2-méthyl-benzo[b]thiophèn-5-yl)-3,6-dihydro-2H-pyridin-1-yl]-2-oxo-éthyl}-pipérazin-1-yl)-nicotinique ;
- Composé n°63 : 1-(5-Benzofuran-7-yl-3,6-dihydro-2H-pyridin-1-yl)-2-[(2S,6R)-2,6-diméthyl-4-(5-trifluorométhyl-pyridin-2-yl)-pipérazin-1-yl]-éthanone ;
- Composé n°64 : Ethyl ester de l'acide 6-{(3S,5R)-4-[2-(4-Benzofuran-7-yl-3,6-dihydro-2H-pyridin-1-yl)-2-oxo-éthyl]-3,5-diméthyl-pipérazin-1-yl}-nicotinique ;
- Composé n°65 :Méthyl ester de l'acide 6-{3-[2-(4-Benzofuran-7-yl-3,6-dihydro-2H-pyridin-1-yl)-2-oxo-éthyl]-3,8-diaza-bicyclo[3.2.1]oct-8-yl}-nicotinique;
- Composé n°66 : Méthyl ester de l'acide 7-(1-{2-[(2S,6R)-2,6-Diméthyl-4-(5-trifluorométhyl-pyridin-2-yl)-pipérazin-1-yl]-acétyl}-1,2,3,6-tétrahydro-pyridin-4-yl)-benzo[b]thiophène-2-carboxylique ;
- Composé n°67 : Méthyl ester de l'acide 5-(1-{2-[(2S,6R)-2,6-Diméthyl-4-(5-trifluorométhyl-pyridin-2-yl)-pipérazin-1-yl]-acétyl}-1,2,3,6-tétrahydro-pyridin-4-yl)-benzo[b]thiophène-2-carboxylique ;
- Composé n°68 : 2-[(2S,6R)-2,6-Diméthyl-4-(5-trifluorométhyl-pyridin-2-yl)-pipérazin -1-yl]-1-[4-(2-propyl-benzo[b]thiophèn-5-yl)-3,6-dihydro-2H-pyridin-1-yl]-éhanone ;
- Composé n°69: Méthtyl ester de l'acide 6-{3-[2-(4-Benzo[b]thiophèn-7-yl-3,6-dihydro-2H-pyridin-1-yl)-2-oxo-éthyl]-3,8-diaza-bicyclo[3.2.1]oct-8-yl}-nicotinique ;
- Composé n°70: Méthyl ester de l'acide 6-{3-[2-(4-Benzo[b]thiophèn-5-yl-3,6-dihydro-2H-pyridin-1-yl)-2-oxo-éthyl]-3,8-diaza-bicyclo[3.2.1]oct-8-yl}-nicotinique ;
- Composé n°71 : Acide 6-{3-[2-(4-Benzo[b]thiophèn-7-yl-3,6-dihydro-2H-pyridin-1-yl)-2-oxo-éthyl]-3,8-diaza-bicyclo[3.2.1]oct-8-yl}-nicotinique ;
- Composé n°72 : 2-[(2S,6R)-2,6-Diméthyl-4-(5-trifluorométhyl-pyridin-2-yl)-pipérazin -1-yl]-1-[4-(7-fluoro-benzofuran-5-yl)-3,6-dihydro-2H-pyridin-1-yl]-éthanone ;
- Composé n°73 : 1-[4-(2,3-Diméthyl-benzofuran-6-yl)-3,6-dihydro-2H-pyridin-1-yl]-2-[(2S,6R) -2,6-diméthyl-4-(5-trifluorométhyl-pyridin-2-yl)-pipérazin-1-yl]-éthanone ;
- Composé n°74 : 2-[(2S,6R)-2,6-Diméthyl-4-(5-trifluorométhyl-pyridin-2-yl)-pipérazin -1-yl]-1-(4-quinolein-2-yl-3,6-dihydro-2H-pyridin-1-yl)-éthanone ;
- Composé n°75 : 1-(4-Benzofuran-5-yl-3,6-dihydro-2H-pyridin-1-yl)-2-(2,3,5,6-tétrahydro-[1,2']bipyrazinyl-4-yl)-éthanone ;
- Composé n°76 : Méthyl ester de l'acide 6-{3-[2-Oxo-2-(4-thiéno[3,2-c]pyridin-4-yl-3,6-dihydro-2H-pyridin-1-yl)-éthyl]-3,8-diaza-bicyclo[3.2.1]oct-8-yl}-nicotinique ;
- Composé n°77 : Méthyl ester de l'acide 6-(3-{2-[4-(2-Méthyl-benzo[b]thiophèn-5-yl) -3,6-dihydro-2H-pyridin-1-yl]-2-oxo-éthyl}-3,8-diaza-bicyclo[3.2.1]oct-8-yl)-nicotinique ;
- Composé n°78 : 1-(4-Benzofuran-7-yl-3,6-dihydro-2H-pyridin-1-yl)-2-(4-pyridin-3-yl-[1,4]diazepan-1-yl)-éthanone ;
- Composé n°79 : Acide 6-{3-[2-Oxo-2-(4-thiéno[3,2-c]pyridin-4-yl-3,6-dihydro-2H-pyridin-1-yl)-éthyl]-3,8-diaza-bicyclo[3.2.1]oct-8-yl}-nicotinique ;
- Composé n°80 : Acide 6-(3-{2-[4-(2-Méthyl-benzo[b]thiophèn-5-yl)-3,6-dihydro-2H-pyridin-1-yl]-2-oxo-éthyl}-3,8-diaza-bicyclo[3.2.1]oct-8-yl)-nicotinique ;
- Composé n°81 : Méthyl ester de l'acide 6-{3-[2-(3-Benzofuran-7-yl-2,5-dihydro-pyrrol-1-yl)-2-oxo-éthyl]-3,8-diaza-bicyclo[3.2.1]oct-8-yl}-nicotinique ;
- Composé n°82 : Méthyl ester de l'acide 6-{3-[2-(3-Benzo[b]thiophèn-7-yl-2,5-dihydro-pyrrol-1-yl)-2-oxo-ethyl]-3,8-diaza-bicyclo[3.2.1]oct-8-yl}-nicotinique ;
- Composé n°83 : 4-[2-(3-Benzofuran-7-yl-2,5-dihydro-pyrrol-1-yl)-2-oxo-éthyl]-1-(5-trifluorométhyl-pyridin-2-yl)-pipérazin-2-one ;
- Composé n°84 : acide 6-{3-[2-(4-Benzofuran-7-yl-2,3-dihydro-pyrrol-1-yl)-2-oxo-ethyl]-3,8-diaza-bicyclo[3.2.1]oct-8-yl}-nicotinique;
- Composé n°85 : acide 6-{3-[2-(5-Benzo[b]thiophen-7-yl-3,4-dihydro-2H-pyridin-1-yl) -2-oxo-ethyl]-3,8-diaza-bicyclo[3.2.1]oct-8-yl}-nicotinique;
- Composé n°86 : acide 6-{3-[2-(4-Benzo[b]thiophen-7-yl-2,3-dihydro-pyrrol-1-yl)-2-oxo-ethyl]-3,8-diaza-bicyclo[3.2.1]oct-8-yl}-nicotinique ;
- Composé n°87 : méthyl ester de l'acide 6-(3-{2-[4-(2,3-Dihydro-benzo[1,4]dioxin-6-yl)-3,6-dihydro-2H-pyridin-1-yl]-2-oxo-ethyl}-3,8-diaza-bicyclo[3.2.1]oct-8-yl)-nicotinique;
- Composé n°88 : acide 2-{(3S,5R)-4-[2-(4-Benzo[b]thiophen-7-yl-3,6-dihydro-2H-pyridin-1-yl)-2-oxo-ethyl]-3,5-dimethyl-piperazin-1-yl}-pyrimidine-5-carboxylique;
- Composé n°89 : 3-(6-{3-[2-(4-Benzofuran-7-yl-3,6-dihydro-2H-pyridin-1-yl)-2-oxo-ethyl]-3,8-diaza-bicyclo[3.2.1]oct-8-yl}-pyridin-3-yl)-4H-[1,2,4]oxadiazol-5-one ;
- Composé n°90 : 3-(6-{3-[2-(4-Benzo[b]thiophen-7-yl-3,6-dihydro-2H-pyridin-1-yl)-2-oxo-ethyl]-3,8-diaza-bicyclo[3.2.1]oct-8-yl}-pyridin-3-yl)-4H-[1,2,4]oxadiazol-5-one ;
- Composé n°91 : 6-(3-{2-[4-(2-Methyl-benzo[b]thiophen-5-yl)-3,6-dihydro-2H-pyridin-1-yl]-2-oxo-ethyl}-3,8-diaza-bicyclo[3.2.1]oct-8-yl)-nicotinonitrile ;
- Composé n°92 : 1-(4-Benzofuran-7-yl-3,6-dihydro-2H-pyridin-1-yl)-2-[(2S,6R)-4-(3-chloro-5-trifluoromethyl-pyridin-2-yl)-2,6-dimethyl-piperazin-1-yl]-ethanone ;
- Composé n°93 : 1-(4-Benzofuran-7-yl-3,6-dihydro-2H-pyridin-1-yl)-2-(8-pyridin-3-yl-3,8-diaza-bicyclo[3.2.1]oct-3-yl)-ethanone ;
- Composé n°94 : 6-{3-[2-(4-Benzofuran-7-yl-3,6-dihydro-2H-pyridin-1-yl)-2-oxo-ethyl]-3,8-diaza-bicyclo[3.2.1]oct-8-yl}-nicotinonitrile ;
- Composé n°95 : 1-(4-Benzofuran-7-yl-3,6-dihydro-2H-pyridin-1-yl)-2-[5-(5-trifluoromethyl-pyridin-2-yl)-2,5-diaza-bicyclo[2.2.1]hept-2-yl]-ethanone ;
- Composé n°96 : cyclobutyl ester de l'acide 6-(3-{2-[4-(2-Methyl-benzo[b]thiophen-5 -yl)-3,6-dihydro-2H-pyridin-1-yl]-2-oxo-ethyl}-3,8-diaza-bicyclo[3.2.1]oct-8-yl)-nicotinique ;
- Composé n°97 : 2-[(2S,6R)-2,6-Dimethyl-4-(5-trifluoromethyl-pyridin-2-yl)-piperazin -1-yl]-1-[4-(1H-indol-3-yl)-3,6-dihydro-2H-pyridin-1-yl]-ethanone ;
- Composé n°98 : 1-[4-(1H-Indol-3-yl)-3,6-dihydro-2H-pyridin-1-yl]-2-(4-quinolin-2-yl-piperazin-1-yl)-ethanone ;
- Composé n°99 : 1-(4-Benzofuran-7-yl-3,6-dihydro-2H-pyridin-1-yl)-2-[4-(7-chloro-quinolin-4-yl)-piperazin-1-yl]-ethanone ;
- Composé n°100: 2-[(2S,6R)-4-(5-Fluoro-pyrimidin-2-yl)-2,6-dimethyl-piperazin-1-yl]-1-[4-(1H-indol-3-yl)-3,6-dihydro-2H-pyridin-1-yl]-ethanone ;
- Composé n°101 : 1-(4-Benzo[b]thiophen-7-yl-3,6-dihydro-2H-pyridin-1-yl)-2-[(2S,6R)-4-(5-chloro-pyridin-2-yl)-2,6-dimethyl-piperazin-1-yl]-ethanone ;
- Composé n°102 : 1-(4-Benzo[b]thiophen-7-yl-3,6-dihydro-2H-pyridin-1-yl)-2-((2R,5S)-2,5-dimethyl-2,3,5,6-tetrahydro-[1,2']bipyrazinyl-4-yl)-ethanone ;
- Composé n°103 : 1-(4-Benzo[b]thiophen-7-yl-3,6-dihydro-2H-pyridin-1-yl)-2-[8-(5-fluoro-pyrimidin-2-yl)-3,8-diaza-bicyclo[3.2.1]oct-3-yl]-ethanone ;
- Composé n°104 : 1-(4-Benzo[b]thiophen-7-yl-3,6-dihydro-2H-pyridin-1-yl)-2-[8-(6-trifluoromethyl-pyridin-2-yl)-3,8-diaza-bicyclo[3.2.1]oct-3-yl]-ethanone ;
- Composé n°105 : 1-(4-Benzo[b]thiophen-7-yl-3,6-dihydro-2H-pyridin-1-yl)-2-(4-quinolin-2-yl-piperazin-1-yl)-ethanone ;
- Composé n°106 : 1-(4-Benzo[b]thiophen-7-yl-3,6-dihydro-2H-pyridin-1-yl)-2-[4-(7-chloro-quinolin-4-yl)-piperazin-1-yl]-ethanone ;
- Composé n°107 : 1-(4-Benzo[b]thiophen-7-yl-3,6-dihydro-2H-pyridin-1-yl)-2-[4-(5-chloro-pyridin-2-yl)-piperazin-1-yl]-ethanone ;
- Composé n°108 : 2-[4-(6-Chloro-pyridin-2-yl)-piperazin-1-yl]-1-[4-(1H-indol-3-yl)-3,6-dihydro-2H-pyridin-1-yl]-ethanone ;
- Composé n°109 : 1-[4-(1H-Indol-3-yl)-3,6-dihydro-2H-pyridin-1-yl]-2-[4-(5-trifluoromethyl-pyridin-2-yl)-piperazin-1-yl]-ethanone ;
- Composé n°110 : 2-((2S,6R)-2,6-Dimethyl-4-quinolin-2-yl-piperazin-1-yl)-1-[4-(1H-indol-3-yl)-3,6-dihydro-2H-pyridin-1-yl]-ethanone ;
- Composé n°111 : 1-(4-Benzo[b]thiophen-7-yl-3,6-dihydro-2H-pyridin-1-yl)-2-(4-pyridin-3-yl-[1,4]diazepan-1-yl)-ethanone ;
- Composé n°112 : 1-(4-Benzo[b]thiophen-7-yl-3,6-dihydro-2H-pyridin-1-yl)-2-[4-(5,6-dichloro-pyridin-2-yl)-piperazin-1-yl]-ethanone ;
- Composé n°113 : 1-(4-Benzo[b]thiophen-7-yl-3,6-dihydro-2H-pyridin-1-yl)-2-[4-(6-bromo-pyridin-2-yl)-piperazin-1-yl]-ethanone ;
- Composé n°114 : 1-[4-(2-Methyl-benzo[b]thiophen-5-yl)-3,6-dihydro-2H-pyridin-1-yl]-2-(4-quinolin-2-yl-piperazin-1-yl)-ethanone ;
- Composé n°115 : 1-(4-Benzo[b]thiophen-7-yl-3,6-dihydro-2H-pyridin-1-yl)-2-[5-(6-trifluoromethyl-pyridazin-3-yl)-2,5-diaza-bicyclo[2.2.1]hept-2-yl]-ethanone ;
- Composé n°116 : 1-[4-(2-Methyl-benzo[b]thiophen-5-yl)-3,6-dihydro-2H-pyridin-1-yl]-2-[4-(6-trifluoromethyl-pyridin-2-yl)-piperazin-1-yl]-ethanone ;
- Composé n°117 : 2-[4-(7-Chloro-quinolin-4-yl)-piperazin-1-yl]-1-[4-(2-methyl-benzo[b]thiophen-5-yl)-3,6-dihydro-2H-pyridin-1-yl]-ethanone ;
- Composé n°118 : 4-[2-Oxo-2-(4-thieno[3,2-c]pyridin-4-yl-3,6-dihydro-2H-pyridin-1-yl)-ethyl]-1-(5-trifluoromethyl-pyridin-2-yl)-piperazin-2-one ;
- Composé n°119 : 1-(4-Benzofuran-7-yl-3,6-dihydro-2H-pyridin-1-yl)-2-((2S,6R)-2,6-dimethyl-4-(5-thiazol-2-yl-pyridin-2-yl)-piperazin-1-yl]-ethanone ;
- Composé n°120 : 1-(4-Benzofuran-7-yl-3,6-dihydro-2H-pyridin-1-yl)-2-{(2S,6R)-2,6-dimethyl-4-[5-(2-methyl-2H-tetrazol-5-yl)-pyridin-2-yl]-piperazin-1-yl}-ethanone ;
- Composé n°121 : 2-[(2S,6R)-2,6-Dimethyl-4-(5-trifluoromethyl-pyridin-2-yl)-piperazin-1-yl]-1-(4-quinolin-2-yl-3,6-dihydro-2H-pyridin-1-yl)-ethanone ;
- Composé n°122 : 4-[2-Oxo-2-(4-quinolin-2-yl-3,6-dihydro-2H-pyridin-1-yl)-ethyl]-1-(5-trifluoromethyl-pyridin-2-yl)-piperazin-2-one ;
- Composé n°123 : 1-(4-Quinolin-2-yl-3,6-dihydro-2H-pyridin-1-yl)-2-[8-(5-trifluoromethyl-pyridin-2-yl)-3,8-diaza-bicyclo[3.2.1]oct-3-yl]-ethanone ;
- Composé n°124 : 1-(4-Benzofuran-7-yl-3,6-dihydro-2H-pyridin-1-yl)-2-{8-[5-(1-methyl-1H-tetrazol-5-yl)-pyridin-2-yl]-3,8-diaza-bicyclo[3.2.1]oct-3-yl}-ethanone ;
- Composé n°125 : 1-(4-Benzofuran-7-yl-3,6-dihydro-2H-pyridin-1-yl)-2-[(2S,6R)-4-(5-methanesulfonyl-pyridin-2-yl)-2,6-dimethyl-piperazin-1-yl]-ethanone ;
à l'état de base ou de sel d'addition à un acide.

7. Procédé de préparation d'un composé de formule (I) selon l'une quelconque des revendications 1 à 6, **caractérisé en ce que** l'on fait réagir un composé de formule (II) : 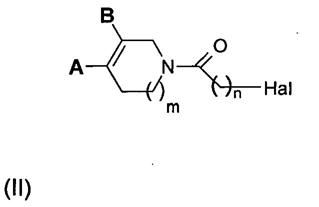
dans laquelle A, B, m et n sont définis selon l'une quelconque des revendications 1 à 6 et Hal représente un atome d'halogène,
et un composé de formule générale (III) :
H-W-R2 (III)
dans laquelle W et R2 sont définis selon l'une quelconque des revendications 1 à 6.

8. Médicament, **caractérisé en ce qu'**il comprend un composé de formule (I) selon l'une quelconque des revendications 1 à 6, ou un sel d'addition de ce composé à un acide pharmaceutiquement acceptable.

9. Composition pharmaceutique, **caractérisée en ce qu'**elle comprend un composé de formule (I) selon l'une quelconque des revendications 1 à 6, ou un sel pharmaceutiquement acceptable, ainsi qu'au moins un excipient pharmaceutiquement acceptable.

10. Composé selon l'une quelconque des revendications 1 à 6 comme médicament.

11. Composé selon l'une quelconque des revendications 1 à 6 pour la préparation d'un médicament destiné à la prévention ou le traitement de maladies neurodégénératives centrales et périphériques, la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire, la sclérose latérale amyotrophique, la sclérose en plaques, les affections cardiovasculaires, les dommages cardiaques post-ischémiques, les cardiomyopathies, l'infarctus du myocarde, l'insuffisance cardiaque, l'ischémie cardiaque, l'infarctus cérébral ; les neuropathies périphériques, les dommages au nerf optique et à la rétine, dégénérescence du pigment rétinien, le glaucome, l'ischémie rétinale , la dégénérescence maculaire, les traumatismes de la moelle épinière, les traumatismes crâniens, l'athérosclérose, les sténoses, les désordres de la cicatrisation, l'alopécie, la pancréatite, la fibrose hépatique, les cancers, les tumeurs, les métastases, les leucémies, les désordres respiratoires, l'inflammation pulmonaire, l'allergie, l'asthme, la broncho-pneumopathie chronique obstructive, la douleur cutanée, somatique, viscérale, et neurologique, les douleurs chroniques neuropathiques et inflammatoires, les maladies auto-immunes, la polyarthrite rhumatoïde, la spondylarthrite ankylosante, le rhumatisme psoriasique, le psoriasis en plaques, les fractures osseuses, les maladies osseuses, l'ostéoporose.

12. Composé selon l'une des revendications 1 à 6 en tant qu'inhibiteur de la dimérisation du récepteur p75^{NTR}.

13. Utilisation d'un composé selon l'une des revendications 1 à 6 pour la préparation de médicaments utiles dans le traitement et la prévention de maladies neurodégénératives centrales et périphériques, la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire, la sclérose latérale amyotrophique, la sclérose en plaques, les affections cardiovasculaires, les dommages cardiaques post-ischémiques, les cardiomyopathies, l'infarctus du myocarde, l'insuffisance cardiaque, l'ischémie cardiaque, l'infarctus cérébral ; les neuropathies périphériques, les dommages au nerf optique et à la rétine, dégénérescence du pigment rétinien, le glaucome, l'ischémie rétinale , la dégénérescence maculaire, les traumatismes de la moelle épinière, les traumatismes crâniens, l'athérosclérose, les sténoses, les désordres de la cicatrisation, l'alopécie, la pancréatite, la fibrose hépatique, les cancers, les tumeurs, les métastases, les leucémies, les désordres respiratoires, l'inflammation pulmonaire, l'allergie, l'asthme, la broncho-pneumopathie chronique obstructive, la douleur cutanée, somatique, viscérale, et neurologique, les douleurs chroniques neuropathiques et inflammatoires, les maladies auto-immunes, la polyarthrite rhumatoïde, la spondylarthrite ankylosante, le rhumatisme psoriasique, le psoriasis en plaques, les fractures osseuses, les maladies osseuses, l'ostéoporose.

## Patentansprüche

1. Verbindung der Formel (I): 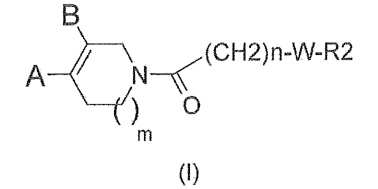 worin:
- n für 1 oder 2 steht;
- m für 0 oder 1 steht;
- A für eine kondensierte heterocyclische Gruppe der Formel (Y) 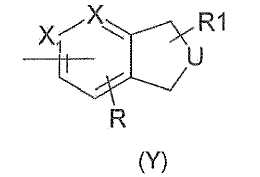
steht und B für ein Wasserstoffatom steht;
oder
A für ein Wasserstoffatom steht
und B für eine kondensierte heterocyclische Gruppe der Formel (Y) 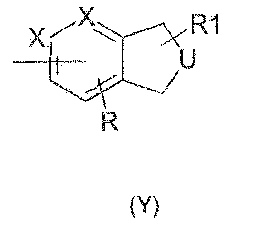 steht,
wobei der kondensierte Heterocyclus der Formel Y über ein beliebiges der verfügbaren Kohlenstoffatome an den Rest des Moleküls gebunden sein kann und wobei:
- U Folgendes vervollständigt:
- entweder einen aromatischen oder gesättigten Kern mit 6 Atomen, der 1 oder 2 Stickstoffatome enthält, wobei der Kern durch ein oder zwei Halogenatome, eine oder zwei (C1-C4)-Alkyl- oder (C1-C4)-Alkoxygruppen oder einen oder zwei Perfluoralkylreste substituiert sein kann;
- oder einen aromatischen oder gesättigten Kern mit 5 Atomen, der ein Stickstoff-, Sauerstoffoder Schwefelatom enthält, wobei der Kern durch eine oder zwei (C1-C4)-Alkylgruppen substituiert sein kann;
- X und X1 für CH oder N stehen;
- R und R1 an einer beliebigen der verfügbaren Positionen stehen und unabhängig voneinander für ein Wasserstoffatom, ein Halogenatom, eine (C1-C4)-Alkylgruppe, eine (C1-C4)-Alkoxygruppe, einen Perfluoralkylrest, einen Trifluormethoxyrest, einen Cyanorest, eine COOH-Gruppe, eine COO-Alkyl-Gruppe, eine CONR3R4-Gruppe oder eine NHCOR3-Gruppe stehen;
- -W- für einen stickstoffhaltigen Heterocyclus steht, der aus:
-W-= 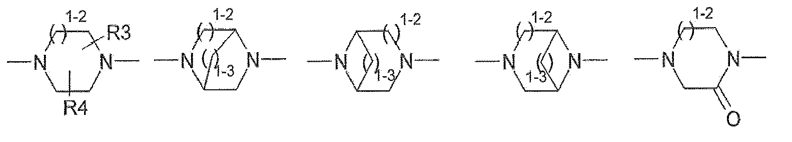 ausgewählt ist;
- 1-2 für 1 oder 2 steht;
- 1-3 für 1, 2 oder 3 steht;
- R2 für eine Gruppe der Formel: 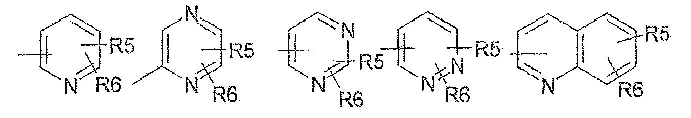 steht,
- worin R5 und R6 an einer beliebigen der verfügbaren Positionen stehen und unabhängig voneinander für ein Wasserstoffatom, ein Halogenatom, eine (C1-C4)-Alkylgruppe, eine (C1-C4)-Alkoxygruppe, einen Trifluormethylrest, einen Trifluormethoxyrest, einen Cyanorest, eine COOH-Gruppe, eine COO-Alkyl-Gruppe, eine COO-Cycloalkyl-Gruppe, eine SO-Alkyl-Gruppe, eine SO₂-Alkyl-Gruppe, eine CONR3R4-Gruppe, eine NR3R4-Gruppe oder eine NHCOR3-Gruppe stehen;
oder eine der Gruppen R5 und R6 auch für einen Heterocyclus, der aus: 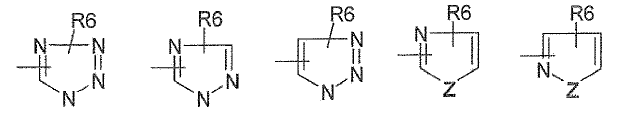 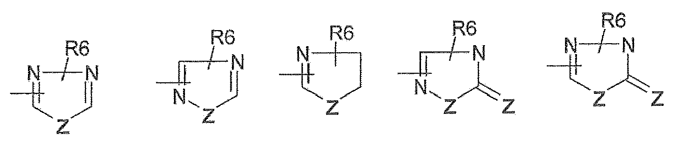 ausgewählt ist, stehen kann;
- Z für ein Sauerstoff- oder Schwefelatom steht;
- R3 und R4 für ein Wasserstoffatom oder eine (C1-C6)-Alkylgruppe stehen;
in Basen- oder Säureadditionssalzform.

2. Verbindung nach Anspruch 1, wobei
- A für eine kondensierte heterocyclische Gruppe der Formel (Y) 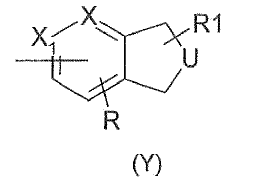 steht und B für ein Wasserstoffatom steht;
oder
A für ein Wasserstoffatom steht
und B für eine kondensierte heterocyclische Gruppe der Formel (Y) 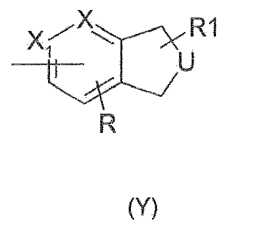 steht,
wobei der kondensierte Heterocyclus der Formel Y über ein beliebiges der verfügbaren Kohlenstoffatome des Benzolkerns an den Rest des Moleküls gebunden sein kann;
in Basen- oder Säureadditionssalzform.

3. Verbindung nach einem der Ansprüche 1 bis 2, wobei R und R1 an einer beliebigen der verfügbaren Positionen stehen und unabhängig voneinander für ein Wasserstoffatom, ein Halogenatom, eine (C1-C4)-Alkylgruppe oder eine COO-Alkyl-Gruppe stehen; in Basen- oder Säureadditionssalzform.

4. Verbindung nach einem der Ansprüche 1 bis 3, wobei:
- -W- für einen stickstoffhaltigen Heterocyclus steht, der aus:
-W-= 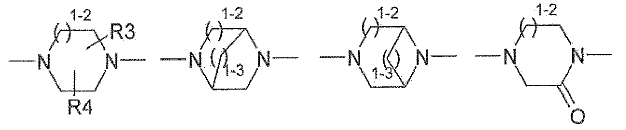 ausgewählt ist;
- R3 und R4 für ein Wasserstoffatom oder eine Methylgruppe stehen;
in Basen- oder Säureadditionssalzform.

5. Verbindung nach einem der Ansprüche 1 bis 4, wobei:
- R2 für eine Gruppe der Formel: 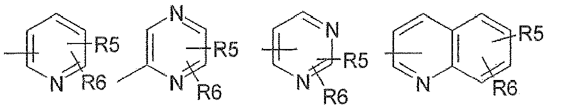 steht; 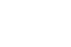
- R5 und R6 an einer beliebigen der verfügbaren Positionen stehen und unabhängig voneinander für ein Wasserstoffatom, einen Trifluormethylrest, eine COOH-Gruppe, eine COO-Alkyl-Gruppe oder eine COO-Cycloalkyl-Gruppe stehen; oder
eine der Gruppen R5 und R6 auch für einen Heterocyclus, der aus: 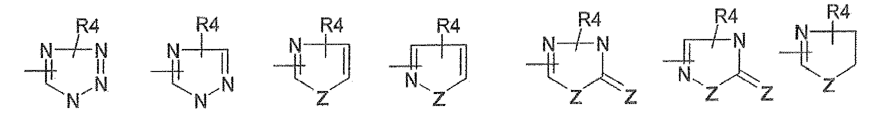 ausgewählt ist, stehen kann;
- Z für ein Sauerstoff- oder Schwefelatom steht;
- R3 und R4 für ein Wasserstoffatom oder eine Methylgruppe stehen;
in Basen- oder Säureadditionssalzform.

6. Verbindung nach einem der Ansprüche 1 bis 5, ausgewählt aus:
Verbindung 1: 1-(4-Benzo[b]thiophen-7-yl-3,6-dihydro-2H-pyridin-1-yl)-2-(3,5-dimethyl-2,3,5,6-tetrahydro[1,2']bipyrazinyl-4-yl)ethanon;
Verbindung 2: 1-(4-Benzo[b]thiophen-7-yl-3,6-dihydro-2H-pyridin-1-yl)-2-(8-pyrimidin-2-yl-3,8-diazabicyclo[3.2.1]oct-3-yl)ethanon;
Verbindung 3: 1-(4-Benzo[b]thiophen-5-yl-3,6-dihydro-2H-pyridin-1-yl)-2-(8-pyrimidin-2-yl-3,8-diazabicyclo[3.2.1]oct-3-yl)ethanon;
Verbindung 4: 1-(4-Benzo[b]thiophen-7-yl-3,6-dihydro-2H-pyridin-1-yl)-2-[(2S,6R)-2,6-dimethyl-4-(5-trifluormethylpyridin-2-yl)piperazin-1-yl]ethanon;
Verbindung 5: 1-(4-Benzo[b]thiophen-5-yl-3,6-dihydro-2H-pyridin-1-yl)-2-((2S,5R)-2,5-dimethyl-2,3,5,6-tetrahydro[1,2']bipyrazinyl-4-yl)ethanon;
Verbindung 6: 1-(4-Benzo[b]thiophen-7-yl-3,6-dihydro-2H-pyridin-1-yl)-2-((2S,5R)-2,5-dimethyl-2,3,5,6-tetrahydro[1,2']bipyrazinyl-4-yl)ethanon;
Verbindung 7: 1-(4-Benzo[b]thiophen-5-yl-3,6-dihydro-2H-pyridin-1-yl)-2-[(2S,6R)-2,6-dimethyl-4-(5-trifluormethylpyridin-2-yl)piperazin-1-yl]ethanon;
Verbindung 8: 1-(4-Benzo[b]thiophen-7-yl-3,6-dihydro-2H-pyridin-1-yl)-2-[8-(5-trifluormethylpyridin-2-yl)-3,8-diazabicyclo[3.2.1]oct-3-yl]ethanon;
Verbindung 9: 1-(4-Benzo[b]thiophen-7-yl-3,6-dihydro-2H-pyridin-1-yl)-2-[(2R,5S)-2,5-dimethyl-4-(5-trifluormethylpyridin-2-yl)piperazin-1-yl]ethanon;
Verbindung 10: 2-[(2S,6R)-2,6-Dimethyl-4-(5-trifluormethylpyridin-2-yl)piperazin-1-yl]-1-[4-(2-methylbenzo[b]thiophen-7-yl)-3,6-dihydro-2H-pyridin-1-yl]ethanon;
Verbindung 11: 2-[(2S,6R)-2,6-Dimethyl-4-(5-trifluormethylpyridin-2-yl)piperazin-1-yl]-1-[4-(2-propylbenzo[b]thiophen-7-yl)-3,6-dihydro-2H-pyridin-1-yl]ethanon;
Verbindung 12: 1-(4-Benzo[b]thiophen-7-yl-3,6-dihydro-2H-pyridin-1-yl)-2-[5-(5-trifluormethylpyridin-2-yl)-2,5-diazabicyclo[2.2.1]hept-2-yl]ethanon;
Verbindung 13: 1-(4-benzo[b]thiophen-6-yl-3,6-dihydro-2H-pyridin-1-yl)-2-[(2R,5S)-2,5-dimethyl-4-(5-trifluormethylpyridin-2-yl)piperazin-1-yl]ethanon;
Verbindung 14: 1-(4-Benzofuran-7-yl-3,6-dihydro-2H-pyridin-1-yl)-2-[(2R,5S)-2,5-dimethyl-4-(5-trifluormethylpyridin-2-yl)piperazin-1-yl]ethanon;
Verbindung 15: 1-[4-(2-Methylbenzo[b]thiophen-5-yl)-3,6-dihydro-2H-pyridin-1-yl]-2-(8-pyrimidin-2-yl-3,8-diazabicyclo[3.2.1]oct-3-yl)ethanon;
Verbindung 16: 1-[4-(2-Methylbenzo[b]thiophen-5-yl)-3,6-dihydro-2H-pyridin-1-yl]-2-[4-(5-trifluormethylpyridin-2-yl)piperazin-1-yl]ethanon;
Verbindung 17: 4-{2-[4-(2-Methylbenzo[b]thiophen-5-yl)-3,6-dihydro-2H-pyridin-1-yl]-2-oxoethyl}-1-(5-trifluormethylpyridin-2-yl)piperazin-2-on;
Verbindung 18: 2-[(2S,6R)-2,6-Dimethyl-4-(5-trifluormethylpyridin-2-yl)piperazin-1-yl]-1-[4-(2-methylbenzo[b]thiophen-5-yl)-3,6-dihydro-2H-pyridin-1-yl]ethanon;
Verbindung 19: 1-(4-Benzo[b]thiophen-7-yl-3,6-dihydro-2H-pyridin-1-yl)-2-((2S,6R)-2,6-dimethyl-4-chinolin-2-ylpiperazin-1-yl)ethanon;
Verbindung 20: 1-(4-Chinolin-8-yl-3,6-dihydro-2H-pyridin-1-yl)-2-[4-(5-trifluormethylpyridin-2-yl)piperazin-1-yl]ethanon;
Verbindung 21: 1-[4-(2,3-Dihydrobenzo[1,4]dioxin-6-yl)-3,6-dihydro-2H-pyridin-1-yl]-2-[4-(5-trifluormethylpyridin-2-yl)piperazin-1-yl]ethanon;
Verbindung 22: 2-[8-(5-Fluorpyrimidin-2-yl)-3,8-diazabicyclo[3.2.1]oct-3-yl]-1-[4-(2-methylbenzo[b]thiophen-5-yl)-3,6-dihydro-2H-pyridin-1-yl]ethanon;
Verbindung 23: 1-(4-Benzofuran-7-yl-3,6-dihydro-2H-pyridin-1-yl)-2-[8-(5-fluorpyrimidin-2-yl)-3,8-diazabicyclo[3.2.1]oct-3-yl]ethanon;
Verbindung 24: 1-[4-(2,3-Dihydrobenzo[1,4]dioxin-6-yl)-3,6-dihydro-2H-pyridin-1-yl]-2-[8-(5-fluorpyrimidin-2-yl)-3,8-diazabicyclo[3.2.1]oct-3-yl]ethanon;
Verbindung 25: 2-[8-(5-Fluorpyrimidin-2-yl)-3,8-diazabicyclo[3.2.1]oct-3-yl]-1-(4-thieno[3,2-c]pyridin-4-yl-3,6-dihydro-2H-pyridin-1-yl)ethanon;
Verbindung 26: 1-(4-Benzofuran-3-yl-3,6-dihydro-2H-pyridin-1-yl)-2-[8-(5-fluorpyrimidin-2-yl)-3,8-diazabicyclo[3.2.1]oct-3-yl]ethanon;
Verbindung 27: 2-[8-(5-Fluorpyrimidin-2-yl)-3,8-diazabicyclo[3.2.1]oct-3-yl]-1-[4-(1H-indol-3-yl)-3,6-dihydro-2H-pyridin-1-yl]ethanon;
Verbindung 28: 1-[4-(6-Fluor-1H-indol-3-yl)-3,6-dihydro-2H-pyridin-1-yl]-2-[8-(5-fluorpyrimidin-2-yl)-3,8-diazabicyclo[3.2.1]oct-3-yl]ethanon;
Verbindung 29: 1-(4-Benzo[b]thiophen-3-yl-3,6-dihydro-2H-pyridin-1-yl)-2-[8-(5-fluorpyrimidin-2-yl)-3,8-diazabicyclo[3.2.1]oct-3-yl]ethanon;
Verbindung 30: 4-{2-[4-(1H-Indol-3-yl)-3,6-dihydro-2H-pyridin-1-yl]-2-oxoethyl}-1-(5-trifluormethylpyridin-2-yl)piperazin-2-on;
Verbindung 31: 4-[2-(4-Benzo[b]thiophen-7-yl-3,6-dihydro-2H-pyridin-1-yl)-2-oxoethyl]-1-(5-trifluormethylpyridin-2-yl)piperazin-2-on;
Verbindung 32: 4-[2-(4-Benzofuran-7-yl-3,6-dihydro-2H-pyridin-1-yl)-2-oxoethyl]-1-(5-trifluormethylpyridin-2-yl)piperazin-2-on;
Verbindung 33: 1-(4-Benzofuran-7-yl-3,6-dihydro-2H-pyridin-1-yl)-2-[4-(5-trifluormethylpyridin-2-yl)piperazin-1-yl]ethanon;
Verbindung 34: 1-(4-Benzofuran-7-yl-3,6-dihydro-2H-pyridin-1-yl)-2-[(2S,6R)-2,6-dimethyl-4-(5-trifluormethylpyridin-2-yl)piperazin-1-yl]ethanon;
Verbindung 35: 1-(4-Benzofuran-7-yl-3,6-dihydro-2H-pyridin-1-yl)-2-((2S,6R)-2,6-dimethyl-4-pyrimidin-5-ylpiperazin-1-yl)ethanon;
Verbindung 36: 1-(4-Benzofuran-7-yl-3,6-dihydro-2H-pyridin-1-yl)-2-[(2S,6R)-4-(5-fluorpyrimidin-2-yl)-2,6-dimethylpiperazin-1-yl]ethanon;
Verbindung 37: 1-(4-Benzofuran-7-yl-3,6-dihydro-2H-pyridin-1-yl)-2-[(2S,6R)-2,6-dimethyl-4-(6-trifluormethylpyridin-3-yl)piperazin-1-yl]ethanon;
Verbindung 38: 2-[(2S,6R)-4-(5-Fluorpyrimidin-2-yl)-2,6-dimethylpiperazin-1-yl]-1-[4-(2-methylbenzo[b]thiophen-5-yl)-3,6-dihydro-2H-pyridin-1-yl]ethanon;
Verbindung 39: 2-[(2S,6R)-2,6-Dimethyl-4-(6-trifluormethylpyridin-3-yl)piperazin-1-yl]-1-[4-(2-methylbenzo[b]thiophen-5-yl)-3,6-dihydro-2H-pyridin-1-yl]ethanon;
Verbindung 40: 2-((2S,6R)-2,6-Dimethyl-4-pyrimidin-5-ylpiperazin-1-yl)-1-[4-(2-methylbenzo[b]thiophen-5-yl)-3,6-dihydro-2H-pyridin-1-yl]ethanon;
Verbindung 41: 1-(4-Benzo[b]thiophen-5-yl-3,6-dihydro-2H-pyridin-1-yl)-2-((2S,6R)-2,6-dimethyl-4-pyrimidin-5-ylpiperazin-1-yl)ethanon;
Verbindung 42: 1-(4-Benzo[b]thiophen-5-yl-3,6-dihydro-2H-pyridin-1-yl)-2-[(2S,6R)-4-(5-fluorpyrimidin-2-yl)-2,6-dimethylpiperazin-1-yl)ethanon;
Verbindung 43: 4-[2-(4-Benzo[b]thiophen-5-yl-3,6-dihydro-2H-pyridin-1-yl)-2-oxoethyl]-1-(5-trifluormethylpyridin-2-yl)piperazin-2-on;
Verbindung 44: 1-[4-(2,3-Dihydrobenzo[1,4]dioxin-6-yl)-3,6-dihydro-2H-pyridin-1-yl]-2-[(2S,6R)-4-(5-fluorpyrimidin-2-yl)-2,6-dimethylpiperazin-1-yl]ethanon;
Verbindung 45: 1-[4-(2,3-Dihydrobenzo[1,4]dioxin-6-yl)-3,6-dihydro-2H-pyridin-1-yl]-2-[(2S,6R)-2,6-dimethyl-4-(6-trifluormethylpyridin-3-yl)piperazin-1-yl]ethanon;
Verbindung 46: 1-(4-Benzo[b]thiophen-5-yl-3,6-dihydro-2H-pyridin-1-yl)-2-[4-(5-trifluormethylpyridin-2-yl)piperazin-1-yl]ethanon;
Verbindung 47: 1-(4-Benzo[b]thiophen-5-yl-3,6-dihydro-2H-pyridin-1-yl)-2-[(2S,6R)-2,6-dimethyl-4-(6-trifluormethylpyridin-3-yl)piperazin-1-yl]ethanon;
Verbindung 48: 5-{(3S,5R)-4-[2-(4-Benzo[b]thiophen-4-yl-3,6-dihydro-2H-pyridin-1-yl)-2-oxoethyl]-3,5-dimethylpiperazin-1-yl}pyridin-2-carbonsäure;
Verbindung 49: 4-{2-[4-(2,3-Dihydrobenzo[1,4]dioxin-6-yl)-3,6-dihydro-2H-pyridin-1-yl]-2-oxoethyl}-1-(6-trifluormethylpyridin-3-yl)piperazin-2-on;
Verbindung 50: 1-(4-Benzo[b]thiophen-7-yl-3,6-dihydro-2H-pyridin-1-yl)-2-[4-(6-trifluormethylpyridin-3-yl)piperazin-1-yl]ethanon;
Verbindung 51: 5-{(3S,5R)-4-[2-(4-Benzofuran-7-yl-3,6-dihydro-2H-pyridin-1-yl)-2-oxoethyl]-3,5-dimethylpiperazin-1-yl}pyridin-2-carbonsäuremethylester;
Verbindung 52: 6-{(3S,5R)-4-[2-(4-Benzo[b]thiophen-5-yl-3,6-dihydro-2H-pyridin-1-yl)-2-oxoethyl]-3,5-dimethylpiperazin-1-yl}nicotinsäuremethylester;
Verbindung 53: 1-(4-Benzo[b]thiophen-7-yl-3,6-dihydro-2H-pyridin-1-yl)-2-[(2S,6R)-4-(5-fluorpyrimidin-2-yl)-2,6-dimethylpiperazin-1-yl]ethanon;
Verbindung 54: 1-(4-Benzo[b]thiophen-7-yl-3,6-dihydro-2H-pyridin-1-yl)-2-[(2S,6R)-2,6-dimethyl-4-(6-trifluormethylpyridin-3-yl)piperazin-1-yl]ethanon;
Verbindung 55: 1-(4-Benzo[b]thiophen-7-yl-3,6-dihydro-2H-pyridin-1-yl)-2-((2S,6R)-2,6-dimethyl-4-pyrimidin-5-ylpiperazin-1-yl)ethanon;
Verbindung 56: 6-{(3S,5R)-4-[2-(4-Benzo[b]thiophen-5-yl-3,6-dihydro-2H-pyridin-1-yl)-2-oxoethyl]-3,5-dimethylpiperazin-1-yl}nicotinsäure;
Verbindung 57: 6-{(3S,5R)-4-[2-(4-Benzofuran-7-yl-3,6-dihydro-2H-pyridin-1-yl)-2-oxoethyl]-3,5-dimethylpiperazin-1-yl}nicotinsäure;
Verbindung 58: 6-{(3S,5R)-4-[2-(4-Benzo[b]thiophen-7-yl-3,6-dihydro-2H-pyridin-1-yl)-2-oxoethyl]-3,5-dimethylpiperazin-1-yl}nicotinsäuremethylester;
Verbindung 59: 6-{(3S,5R)-4-[2-(4-Benzo[b]thiophen-7-yl-3,6-dihydro-2H-pyridin-1-yl)-2-oxoethyl]-3,5-dimethylpiperazin-1-yl}nicotinsäure;
Verbindung 60: 4-[2-(4-Benzofuran-5-yl-3,6-dihydro-2H-pyridin-1-yl)-2-oxoethyl]-1-(5-trifluormethylpyridin-2-yl)piperazin-2-on;
Verbindung 61: 6-((3S,5R)-3,5-Dimethyl-4-{2-[4-(2-methylbenzo[b]thiophen-5-yl)-3,6-dihydro-2H-pyridin-1-yl]-2-oxoethyl}piperazin-1-yl)nicotinsäuremethylester;
Verbindung 62: 6-((3S,5R)-3,5-Dimethyl-4-{2-[4-(2-methylbenzo[b]thiophen-5-yl-)-3,6-dihydro-2H-pyridin-1-yl]-2-oxoethyl}piperazin-1-yl)nicotinsäure;
Verbindung 63: 1-(5-Benzofuran-7-yl-3,6-dihydro-2H-pyridin-1-yl)-2-[(2S,6R)-2,6-dimethyl-4-(5-trifluormethylpyridin-2-yl)piperazin-1-yl]ethanon;
Verbindung 64: 6-{(3S,5R)-4-[2-(4-Benzofuran-7-yl-3,6-dihydro-2H-pyridin-1-yl)-2-oxoethyl]-3,5-dimethylpiperazin-1-yl}nicotinsäureethylester;
Verbindung 65: 6-{3-[2-(4-Benzofuran-7-yl-3,6-dihydro-2H-pyridin-1-yl)-2-oxoethyl]-3,8-diazabicyclo[3.2.1]oct-8-yl}nicotinsäuremethylester;
Verbindung 66: 7-(1-{2-[(2S,6R)-2,6-Dimethyl-4-(5-trifluormethylpyridin-2-yl)piperazin-1-yl]acetyl}-1,2,3,6-tetrahydropyridin-4-yl)benzo[b]thiophen-2-carbonsäuremethylester;
Verbindung 67: 5-(1-{2-[(2S,6R)-2,6-Dimethyl-4-(5-trifluormethylpyridin-2-yl)piperazin-1-yl]acetyl}-1,2,3,6-tetrahydropyridin-4-yl)benzo[b]thiophen-2-carbonsäuremethylester;
Verbindung 68: 2-[(2S,6R)-2,6-Dimethyl-4-(5-trifluormethylpyridin-2-yl)piperazin-1-yl]-1-[4-(2-propylbenzo[b]thiophen-5-yl)-3,6-dihydro-2H-pyridin-1-yl]ethanon;
Verbindung 69: 6-{3-[2-(4-Benzo[b]thiophen-7-yl-3,6-dihydro-2H-pyridin-1-yl)-2-oxoethyl]-3,8-diazabicyclo[3.2.1]oct-8-yl}nicotinsäuremethylester;
Verbindung 70: 6-{3-[2-(4-Benzo[b]thiophen-5-yl-3,6-dihydro-2H-pyridin-1-yl)-2-oxoethyl]-3,8-diazabicyclo[3.2.1]oct-8-yl}nicotinsäuremethylester;
Verbindung 71: 6-{3-[2-(4-Benzo[b]thiophen-7-yl-3,6-dihydro-2H-pyridin-1-yl)-2-oxoethyl]-3,8-diazabicyclo[3.2.1]oct-8-yl}nicotinsäure;
Verbindung 72: 2-[(2S,6R)-2,6-Dimethyl-4-(5-trifluor-methylpyridin-2-yl)piperazin-1-yl]-1-[4-(7-fluorbenzofuran-5-yl)-3,6-dihydro-2H-pyridin-1-yl]ethanon;
Verbindung 73: 1-[4-(2,3-Dimethylbenzofuran-6-yl)-3,6-dihydro-2H-pyridin-1-yl]-2-[(2S,6R)-2,6-dimethyl-4-(5-trifluormethylpyridin-2-yl)piperazin-1-yl]ethanon;
Verbindung 74: 2-[(2S,6R)-2,6-Dimethyl-4-(5-trifluormethylpyridin-2-yl)piperazin-1-yl]-1-(4-chinolin-2-yl-3,6-dihydro-2H-pyridin-1-yl)ethanon;
Verbindung 75: 1-(4-Benzofuran-5-yl-3,6-dihydro-2H-pyridin-1-yl)-2-(2,3,5,6-tetrahydro[1,2']bipyrazinyl-4-yl)ethanon;
Verbindung 76: 6-13-[2-Oxo-2-(4-thieno[3,2-c]pyridin-4-yl-3,6-dihydro-2H-pyridin-1-yl)ethyl]-3,8-diazabicyclo[3.2.1]oct-8-yl}nicotinsäuremethylester;
Verbindung 77: 6-(3-{2-[4-(2-Methylbenzo[b]thiophen-5-yl)-3,6-dihydro-2H-pyridin-1-yl]-2-oxoethyl}-3,8-diazabicyclo[3.2.1]oct-8-yl)nicotinsäuremethylester;
Verbindung 78: 1-(4-Benzofuran-7-yl-3,6-dihydro-2H-pyridin-1-yl)-2-(4-pyridin-3-yl-[1,4]diazepan-1-yl)ethanon;
Verbindung 79: 6-13-[2-Oxo-2-(4-thieno[3,2-c]pyridin-4-yl-3,6-dihydro-2H-pyridin-1-yl)ethyl]-3,8-diazabicyclo[3.2.1]oct-8-yl}nicotinsäure;
Verbindung 80: 6-(3-{2-[4-(2-Methylbenzo[b]thiophen-5-yl)-3,6-dihydro-2H-pyridin-1-yl]-2-oxoethyl}-3,8-diazabicyclo[3.2.1]oct-8-yl)nicotinsäure;
Verbindung 81: 6-{3-[2-(3-Benzofuran-7-yl-2,5-dihydropyrrol-1-yl)-2-oxoethyl]-3,8-diazabicyclo[3.2.1]oct-8-yl}nicotinsäuremethylester;
Verbindung 82: 6-{3-[2-(3-Benzo[b]thiophen-7-yl-2,5-dihydropyrrol-1-yl)-2-oxoethyl]-3,8-diazabicyclo[3.2.1]oct-8-yl}nicotinsäuremethylester;
Verbindung 83: 4-[2-(3-Benzofuran-7-yl-2,5-dihydropyrrol-1-yl)-2-oxoethyl]-1-(5-trifluormethylpyridin-2-yl)piperazin-2-on;
Verbindung 84: 6-{3-[2-(4-Benzofuran-7-yl-2,3-dihydropyrrol-1-yl)-2-oxoethyl]-3,8-diazabicyclo[3.2.1]oct-8-yl}nicotinsäure;
Verbindung 85: 6-{3-[2-(5-Benzo[b]thiophen-7-yl-3,4-dihydro-2H-pyridin-1-yl)-2-oxoethyl]-3,8-diazabicyclo[3.2.1]oct-8-yl}nicotinsäure;
Verbindung 86: 6-{3-[2-(4-Benzo[b]thiophen-7-yl-2,3-dihydropyrrol-1-yl)-2-oxoethyl]-3,8-diazabicyclo[3.2.1]oct-8-yl}nicotinsäure;
Verbindung 87: 6-(3-{2-[4-(2,3-Dihydrobenzo[1,4]dioxin-6-yl)-3,6-dihydro-2H-pyridin-1-yl]-2-oxoethyl}-3,8-diazabicyclo[3.2.1]oct-8-yl)nicotinsäuremethylester;
Verbindung 88: 2-{(3S,5R)-4-[2-(4-Benzo[b]thiophen-7-yl-3,6-dihydro-2H-pyridin-1-yl)-2-oxoethyl]-3,5-dimethylpiperazin-1-yl}pyrimidin-5-carbonsäure;
Verbindung 89: 3-(6-{3-[2-(4-Benzofuran-7-yl-3,6-dihydro-2H-pyridin-1-yl)-2-oxoethyl]-3,8-diazabicyclo[3.2.1]oct-8-yl)}pyridin-3-yl)-4H-[1,2,4]oxadiazol-5-on;
Verbindung 90: 3-(6-{3-[2-(4-Benzo[b]thiophen-7-yl-3,6-dihydro-2H-pyridin-1-yl)-2-oxoethyl]-3,8-diazabicyclo[3.2.1]oct-8-yl}pyridin-3-yl)-4H-[1,2,4]oxadiazol-5-on;
Verbindung 91: 6-(3-{2-[4-(2-Methylbenzo[b]thiophen-5-yl)-3,6-dihydro-2H-pyridin-1-yl]-2-oxoethyl}-3,8-diazabicyclo[3.2.1]oct-8-yl)nicotinonitril;
Verbindung 92: 1-(4-Benzofuran-7-yl-3,6-dihydro-2H-pyridin-1-yl)-2-[(2S,6R)-4-(3-chlor-5-trifluormethylpyridin-2-yl)-2,6-dimethylpiperazin-1-yl]ethanon;
Verbindung 93: 1-(4-Benzofuran-7-yl-3,6-dihydro-2H-pyridin-1-yl)-2-(8-pyridin-3-yl-3,8-diazabicyclo[3.2.1]oct-3-yl)ethanon;
Verbindung 94: 6-{3-[2-(4-Benzofuran-7-yl-3,6-dihydro-2H-pyridin-1-yl)-2-oxoethyl]-3,8-diazabicyclo[3.2.1]oct-8-yl}nicotinonitril;
Verbindung 95: 1-(4-Benzofuran-7-yl-3,6-dihydro-2H-pyridin-1-yl)-2-[5-(5-trifluormethylpyridin-2-yl)-2,5-diazabicyclo[2.2.1]hept-2-yl]ethanon;
Verbindung 96: 6-(3-{2-[4-(2-Methylbenzo[b]thiophen-5-yl)-3,6-dihydro-2H-pyridin-1-yl]-2-oxoethyl}-3,8-diazabicyclo[3.2.1]oct-8-yl)nicotinsäurecyclobutylester;
Verbindung 97: 2-[(2S,6R)-2,6-Dimethyl-4-(5-trifluormethylpyridin-2-yl)piperazin-1-yl]-1-[4-(1H-indol-3-yl)-3,6-dihydro-2H-pyridin-1-yl]ethanon;
Verbindung 98: 1-[4-(1H-Indol-3-yl)-3,6-dihydro-2H-pyridin-1-yl]-2-(4-chinolin-2-ylpiperazin-1-yl)ethanon;
Verbindung 99: 1-(4-Benzofuran-7-yl-3,6-dihydro-2H-pyridin-1-yl)-2-[4-(7-chlorchinolin-4-yl)piperazin-1-yl]ethanon;
Verbindung 100 : 2-[(2S,6R)-4-(5-Fluorpyrimidin-2-yl)-2,6-dimethylpiperazin-1-yl]-1-[4-(lH-indol-3-yl)-3,6-dihydro-2H-pyridin-1-yl]ethanon;
Verbindung 101: 1-(4-Benzo[b]thiophen-7-yl-3,6-dihydro-2H-pyridin-1-yl)-2-[(2S,6R)-4-(5-chlorpyridin-2-yl)-2,6-dimethylpiperazin-1-yl]ethanon;
Verbindung 102: 1-(4-Benzo[b]thiophen-7-yl-3,6-dihydro-2H-pyridin-1-yl)-2-((2R,5S)-2,5-dimethyl-2,3,5,6-tetrahydro-[1,2']bipyrazinyl-4-yl)ethanon;
Verbindung 103: 1-(4-Benzo[b]thiophen-7-yl-3,6-dihydro-2H-pyridin-1-yl)-2-[8-(5-fluorpyrimidin-2-yl)-3,8-diazabicyclo[3.2.1]oct-3-yl]ethanon;
Verbindung 104: 1-(4-Benzo[b]thiophen-7-yl-3,6-dihydro-2H-pyridin-1-yl)-2-[8-(6-trifluormethylpyridin-2-yl)-3,8-diazabicyclo[3.2.1]oct-3-yl]ethanon;
Verbindung 105: 1-(4-Benzo[b]thiophen-7-yl-3,6-dihydro-2H-pyridin-1-yl)-2-(4-chinolin-2-ylpiperazin-1-yl)ethanon;
Verbindung 106: 1-(4-Benzo[b]thiophen-7-yl-3,6-dihydro-2H-pyridin-1-yl)-2-[4-(7-chlor-chinolin-4-yl)piperazin-1-yl]ethanon;
Verbindung 107: 1-(4-Benzo[b]thiophen-7-yl-3,6-dihydro-2H-pyridin-1-yl)-2-[4-(5-chlor-pyridin-2-yl)piperazin-1-yl]ethanon;
Verbindung 108: 2-[4-(6-Chlorpyridin-2-yl)piperazin-1-yl]-1-[4-(lH-indol-3-yl)-3,6-dihydro-2H-pyridin-1-yl]ethanon;
Verbindung 109: 1-[4-(1H-Indol-3-yl)-3,6-dihydro-2H-pyridin-1-yl]-2-[4-(5-trifluormethylpyridin-2-yl)piperazin-1-yl]ethanon;
Verbindung 110: 2-((2S,6R)-2,6-Dimethyl-4-chinolin-2-yl-piperazin-1-yl)-1-[4-(1H-indol-3-yl)-3,6-dihydro-2H-pyridin-1-yl]ethanon;
Verbindung 111: 1-(4-Benzo[b]thiophen-7-yl-3,6-dihydro-2H-pyridin-1-yl)-2-(4-pyridin-3-yl[1,4]diazepan-1-yl)ethanon;
Verbindung 112: 1-(4-Benzo[b]thiophen-7-yl-3,6-dihydro-2H-pyridin-1-yl)-2-[4-(5,6-dichlorpyridin-2-yl)piperazin-1-yl]ethanon;
Verbindung 113: 1-(4-Benzo[b]thiophen-7-yl-3,6-dihydro-2H-pyridin-1-yl)-2-[4-(6-brompyridin-2-yl)piperazin-1-yl]ethanon;
Verbindung 114: 1-[4-(2-Methylbenzo[b]thiophen-5-yl)-3,6-dihydro-2H-pyridin-1-yl]-2-(4-chinolin-2-ylpiperazin-1-yl)ethanon;
Verbindung 115: 1-(4-Benzo[b]thiophen-7-yl-3,6-dihydro-2H-pyridin-1-yl)-2-[5-(6-trifluormethylpyridazin-3-yl)-2,5-diazabicyclo[2.2.1]hept-2-yl]ethanon;
Verbindung 116: 1-[4-(2-Methylbenzo[b]thiophen-5-yl)-3,6-dihydro-2H-pyridin-1-yl]-2-[4-(6-trifluormethylpyridin-2-yl)piperazin-1-yl]ethanon;
Verbindung 117: 2-[4-(7-Chlorchinolin-4-yl)piperazin-1-yl]-1-[4-(2-methylbenzo[b]thiophen-5-yl)-3,6-dihydro-2H-pyridin-1-yl]ethanon;
Verbindung 118: 4-[2-Oxo-2-(4-thieno[3,2-c]pyridin-4-yl-3,6-dihydro-2H-pyridin-1-yl)ethyl]-1-(5-trifluormethylpyridin-2-yl)piperazin-2-on;
Verbindung 119: 1-(4-Benzofuran-7-yl-3,6-dihydro-2H-pyridin-1-yl)-2-[(2S,6R)-2,6-dimethyl-4-(5-thiazol-2-ylpyridin-2-yl)piperazin-1-yl]ethanon;
Verbindung 120: 1-(4-Benzofuran-7-yl-3,6-dihydro-2H-pyridin-1-yl)-2-{(2S,6R)-2,6-dimethyl-4-[5-(2-methyl-2H-tetrazol-5-yl)pyridin-2-yl]piperazin-1-yl}ethanon;
Verbindung 121: 2-[(2S,6R)-2,6-Dimethyl-4-(5-trifluormethylpyridin-2-yl)piperazin-1-yl]-1-(4-chinolin-2-yl-3,6-dihydro-2H-pyridin-1-yl)ethanon;
Verbindung 122: 4-[2-Oxo-2-(4-chinolin-2-yl-3,6-dihydro-2H-pyridin-1-yl)ethyl]-1-(5-trifluormethylpyridin-2-yl)piperazin-2-on;
Verbindung 123: 1-(4-Chinolin-2-yl-3,6-dihydro-2H-pyridin-1-yl)-2-[8-(5-trifluormethylpyridin-2-yl)-3,8-diazabicyclo[3.2.1]oct-3-yl]ethanon;
Verbindung 124: 1-(4-Benzofuran-7-yl-3,6-dihydro-2H-pyridin-1-yl)-2-{8-[5-(1-methyl-1H-tetrazol-5-yl)pyridin-2-yl]-3,8-diazabicyclo[3.2.1]oct-3-yl}ethanon;
Verbindung 125: 1-(4-Benzofuran-7-yl-3,6-dihydro-2H-pyridin-1-yl)-2-[(2S,6R)-4-(5-methansulfonyl-pyridin-2-yl)-2,6-dimethylpiperazin-1-yl]ethanon;
in Basen- oder Säureadditionssalzform.

7. Verfahren zur Herstellung einer Verbindung der Formel (I) nach einem der Ansprüche 1 bis 6, **dadurch gekennzeichnet, dass** man eine Verbindung der Formel (II): 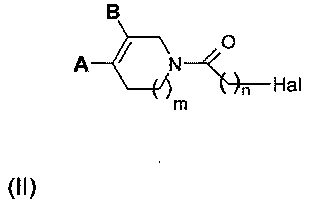 worin A, B, m und n gemäß einem der Ansprüche 1 bis 6 definiert sind und Hal für ein Halogenatom steht,
und eine Verbindung der allgemeinen Formel (III):
H-W-R2 (III)
worin W und R2 gemäß einem der Ansprüche 1 bis 6 definiert sind, umsetzt.

8. Medikament, **dadurch gekennzeichnet, dass** es eine Verbindung der Formel (I) nach einem der Ansprüche 1 bis 6 oder ein Additionssalz dieser Verbindung mit einer pharmazeutisch unbedenklichen Säure umfasst.

9. Pharmazeutische Zusammensetzung, **dadurch gekennzeichnet, dass** sie eine Verbindung der Formel (I) nach einem der Ansprüche 1 bis 6 oder ein pharmazeutisch unbedenkliches Salz sowie mindestens einen pharmazeutisch unbedenklichen Hilfsstoff umfasst.

10. Verbindung nach einem der Ansprüche 1 bis 6 als Medikament.

11. Verbindung nach einem der Ansprüche 1 bis 6 zur Herstellung eines Medikaments zur Prävention oder Behandlung von degenerativen Erkrankungen des zentralen und peripheren Nervensystems, Altersdemenz, Epilepsie, Morbus Alzheimer, Morbus Parkinson, Huntington-Chorea, Down-Syndrom, Prionerkrankungen, Gedächtnisschwund, Schizophrenie, Depression, manisch-depressiver Psychose, amyotropher Lateralsklerose, multipler Sklerose, Herz-Kreislauf-Leiden, postischämischen Herzschäden, Kardiomyopathien, Myokardinfarkt, Herzinsuffizienz, Herzischämie, Hirninfarkt; peripheren Neuropathien, Schädigungen des Sehnervs und der Retina, Degeneration des Retinapigments, Glaukom, retinaler Ischämie, Makuladegeneration, Rückenmarkstraumen, Schädeltraumen, Atherosklerose, Stenosen, Vernarbungsleiden, Alopecia, Bauchspeicheldrüsenentzündung, Leberfibrose, Krebserkrankungen, Tumoren, Metastasen, Leukämien, Erkrankungen der Atemwege, Lungenentzündung, Allergie, Asthma, chronisch-obstruktiver Bronchopneumopathie, Haut-, Körper-, Eingeweide- und Nervenschmerz, chronischen neuropathischen und entzündlichen Schmerzen, Autoimmunerkrankungen, rheumatoider Polyarthritis, ankylosierender Spondylarthritis, Psoriasis-Rheumatismus, Plaque-Psoriasis, Knochenbrüchen, Knochenerkrankungen oder Osteoporose.

12. Verbindung nach einem der Ansprüche 1 bis 6 als Inhibitor der Dimerisation des p75^{NTR}-Rezeptors.

13. Verwendung einer Verbindung nach einem der Ansprüche 1 bis 6 bei der Herstellung von Medikamenten, die bei der Behandlung und Prävention von degenerativen Erkrankungen des zentralen und peripheren Nervensystems, Altersdemenz, Epilepsie, Morbus Alzheimer, Morbus Parkinson, Huntington-Chorea, Down-Syndrom, Prionerkrankungen, Gedächtnisschwund, Schizophrenie, Depression, manisch-depressiver Psychose, amyotropher Lateralsklerose, multipler Sklerose, Herz-Kreislauf-Leiden, postischämischen Herzschäden, Kardiomyopathien, Myokardinfarkt, Herzinsuffizienz, Herzischämie, Hirninfarkt; peripheren Neuropathien, Schädigungen des Sehnervs und der Retina, Degeneration des Retinapigments, Glaukom, retinaler Ischämie, Makuladegeneration, Rückenmarkstraumen, Schädeltraumen, Atherosklerose, Stenosen, Vernarbungsleiden, Alopecia, Bauchspeicheldrüsenentzündung, Leberfibrose, Krebserkrankungen, Tumoren, Metastasen, Leukämien, Erkrankungen der Atemwege, Lungenentzündung, Allergie, Asthma, chronisch-obstruktiver Bronchopneumopathie, Haut-, Körper-, Eingeweide- und Nervenschmerz, chronischen neuropathischen und entzündlichen Schmerzen, Autoimmunerkrankungen, rheumatoider Polyarthritis, ankylosierender Spondylarthritis, Psoriasis-Rheumatismus, Plaque-Psoriasis, Knochenbrüchen, Knochenerkrankungen oder Osteoporose verwendet werden können.

## Claims

1. Compound corresponding to formula (I): 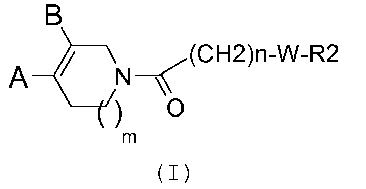 in which:
- n represents 1 or 2;
- m represents 0 or 1;
- A represents a fused heterocyclic group of formula (Y) 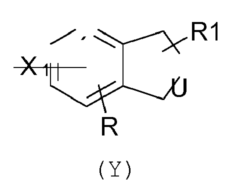
and B represents a hydrogen atom;
or
A represents a hydrogen atom; and
B represents a fused heterocyclic group of formula (Y) 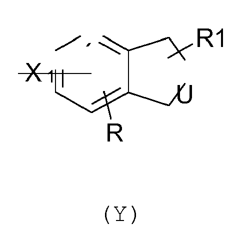 The fused heterocycle of formula Y may be attached to the rest of the molecule via any of the available carbon atoms, and in which:
- U completes:
- either an aromatic or saturated 6-atom nucleus, containing one or two nitrogen atoms, the nucleus possibly being substituted with one or two halogen atoms, one or two (C1-C4)alkyl or (C1-C4)alkoxy groups, or one or two perfluoroalkyl radicals;
- or an aromatic or saturated 5-atom nucleus, containing a nitrogen, oxygen or sulfur atom, the nucleus possibly being substituted with one or two groups (C1-C4)alkyl;
- X and X1 represent CH or N;
- R and R1 located on any of the available positions, independently represent a hydrogen atom, a halogen atom, a group (C1-C4)alkyl, (C1-C4)alkoxy, a perfluoroalkyl or trifluoromethoxy radical, a cyano or a group COOH, COOalkyl, CONR3R4 or NHCOR3;
- -W- is a nitrogenous heterocycle chosen from:
-W- = 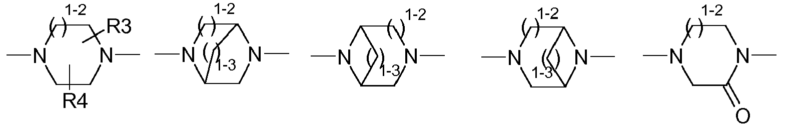
- 1-2 represents 1 or 2;
- 1-3 represents 1, 2 or 3;
- R2 represents a group of formula: 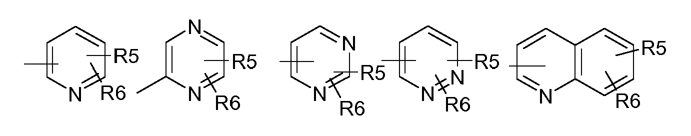
- in which R5 and R6, located on any of the available positions, independently represent a hydrogen atom, a halogen atom, a group (C1-C4)alkyl or (C1-C4)alkoxy, a trifluoromethyl or trifluoromethoxy radical, a cyano or a group COOH, COOalkyl, COOcycloalkyl, SOalkyl, SO₂alkyl, CONR3R4, NR3R4 or NHCOR3;
or one of the groups R5 and R6 may also represent a heterocycle chosen from: 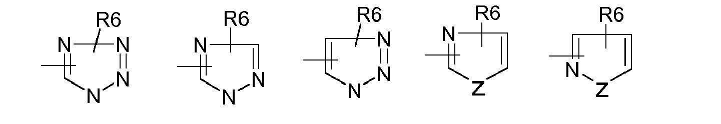 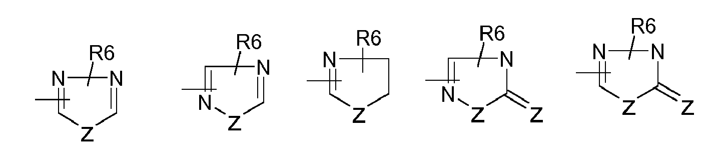
- Z represents an oxygen or sulfur atom;
- R3 and R4 represent a hydrogen or a group (C1-C6) alkyl;
in the form of the base or of an acid-addition salt.

2. Compound according to Claim 1, such that
- A represents a fused heterocyclic group of formula (Y) 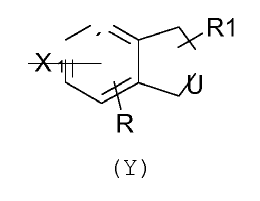 and B represents a hydrogen atom;
or
A represents a hydrogen atom;
and B represents a fused heterocyclic group of formula (Y) 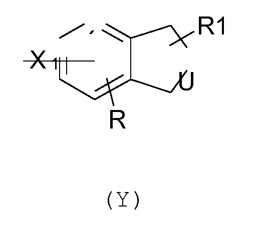 the fused heterocycle of formula Y possibly being attached to the rest of the molecule via any of the available carbon atoms of the benzene nucleus;
in the form of the base or of an acid-addition salt.

3. Compound according to either of Claims 1 and 2, such that R and R1 located on any of the available positions, independently represent a hydrogen atom, a halogen atom or a group (C₁-C₄)alkyl or COOalkyl; in the form of the base or of an acid-addition salt.

4. Compound according to one of Claims 1 to 3, such that:
- -W- is a nitrogenous heterocycle chosen from:
-W- = 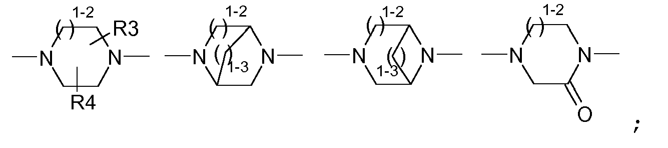
- R3 and R4 represent a hydrogen atom or a methyl group;
in the form of the base or of an acid-addition salt.

5. Compound according to one of Claims 1 to 4, such that:
- R2 represents a group of formula: 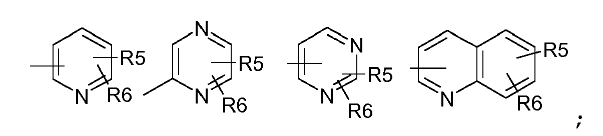
- R5 and R6, located on any of the available positions, independently represent a hydrogen atom, a trifluoromethyl radical or a group COOH, COOalkyl or COOcycloalkyl; or
one of the groups R5 and R6 may also represent a heterocycle chosen from: 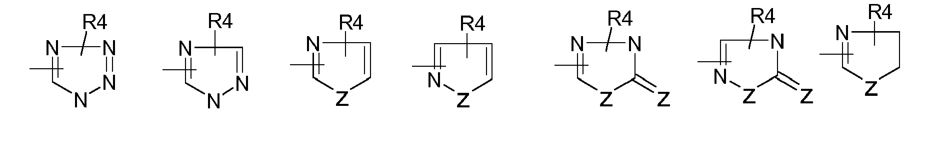
- Z represents an oxygen or sulfur atom; and/or
- R3 and R4 represent a hydrogen or a methyl group;
in the form of the base or of an acid-addition salt.

6. Compound according to any one of Claims 1 to 5, chosen from:
- Compound 1: 1-(4-benzo[b]thiophen-7-yl-3,6-dihydro-2H-pyridin-1-yl)-2-(3,5-dimethyl-2,3,5,6-tetrahydro[1,2']bipyrazinyl-4-yl)ethanone;
- Compound 2: 1-(4-benzo[b]thiophen-7-yl-3,6-dihydro-2H-pyridin-1-yl)-2-(8-pyrimidin-2-yl-3,8-diazabicyclo[3.2.1]oct-3-yl)ethanone;
- Compound 3: 1-(4-benzo[b]thiophen-5-yl-3,6-dihydro-2H-pyridin-1-yl)-2-(8-pyrimidin-2-yl-3,8-diazabicyclo[3.2.1]oct-3-yl)ethanone;
- Compound 4: 1-(4-benzo[b]thiophen-7-yl-3,6-dihydro-2H-pyridin-1-yl)-2-[(2S,6R)-2,6-dimethyl-4-(5-trifluoromethylpyridin-2-yl)piperazin-1-yl]ethanone;
- Compound 5: 1-(4-benzo[b]thiophen-5-yl-3,6-dihydro-2H-pyridin-1-yl)-2-((2S,5R)-2,5-dimethyl-2,3,5,6-tetrahydro[1,2']bipyrazinyl-4-yl)ethanone;
- Compound 6: 1-(4-benzo[b]thiophen-7-yl-3,6-dihydro-2H-pyridin-1-yl)-2-((2S,5R)-2,5-dimethyl-2,3,5,6-tetrahydro[1,2']bipyrazinyl-4-yl)ethanone;
- Compound 7: 1-(4-benzo[b]thiophen-5-yl-3,6-dihydro-2H-pyridin-1-yl)-2-[(2S,6R)-2,6-dimethyl-4-(5-trifluoromethylpyridin-2-yl)piperazin-1-yl]ethanone;
- Compound 8: 1-(4-benzo[b]thiophen-7-yl-3,6-dihydro-2H-pyridin-1-yl)-2-[8-(5-trifluoromethylpyridin-2-yl)-3,8-diazabicyclo[3.2.1]oct-3-yl]ethanone;
- Compound 9: 1-(4-benzo[b]thiophen-7-yl-3,6-dihydro-2H-pyridin-1-yl)-2-[(2R,5S)-2,5-dimethyl-4-(5-trifluoromethylpyridin-2-yl)piperazin-1-yl]ethanone;
- Compound 10: 2-[(2S,6R)-2,6-dimethyl-4-(5-trifluoromethylpyridin-2-yl)piperazin-1-yl]-1-[4-(2-methylbenzo[b]thiophen-7-yl)-3,6-dihydro-2H-pyridin-1-yl]ethanone;
- Compound 11: 2-[(2S,6R)-2,6-dimethyl-4-(5-trifluoromethylpyridin-2-yl)piperazin-1-yl]-1-[4-(2-propylbenzo[b]thiophen-7-yl)-3,6-dihydro-2H-pyridin-1-yl]ethanone;
- Compound 12: 1-(4-benzo[b]thiophen-7-yl-3,6-dihydro-2H-pyridin-1-yl)-2-[5-(5-trifluoromethylpyridin-2-yl)-2,5-diazabicyclo[2.2.1]hept-2-yl]ethanone;
- Compound 13: 1-(4-benzo[b]thiophen-6-yl-3,6-dihydro-2H-pyridin-1-yl)-2-[(2R,5S)-2,5-dimethyl-4-(5-trifluoromethylpyridin-2-yl)piperazin-1-yl]ethanone;
- Compound 14: 1-(4-benzofuran-7-yl-3,6-dihydro-2H-pyridin-1-yl)-2-[(2R,5S)-2,5-dimethyl-4-(5-trifluoromethylpyridin-2-yl)piperazin-1-yl]ethanone;
- Compound 15: 1-[4-(2-methylbenzo[b]thiophen-5-yl)-3,6-dihydro-2H-pyridin-1-yl]-2-(8-pyrimidin-2-yl-3,8-diazabicyclo[3.2.1]oct-3-yl)ethanone;
- Compound 16: 1-[4-(2-methylbenzo[b]thiophen-5-yl)-3,6-dihydro-2H-pyridin-1-yl]-2-[4-(5-trifluoromethylpyridin-2-yl)piperazin-1-yl]ethanone;
- Compound 17: 4-{2-[4-(2-methylbenzo[b]thiophen-5-yl)-3,6-dihydro-2H-pyridin-1-yl]-2-oxoethyl}-1-(5-trifluoromethylpyridin-2-yl)piperazin-2-one;
- Compound 18: 2-[(2S,6R)-2,6-dimethyl-4-(5-trifluoromethylpyridin-2-yl)piperazin-1-yl]-1-[4-(2-methylbenzo[b]thiophen-5-yl)-3,6-dihydro-2H-pyridin-1-yl]ethanone;
- Compound 19: 1-(4-benzo[b]thiophen-7-yl-3,6-dihydro-2H-pyridin-1-yl)-2-((2S,6R)-2,6-dimethyl-4-quinolin-2-ylpiperazin-1-yl)ethanone;
- Compound 20: 1-(4-quinolin-8-yl-3,6-dihydro-2H-pyridin-1-yl)-2-[4-(5-trifluoromethylpyridin-2-yl)piperazin-1-yl]ethanone;
- Compound 21: 1-[4-(2,3-dihydrobenzo[1,4]dioxin-6-yl)-3,6-dihydro-2H-pyridin-1-yl]-2-[4-(5-trifluoromethylpyridin-2-yl)piperazin-1-yl]ethanone;
- Compound 22: 2-[8-(5-fluoropyrimidin-2-yl)-3,8-diazabicyclo[3.2.1]oct-3-yl]-1-[4-(2-methylbenzo[b]thiophen-5-yl)-3,6-dihydro-2H-pyridin-1-yl]ethanone;
- Compound 23: 1-(4-benzofuran-7-yl-3,6-dihydro-2H-pyridin-1-yl)-2-[8-(5-fluoropyrimidin-2-yl)-3,8-diazabicyclo[3.2.1]oct-3-yl]ethanone;
- Compound 24: 1-[4-(2,3-dihydrobenzo[1,4]dioxin-6-yl)-3,6-dihydro-2H-pyridin-1-yl]-2-[8-(5-fluoropyrimidin-2-yl)-3,8-diazabicyclo[3.2.1]oct-3-yl]ethanone;
- Compound 25: 2-[8-(5-fluoropyrimidin-2-yl)-3,8-diazabicyclo[3.2.1]oct-3-yl]-1-(4-thieno[3,2-c]pyridin-4-yl-3,6-dihydro-2H-pyridin-1-yl)ethanone;
- Compound 26: 1-(4-benzofuran-3-yl-3,6-dihydro-2H-pyridin-1-yl)-2-[8-(5-fluoropyrimidin-2-yl)-3,8-diazabicyclo[3.2.1]oct-3-yl]ethanone;
- Compound 27: 2-[8-(5-fluoropyrimidin-2-yl)-3,8-diazabicyclo[3.2.1]oct-3-yl]-1-[4-(1H-indol-3-yl)-3,6-dihydro-2H-pyridin-1-yl]ethanone;
- Compound 28: 1-[4-(6-fluoro-1H-indol-3-yl)-3,6-dihydro-2H-pyridin-1-yl]-2-[8-(5-fluoropyrimidin-2-yl)-3,8-diazabicyclo[3.2.1]oct-3-yl]ethanone;
- Compound 29: 1-(4-benzo[b]thiophen-3-yl-3,6-dihydro-2H-pyridin-1-yl)-2-[8-(5-fluoropyrimidin-2-yl)-3,8-diazabicyclo[3.2.1]oct-3-yl]ethanone;
- Compound 30: 4-{2-[4-(1H-indol-3-yl)-3,6-dihydro-2H-pyridin-1-yl]-2-oxoethyl}-1-(5-trifluoromethylpyridin-2-yl)piperazin-2-one;
- Compound 31: 4-[2-(4-benzo[b]thiophen-7-yl-3,6-dihydro-2H-pyridin-1-yl)-2-oxoethyl]-1-(5-trifluoromethylpyridin-2-yl)piperazin-2-one;
- Compound 32: 4-[2-(4-benzofuran-7-yl-3,6-dihydro-2H-pyridin-1-yl)-2-oxoethyl]-1-(5-trifluoromethylpyridin-2-yl)piperazin-2-one;
- Compound 33: 1-(4-benzofuran-7-yl-3,6-dihydro-2H-pyridin-1-yl)-2-[4-(5-trifluoromethylpyridin-2-yl)piperazin-1-yl]ethanone;
- Compound 34: 1-(4-benzofuran-7-yl-3,6-dihydro-2H-pyridin-1-yl)-2-[(2S,6R)-2,6-dimethyl-4-(5-trifluoromethylpyridin-2-yl)piperazin-1-yl]ethanone;
- Compound 35: 1-(4-benzofuran-7-yl-3,6-dihydro-2H-pyridin-1-yl)-2-((2S,6R)-2,6-dimethyl-4-pyrimidin-5-ylpiperazin-1-yl)ethanone;
- Compound 36: 1-(4-benzofuran-7-yl-3,6-dihydro-2H-pyridin-1-yl)-2-[(2S,6R)-4-(5-fluoropyrimidin-2-yl)-2,6-dimethylpiperazin-1-yl]ethanone;
- Compound 37: 1-(4-benzofuran-7-yl-3,6-dihydro-2H-pyridin-1-yl)-2-[(2S,6R)-2,6-dimethyl-4-(6-trifluoromethylpyridin-3-yl)piperazin-1-yl]ethanone;
- Compound 38: 2-[(2S,6R)-4-(5-fluoropyrimidin-2-yl)-2,6-dimethylpiperazin-1-yl]-1-[4-(2-methylbenzo[b]thiophen-5-yl)-3,6-dihydro-2H-pyridin-1-yl]ethanone;
- Compound 39: 2-[(2S,6R)-2,6-dimethyl-4-(6-trifluoromethylpyridin-3-yl)piperazin-1-yl]-1-[4-(2-methylbenzo[b]thiophen-5-yl)-3,6-dihydro-2H-pyridin-1-yl]ethanone;
- Compound 40: 2-((2S,6R)-2,6-dimethyl-4-pyrimidin-5-ylpiperazin-1-yl)-1-[4-(2-methylbenzo[b]thiophen-5-yl)-3,6-dihydro-2H-pyridin-1-yl]ethanone;
- Compound 41: 1-(4-benzo[b]thiophen-5-yl-3,6-dihydro-2H-pyridin-1-yl)-2-((2S,6R)-2,6-dimethyl-4-pyrimidin-5-ylpiperazin-1-yl)ethanone;
- Compound 42: 1-(4-benzo[b]thiophen-5-yl-3,6-dihydro-2H-pyridin-1-yl)-2-[(2S,6R)-4-(5-fluoropyrimidin-2-yl)-2,6-dimethylpiperazin-1-yl)ethanone;
- Compound 43: 4-[2-(4-benzo[b]thiophen-5-yl-3,6-dihydro-2H-pyridin-1-yl)-2-oxoethyl]-1-(5-trifluoromethylpyridin-2-yl)piperazin-2-one;
- Compound 44: 1-[4-(2,3-dihydrobenzo[1,4]dioxin-6-yl)-3,6-dihydro-2H-pyridin-1-yl]-2-[(2S,6R)-4-(5-fluoropyrimidin-2-yl)-2,6-dimethylpiperazin-1-yl]ethanone;
- Compound 45: 1-[4-(2,3-dihydrobenzo[1,4]dioxin-6-yl)-3,6-dihydro-2H-pyridin-1-yl]-2-[(2S,6R)-2,6-dimethyl-4-(6-trifluoromethylpyridin-3-yl)piperazin-1-yl]ethanone;
- Compound 46: 1-(4-benzo[b]thiophen-5-yl-3,6-dihydro-2H-pyridin-1-yl)-2-[4-(5-trifluoromethylpyridin-2-yl)piperazin-1-yl]ethanone;
- Compound 47: 1-(4-benzo[b]thiophen-5-yl-3,6-dihydro-2H-pyridin-1-yl)-2-[(2S,6R)-2,6-dimethyl-4-(6-trifluoromethylpyridin-3-yl)piperazin-1-yl]ethanone;
- Compound 48: 5-{(3S,5R)-4-[2-(4-benzo[b]thiophen-4-yl-3,6-dihydro-2H-pyridin-1-yl)-2-oxoethyl]-3,5-dimethylpiperazin-1-yl}pyridine-2-carboxylic acid;
- Compound 49: 4-{2-[4-(2,3-dihydrobenzo[1,4]dioxin-6-yl)-3,6-dihydro-2H-pyridin-1-yl]-2-oxoethyl}-1-(6-trifluoromethylpyridin-3-yl)piperazin-2-one;
- Compound 50: 1-(4-benzo[b]thiophen-7-yl-3,6-dihydro-2H-pyridin-1-yl)-2-[4-(6-trifluoromethylpyridin-3-yl)piperazin-1-yllethanone;
- Compound 51: Methyl 5-{(3S,5R)-4-[2-(4-benzofuran-7-yl-3,6-dihydro-2H-pyridin-1-yl)-2-oxoethyl]-3,5-dimethylpiperazin-1-yl}pyridine-2-carboxylate;
- Compound 52: Methyl 6-{(3S,5R)-4-[2-(4-benzo[b]thiophen-5-yl-3,6-dihydro-2H-pyridin-1-yl)-2-oxoethyl]-3,5-dimethylpiperazin-1-yl}nicotinate;
- Compound 53: 1-(4-benzo[b]thiophen-7-yl-3,6-dihydro-2H-pyridin-1-yl)-2-[(2S,6R)-4-(5-fluoropyrimidin-2-yl)-2,6-dimethylpiperazin-1-yl]ethanone;
- Compound 54: 1-(4-benzo[b]thiophen-7-yl-3,6-dihydro-2H-pyridin-1-yl)-2-[(2S,6R)-2,6-dimethyl-4-(6-trifluoromethylpyridin-3-yl)piperazin-1-yl]ethanone;
- Compound 55: 1-(4-benzo[b]thiophen-7-yl-3,6-dihydro-2H-pyridin-1-yl)-2-((2S,6R)-2,6-dimethyl-4-pyrimidin-5-ylpiperazin-1-yl)ethanone;
- Compound 56: 6-{(3S,5R)-4-[2-(4-benzo[b]thiophen-5-yl-3,6-dihydro-2H-pyridin-1-yl)-2-oxoethyl]-3,5-dimethylpiperazin-1-yllnicotinic acid;
- Compound 57: 6-{(3S,5R)-4-[2-(4-benzofuran-7-yl-3,6-dihydro-2H-pyridin-1-yl)-2-oxoethyl]-3,5-dimethylpiperazin-1-yl}nicotinic acid;
- Compound 58: Methyl 6-{(3S,5R)-4-[2-(4-benzo[b]thiophen-7-yl-3,6-dihydro-2H-pyridin-1-yl)-2-oxoethyl]-3,5-dimethylpiperazin-1-yl}nicotinate;
- Compound 59: 6-{(3S,5R)-4-[2-(4-benzo[b]thiophen-7-yl-3,6-dihydro-2H-pyridin-1-yl)-2-oxoethyl]-3,5-dimethylpiperazin-1-yl}nicotinic acid;
- Compound 60: 4-[2-(4-benzofuran-5-yl-3,6-dihydro-2H-pyridin-1-yl)-2-oxoethyl]-1-(5-trifluoromethylpyridin-2-yl)piperazin-2-one;
- Compound 61: Methyl 6-((3S,5R)-3,5-dimethyl-4-{2-[4-(2-methylbenzo[b]thiophen-5-yl)-3,6-dihydro-2H-pyridin-1-yl]-2-oxoethyl}piperazin-1-yl)nicotinate;
- Compound 62: 6-((3S,5R)-3,5-dimethyl-4-{2-[4-(2-methylbenzo[b]thiophen-5-yl-)-3,6-dihydro-2H-pyridin-1-yl]-2-oxoethyl}piperazin-1-yl)nicotinic acid;
- Compound 63: 1-(5-benzofuran-7-yl-3,6-dihydro-2H-pyridin-1-yl)-2-[(2S,6R)-2,6-dimethyl-4-(5-trifluoromethylpyridin-2-yl)piperazin-1-yl]ethanone;
- Compound 64: Ethyl 6-{(3S,5R)-4-[2-(4-benzofuran-7-yl-3,6-dihydro-2H-pyridin-1-yl)-2-oxoethyl]-3,5-dimethylpiperazin-1-yl}nicotinate;
- Compound 65: Methyl 6-{3-[2-(4-benzofuran-7-yl-3,6-dihydro-2H-pyridin-1-yl)-2-oxoethyl]-3,8-diazabicyclo[3.2.1]oct-8-yl}nicotinate;
- Compound 66: Methyl 7-(1-{2-[(2S,6R)-2,6-dimethyl-4-(5-trifluoromethylpyridin-2-yl)piperazin-1-yl]acetyl}-1,2,3,6-tetrahydropyridin-4-yl)benzo[b]thiophene-2-carboxylate;
- Compound 67: Methyl 5-(1-{2-[(2S,6R)-2,6-dimethyl-4-(5-trifluoromethylpyridin-2-yl)piperazin-1-yl]acetyl}-1,2,3,6-tetrahydropyridin-4-yl)benzo[b]thiophene-2-carboxylate;
- Compound 68: 2-[(2S,6R)-2,6-dimethyl-4-(5-trifluoromethylpyridin-2-yl)piperazin-1-yl]-1-[4-(2-propylbenzo[b]thiophen-5-yl)-3,6-dihydro-2H-pyridin-1-yl]ethanone;
- Compound 69: Methyl 6-{3-[2-(4-benzo[b]thiophen-7-yl-3,6-dihydro-2H-pyridin-1-yl)-2-oxoethyl]-3,8-diazabicyclo[3.2.1]oct-8-yl}nicotinate;
- Compound 70: Methyl 6-{3-[2-(4-benzo[b]thiophen-5-yl-3,6-dihydro-2H-pyridin-1-yl)-2-oxoethyl]-3,8-diazabicyclo[3.2.1]oct-8-yl}nicotinate;
- Compound 71: 6-{3-[2-(4-benzo[b]thiophen-7-yl-3,6-dihydro-2H-pyridin-1-yl)-2-oxoethyl]-3,8-diazabicyclo[3.2.1]oct-8-yl}nicotinic acid;
- Compound 72: 2-[(2S,6R)-2,6-dimethyl-4-(5-trifluoromethyl-pyridin-2-yl)piperazin-1-yl]-1-[4-(7-fluorobenzofuran-5-yl)-3,6-dihydro-2H-pyridin-1-yl]ethanone;
- Compound 73: 1-[4-(2,3-dimethylbenzofuran-6-yl)-3,6-dihydro-2H-pyridin-1-yl]-2-[(2S,6R)-2,6-dimethyl-4-(5-trifluoromethylpyridin-2-yl)piperazin-1-yl]ethanone;
- Compound 74: 2-[(2S,6R)-2,6-dimethyl-4-(5-trifluoromethylpyridin-2-yl)piperazin-1-yl]-1-(4-quinolein-2-yl-3,6-dihydro-2H-pyridin-1-yl)ethanone;
- Compound 75: 1-(4-benzofuran-5-yl-3,6-dihydro-2H-pyridin-1-yl)-2-(2,3,5,6-tetrahydro[1,2']bipyrazinyl-4-yl)ethanone;
- Compound 76: Methyl 6-{3-[2-oxo-2-(4-thieno[3,2-c]pyridin-4-yl-3,6-dihydro-2H-pyridin-1-yl)ethyl]-3,8-diazabicyclo[3.2.1]oct-8-yl}nicotinate;
- Compound 77: Methyl 6-(3-{2-[4-(2-methylbenzo[b]thiophen-5-yl)-3,6-dihydro-2H-pyridin-1-yl]-2-oxoethyl}-3,8-diazabicyclo[3.2.1]oct-8-yl)nicotinate;
- Compound 78: 1-(4-benzofuran-7-yl-3,6-dihydro-2H-pyridin-1-yl)-2-(4-pyridin-3-yl-[1,4]diazepan-1-yl)ethanone;
- Compound 79: 6-{3-[2-oxo-2-(4-thieno[3,2-c]pyridin-4-yl-3,6-dihydro-2H-pyridin-1-yl)ethyl]-3,8-diazabicyclo[3.2.1]oct-8-yl}nicotinic acid;
- Compound 80: 6-(3-{2-[4-(2-methylbenzo[b]thiophen-5-yl)-3,6-dihydro-2H-pyridin-1-yl]-2-oxoethyl}-3,8-diazabicyclo[3.2.1]oct-8-yl)nicotinic acid;
- Compound 81: Methyl 6-{3-[2-(3-benzofuran-7-yl-2,5-dihydropyrrol-1-yl)-2-oxoethyl]-3,8-diazabicyclo[3.2.1]oct-8-yl}nicotinate;
- Compound 82: Methyl 6-{3-[2-(3-benzo[b]thiophen-7-yl-2,5-dihydropyrrol-1-yl)-2-oxoethyl]-3,8-diazabicyclo[3.2.1]oct-8-yl}nicotinate;
- Compound 83: 4-[2-(3-benzofuran-7-yl-2,5-dihydropyrrol-1-yl)-2-oxoethyl]-1-(5-trifluoromethylpyridin-2-yl)piperazin-2-one;
- Compound 84: 6-{3-[2-(4-benzofuran-7-yl-2,3-dihydropyrrol-1-yl)-2-oxoethyl]-3,8-diazabicyclo[3.2.1]oct-8-yl}nicotinic acid;
- Compound 85: 6-{3-[2-(5-benzo[b]thiophen-7-yl-3,4-dihydro-2H-pyridin-1-yl)-2-oxoethyl]-3,8-diazabicyclo[3.2.1]oct-8-yl}nicotinic acid;
- Compound 86: 6-{3-[2-(4-benzo[b]thiophen-7-yl-2,3-dihydropyrrol-1-yl)-2-oxoethyl]-3,8-diazabicyclo[3.2.1]oct-8-yl}nicotinic acid;
- Compound 87: Methyl 6-(3-{2-[4-(2,3-dihydrobenzo[1,4]dioxin-6-yl)-3,6-dihydro-2H-pyridin-1-yl]-2-oxoethyl}-3,8-diazabicyclo[3.2.1]oct-8-yl)nicotinate;
- Compound 88: 2-{(3S,5R)-4-[2-(4-benzo[b]thiophen-7-yl-3,6-dihydro-2H-pyridin-1-yl)-2-oxoethyl]-3,5-dimethylpiperazin-1-yl}pyrimidine-5-carboxylic acid;
- Compound 89: 3-(6-{3-[2-(4-benzofuran-7-yl-3,6-dihydro-2H-pyridin-1-yl)-2-oxoethyl]-3,8-diazabicyclo[3.2.1]oct-8-yl)}pyridin-3-yl)-4H-[1,2,4]oxadiazol-5-one;
- Compound 90: 3-(6-{3-[2-(4-benzo[b]thiophen-7-yl-3,6-dihydro-2H-pyridin-1-yl)-2-oxoethyl]-3,8-diazabicyclo[3.2.1]oct-8-yl}pyridin-3-yl)-4H-[1,2,4]oxadiazol-5-one;
- Compound 91: 6-(3-{2-[4-(2-methylbenzo[b]thiophen-5-yl)-3,6-dihydro-2H-pyridin-1-yl]-2-oxoethyl}-3,8-diazabicyclo[3.2.1]oct-8-yl)nicotinonitrile;
- Compound 92: 1-(4-benzofuran-7-yl-3,6-dihydro-2H-pyridin-1-yl)-2-[(2S,6R)-4-(3-chloro-5-trifluoromethylpyridin-2-yl)-2,6-dimethylpiperazin-1-yl]ethanone;
- Compound 93: 1-(4-benzofuran-7-yl-3,6-dihydro-2H-pyridin-1-yl)-2-(8-pyridin-3-yl-3,8-diazabicyclo[3.2.1]oct-3-yl)ethanone;
- Compound 94: 6-{3-[2-(4-benzofuran-7-yl-3,6-dihydro-2H-pyridin-1-yl)-2-oxoethyl]-3,8-diazabicyclo[3.2.1]oct-8-yl}nicotinonitrile;
- Compound 95: 1-(4-benzofuran-7-yl-3,6-dihydro-2H-pyridin-1-yl)-2-[5-(5-trifluoromethylpyridin-2-yl)-2,5-diazabicyclo[2.2.1]hept-2-yl]ethanone;
- Compound 96: cyclobutyl 6-(3-{2-[4-(2-methylbenzo[b]thiophen-5-yl)-3,6-dihydro-2H-pyridin-1-yl]-2-oxoethyl}-3,8-diazabicyclo[3.2.1]oct-8-yl)nicotinate;
- Compound 97: 2-[(2S,6R)-2,6-dimethyl-4-(5-trifluoromethylpyridin-2-yl)piperazin-1-yl]-1-[4-(1H-indol-3-yl)-3,6-dihydro-2H-pyridin-1-yl]ethanone;
- Compound 98: 1-[4-(1H-indol-3-yl)-3,6-dihydro-2H-pyridin-1-yl]-2-(4-quinolin-2-ylpiperazin-1-yl)ethanone;
- Compound 99: 1-(4-benzofuran-7-yl-3,6-dihydro-2H-pyridin-1-yl)-2-[4-(7-chloroquinolin-4-yl)piperazin-1-yl]ethanone;
- Compound 100: 2-[(2S,6R)-4-(5-fluoropyrimidin-2-yl)-2,6-dimethylpiperazin-1-yl]-1-[4-(1H-indol-3-yl)-3,6-dihydro-2H-pyridin-1-yl]ethanone;
- Compound 101: 1-(4-benzo[b]thiophen-7-yl-3,6-dihydro-2H-pyridin-1-yl)-2-[(2S,6R)-4-(5-chloropyridin-2-yl)-2,6-dimethylpiperazin-1-yl]ethanone;
- Compound 102: 1-(4-benzo[b]thiophen-7-yl-3,6-dihydro-2H-pyridin-1-yl)-2-((2R,5S)-2,5-dimethyl-2,3,5,6-tetrahydro-[1,2']bipyrazinyl-4-yl)ethanone;
- Compound 103: 1-(4-benzo[b]thiophen-7-yl-3,6-dihydro-2H-pyridin-1-yl)-2-[8-(5-fluoropyrimidin-2-yl)-3,8-diazabicyclo[3.2.1]oct-3-yl]ethanone;
- Compound 104: 1-(4-benzo[b]thiophen-7-yl-3,6-dihydro-2H-pyridin-1-yl)-2-[8-(6-trifluoromethylpyridin-2-yl)-3,8-diazabicyclo[3.2.1]oct-3-yl]ethanone;
- Compound 105: 1-(4-benzo[b]thiophen-7-yl-3,6-dihydro-2H-pyridin-1-yl)-2-(4-quinolin-2-ylpiperazin-1-yl)ethanone;
- Compound 106: 1-(4-benzo[b]thiophen-7-yl-3,6-dihydro-2H-pyridin-1-yl)-2-[4-(7-chloro-quinolin-4-yl)piperazin-1-yl]ethanone;
- Compound 107: 1-(4-benzo[b]thiophen-7-yl-3,6-dihydro-2H-pyridin-1-yl)-2-[4-(5-chloro-pyridin-2-yl)piperazin-1-yl]ethanone;
- Compound 108: 2-[4-(6-chloropyridin-2-yl)piperazin-1-yl]-1-[4-(1H-indol-3-yl)-3,6-dihydro-2H-pyridin-1-yl]ethanone;
- Compound 109: 1-[4-(1H-indol-3-yl)-3,6-dihydro-2H-pyridin-1-yl]-2-[4-(5-trifluoromethylpyridin-2-yl)piperazin-1-yl]ethanone;
- Compound 110: 2-((2S,6R)-2,6-dimethyl-4-quinolin-2-yl-piperazin-1-yl)-1-[4-(1H-indol-3-yl)-3,6-dihydro-2H-pyridin-1-yl]ethanone;
- Compound 111: 1-(4-benzo[b]thiophen-7-yl-3,6-dihydro-2H-pyridin-1-yl)-2-(4-pyridin-3-yl[1,4]diazepan-1-yl)ethanone;
- Compound 112: 1-(4-benzo[b]thiophen-7-yl-3,6-dihydro-2H-pyridin-1-yl)-2-[4-(5,6-dichloropyridin-2-yl)piperazin-1-yl]ethanone;
- Compound 113: 1-(4-benzo[b]thiophen-7-yl-3,6-dihydro-2H-pyridin-1-yl)-2-[4-(6-bromopyridin-2-yl)piperazin-1-yl]ethanone;
- Compound 114: 1-[4-(2-methylbenzo[b]thiophen-5-yl)-3,6-dihydro-2H-pyridin-1-yl]-2-(4-quinolin-2-ylpiperazin-1-yl)ethanone;
- Compound 115: 1-(4-benzo[b]thiophen-7-yl-3,6-dihydro-2H-pyridin-1-yl)-2-[5-(6-trifluoromethylpyridazin-3-yl)-2,5-diazabicyclo[2.2.1]hept-2-yl]ethanone;
- Compound 116: 1-[4-(2-methylbenzo[b]thiophen-5-yl)-3,6-dihydro-2H-pyridin-1-yl]-2-[4-(6-trifluoromethylpyridin-2-yl)piperazin-1-yl]ethanone;
- Compound 117: 2-[4-(7-chloroquinolin-4-yl)piperazin-1-yl]-1-[4-(2-methylbenzo[b]thiophen-5-yl)-3,6-dihydro-2H-pyridin-1-yl]ethanone;
- Compound 118: 4-[2-oxo-2-(4-thieno[3,2-c]pyridin-4-yl-3,6-dihydro-2H-pyridin-1-yl)ethyl]-1-(5-trifluoromethylpyridin-2-yl)piperazin-2-one;
- Compound 119: 1-(4-benzofuran-7-yl-3,6-dihydro-2H-pyridin-1-yl)-2-[(2S,6R)-2,6-dimethyl-4-(5-thiazol-2-ylpyridin-2-yl)piperazin-1-yl]ethanone;
- Compound 120: 1-(4-benzofuran-7-yl-3,6-dihydro-2H-pyridin-1-yl)-2-{(2S,6R)-2,6-dimethyl-4-[5-(2-methyl-2H-tetrazol-5-yl)pyridin-2-yl]piperazin-1-yl}ethanone;
- Compound 121: 2-[(2S,6R)-2,6-dimethyl-4-(5-trifluoromethylpyridin-2-yl)piperazin-1-yl]-1-(4-quinolin-2-yl-3,6-dihydro-2H-pyridin-1-yl)ethanone;
- Compound 122: 4-[2-oxo-2-(4-quinolin-2-yl-3,6-dihydro-2H-pyridin-1-yl)ethyl]-1-(5-trifluoromethylpyridin-2-yl)piperazin-2-one;
- Compound 123: 1-(4-quinolin-2-yl-3,6-dihydro-2H-pyridin-1-yl)-2-[8-(5-trifluoromethylpyridin-2-yl)-3,8-diazabicyclo[3.2.1]oct-3-yl]ethanone;
- Compound 124: 1-(4-benzofuran-7-yl-3,6-dihydro-2H-pyridin-1-yl)-2-{8-[5-(1-methyl-1H-tetrazol-5-yl)pyridin-2-yl]-3,8-diazabicyclo[3.2.1]oct-3-yl}ethanone;
- Compound 125: 1-(4-benzofuran-7-yl-3,6-dihydro-2H-pyridin-1-yl)-2-[(2S,6R)-4-(5-methanesulfonyl-pyridin-2-yl)-2,6-dimethylpiperazin-1-yl]ethanone;
in the form of a base or of an acid-addition salt.

7. Process for preparing a compound of formula (I) according to any one of Claims 1 to 6, **characterized in that** a compound of formula (II): 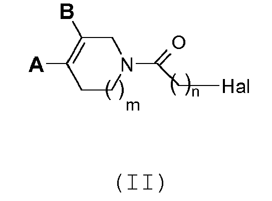 in which A, B, m and n are defined according to any one of Claims 1 to 6 and Hal represents a halogen atom,
and a compound of general formula (III):
H-W-R2 (III)
in which W and R2 are defined according to any one of Claims 1 to 6, are reacted together.

8. Medicament, **characterized in that** it comprises a compound of formula (I) according to any one of Claims 1 to 6, or an addition salt of this compound with a pharmaceutically acceptable acid.

9. Pharmaceutical composition, **characterized in that** it comprises a compound of formula (I) according to any one of Claims 1 to 6, or a pharmaceutically acceptable salt, and also at least one pharmaceutically acceptable excipient.

10. Compound according to any one of Claims 1 to 6, as a medicament.

11. Compound according to any one of Claims 1 to 6, for the preparation of a medicament for preventing or treating central and peripheral neurodegenerative diseases, senile dementia, epilepsy, Alzheimer's disease, Parkinson's disease, Huntington's chorea, Down's syndrome, prion diseases, amnesia, schizophrenia, depression, bipolar disorder, amyotrophic lateral sclerosis, multiple sclerosis, cardiovascular disorders, post-ischemic heart damage, cardiomyopathies, myocardial infarction, cardiac insufficiency, cardiac ischemia, cerebral infarction; peripheral neuropathies, optic nerve and retinal damage, retinal pigment degeneration, glaucoma, retinal ischemia, macular degeneration, spinal cord trauma, cranial trauma, atherosclerosis, stenosis, wound healing disorders, alopecia, pancreatitis, hepatic fibrosis, cancers, tumors, metastases, leukemias, respiratory disorders, pulmonary inflammation, allergy, asthma, chronic obstructive pulmonary disease, cutaneous, somatic, visceral and neurological pain, chronic neuropathic and inflammatory pain, autoimmune diseases, rheumatoid arthritis, ankylosing spondylitis, psoriatic arthritis, plaque psoriasis, bone fractures, bone diseases and osteoporosis.

12. Compound according to one of Claims 1 to 6 as an inhibitor of the dimerization of the p75^{NTR} receptor.

13. Use of a compound according to one of Claims 1 to 6, for the preparation of medicaments that are useful in the treatment and prevention of central and peripheral neurodegenerative diseases, senile dementia, epilepsy, Alzheimer's disease, Parkinson's disease, Huntington's chorea, Down's syndrome, prion diseases, amnesia, schizophrenia, depression, bipolar disorder, amyotrophic lateral sclerosis, multiple sclerosis, cardiovascular disorders, post-ischemic heart damage, cardiomyopathies, myocardial infarction, cardiac insufficiency, cardiac ischemia, cerebral infarction; peripheral neuropathies, optic nerve and retinal damage, retinal pigment degeneration, glaucoma, retinal ischemia, macular degeneration, spinal cord trauma, cranial trauma, atherosclerosis, stenosis, wound healing disorders, alopecia, pancreatitis, hepatic fibrosis, cancers, tumors, metastases, leukemias, respiratory disorders, pulmonary inflammation, allergy, asthma, chronic obstructive pulmonary disease, cutaneous, somatic, visceral and neurological pain, chronic neuropathic and inflammatory pain, autoimmune diseases, rheumatoid arthritis, ankylosing spondylitis, psoriatic arthritis, plaque psoriasis, bone fractures, bone diseases and osteoporosis.
